(19) Europäisches Patentamt
European Patent Office
Office européen des brevets

(11) EP 1 187 837 B1

(12) **EUROPEAN PATENT SPECIFICATION**

(45) Date of publication and mention of the grant of the patent:
**18.07.2007 Bulletin 2007/29**

(21) Application number: **00932279.3**

(22) Date of filing: **10.05.2000**

(51) Int Cl.:
***C07D 513/04*** *(2006.01)* ***A61K 31/55*** *(2006.01)*
***A61P 31/18*** *(2006.01)*

(86) International application number:
**PCT/US2000/012847**

(87) International publication number:
**WO 2000/068235 (16.11.2000 Gazette 2000/46)**

(54) **THIAZEPINE INHIBITORS OF HIV-1 INTEGRASE**

THIAZEPIN-HEMMER VON HIV-1 INTEGRASE

INHIBITEURS DE L'INTEGRASE DU VIH-1 A BASE DE THIAZEPINE

(84) Designated Contracting States:
**AT BE CH CY DE DK ES FI FR GB GR IE IT LI LU MC NL PT SE**

(30) Priority: **12.05.1999 US 133726 P**

(43) Date of publication of application:
**20.03.2002 Bulletin 2002/12**

(73) Proprietor: **THE UNITED STATES GOVERNMENT as represented by THE DEPARTMENT OF HEALTH AND HUMAN SERVICES**
**Rockville, MD 20852-3804 (US)**

(72) Inventors:
- **NEAMATI, Nouri**
  **Kensington, MD 20895 (US)**
- **POMMIER, Yves**
  **Bethesda, MD 20814 (US)**
- **GAROFALO, Antonio**
  **I-53100 Siena (IT)**
- **NACCI, Vito**
  **I-53100 Siena (IT)**

(74) Representative: **Gowshall, Jonathan Vallance et al**
**FORRESTER & BOEHMERT**
**Pettenkoferstrasse 20-22**
**80336 München (DE)**

(56) References cited:
**WO-A-98/18473** **US-A- 4 045 448**

- **GAROFALO ET AL.: "Thioanalogues of anti-tumor antibiotics..." EUR.J.MED.CHEM., vol. 28, 1993, pages 213-220, XP000940751**
- **GAROFALO ET AL.: "Benzothiazine and benzothiazepine derivatives..." IL FARMACO, vol. 48, no. 2, 1993, pages 275-283, XP002044896**
- **NEAMATI N ET AL: "DESIGN AND DISCOVERY OF HIV-1 INTEGRASE INHIBITORS" DRUG DISCOVERY TODAY,GB,ELSEVIER SCIENCE LTD, vol. 2, 1997, pages 487-498, XP000911100 ISSN: 1359-6446**
- **N NEAMATI ET AL: "Design and discovery if HIV-1 integrase inhibitors" DRUG DISCOVERY TODAY,ELSEVIER SCIENCE LTD,GB, vol. 2, no. 11, November 1997 (1997-11), pages 487-498, XP002141742 ISSN: 1359-6446**
- **TETRAHEDRON, vol. 52, no. 22, pages 7745-7754,**
- **J.HETEROCYCL.CHEM., vol. 25, no. 3, pages 1007-1013,**
- **J.HETEROCYCL.CHEM., vol. 22, pages 1071-1075,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 187 837 B1

## Description

### FIELD OF THE INVENTION

The present invention concerns anti-retroviral drugs, and particularly prophylactic and therapeutic treatments for infections with the human immunodeficiency virus (HIV).

### BACKGROUND OF THE INVENTION

HIV is a retrovirus that causes immunosuppression in humans (HIV disease), and leads to a disease complex known as the acquired immunodeficiency syndrome (AIDS). HIV disease is characterized by progressive functional deterioration of the immune system. The treatment of HIV disease has been significantly advanced by the recognition that combining different drugs with specific activities against different biochemical functions of the virus can help reduce the rapid development of drug resistant viruses that were seen in response to single drug treatment. However, even with combined treatments, multi-drug resistant strains of the virus have emerged. There is therefore a continuing need for the development of new anti-retroviral drugs that act specifically at different steps of the viral infection and replication cycle.

The integrase (IN) enzyme is an example of such a specific target. This enzyme catalyzes the insertion by virally-encoded integrase of proviral DNA into the host cell genome, which is the mechanism by which HIV and other retroviruses are introduced into human T-lymphoid cells. For HIV-1, this process is mediated by a 32 kD virally encoded integrase, having conserved sequences in the HIV long terminal repeats (LTR)[1]. Following reverse-transcription in the cytoplasm of infected cells, integrase cleaves two nucleotides from each of the viral DNA ends which contain a highly conserved CA motif. The cleaved DNA migrates to the nucleus as a part of a large nucleoprotein complex, where the integrase catalyzes the insertion of viral DNA into a host chromosome by a direct transesterification reaction.

In vitro assays have previously been developed to identify integrase inhibitors,[2,3] and have permitted the discovery of diverse classes of drugs that inhibit integrase.[5,6] However, the drugs discovered by these assays have not been highly selective and potent inhibitors of the integrase enzyme. Many of these drugs have additionally been non-selective inhibitors of reverse transcriptase or HIV protease, which limits their usefulness in combination therapy directed to different specific steps of the retroviral life cycle.

One class of reported integrase inhibitors is catechol-containing hydroxylated aromatics, which are non-selective integrase inhibitors that can also cross-link proteins[6] and chelate metals[7]. Non-catechol containing compounds, however, have been found to be cytotoxic, perhaps because they are unable to form reactive quinones. Such generalized cytotoxicity is a disadvantage, because it can affect host cells without being selective for retroviral eradication or inhibition.

Some hydrazides have been reported to be novel noncatechol-containing inhibitors of integrase. Structure-activity relationship studies among these inhibitors have indicated that the salicyl moiety is required for activity. Some benzothiazepine derivatives (such as diltiazem) have been reported to have lymphocyte protectant activity in U.S. Patent No. 4,861,770, and benzodiazepine hydrazide derivatives have been reported to inhibit HIV integrase in WO 98/18473. However, a specific anti-integrase inhibitor with minimal cytotoxic activity is not yet available.

### SUMMARY OF THE INVENTION

It has now surprisingly been found that certain noncatechol-containing compounds, including thiazepines, and particularly thiazolothiazepines, and analogs and derivatives thereof, are effective and selective anti-integrase inhibitors. The compounds of the present invention have particularly been found to inhibit both viral replication, and the activity of purified human immunodeficiency virus type-1 integrase (HIV-1 IN). Structure-activity studies have shown that the compounds possessing the pentatomic moiety SC(O)CNC(O) with two keto groups are in general more potent against purified IN than those containing only one keto group. Inhibitory potency against purified IN is maintained when the compounds are substituted with electron-donating or electron-withdrawing groups. Compounds with a naphthalene ring showed enhanced potency, as did those with thiazolo rings. Extension of the thiazole ring diminishes but did not eliminate potency

According to the present invention, there is provided the use of a compound selected from the group consisting of a compound selected from the group consisting of:

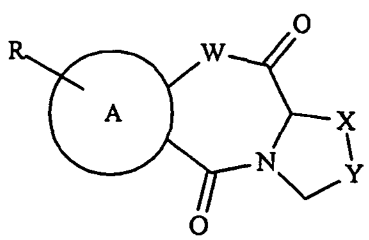

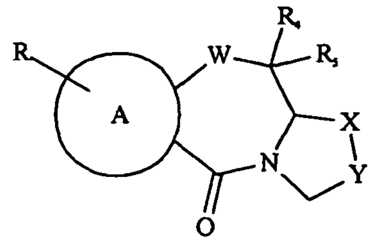

wherein

A is thiazole, benzene, naphthalene, pyridine, pyrimidine, pyrazine, or quinoline;
R is one or more of H, halogen, unsubstituted or substituted cyclic, branched or straight chain alkyl having from 1 to 5 carbon atoms, alkoxy having from 1 to 5 carbon atoms, $NO_2$, or carboxylic acid;
X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$;
$R_4$ is H or hydroxy;
$R_5$ is H, phenyl, or alkylamino; and
W is S or O

or wherein the compound is

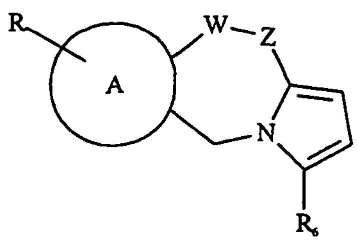

wherein

A is thiazole, benzene, naphthalene, pyridine, pyrimidine, pyrazine, or quinoline; and
R is one or more of H, $NO_2$, halogen, unsubstituted or substituted cyclic, branched or straight chain alkyl having from 1 to 5 carbon atoms, or carboxylic acid;
$R_6$ is H, unsubstituted cyclic, branched or straight chain alkyl or cyclic, branched or straight chain alkyl substituted with one or more of halogen, alkyl, alkoxy, alkylthio, trifluoromethyl, acyloxy, hydroxy, mercapto, carboxy, aryloxy, aryloxy, aryl, arylalkyl, hereroaryl, amino, alkylamino, dialkylamino, morpholino, piperidino, pyrrolidin-1-yl, or piperazin-1-yl or amino.
W is S or O ; and
Z is S, O, $CH_2$, $CH_2CH_2$, or C=O, for the manufacture of a medicament for prophylactic or therapeutic treatment of HIV infection in a subject.

The present invention also provides a compound selected from the group consisting of:

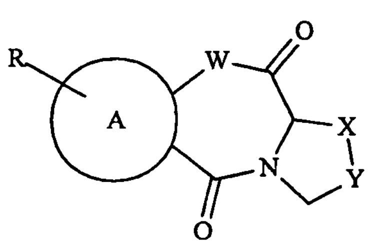

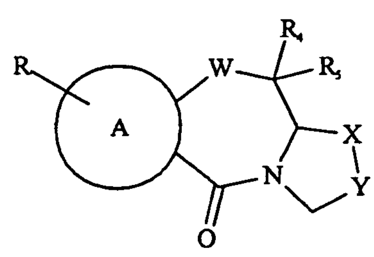

wherein

A is thiazole, benzene, naphthalene, pyridine, pyrimidine, pyrazine, or quinoline;
R is one or more of H, halogen, unsubstituted or substituted cyclic, branched or straight chain alkyl having from 1 to 5 carbon atoms, alkoxy having from 1 to 5 carbon atoms, $NO_2$, or carboxylic acid;
X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$;
$R_4$ is H or hydroxy;
$R_5$ is H, phenyl, or alkylamino; and
W is S or O; with the proviso that the compound is not selected from:

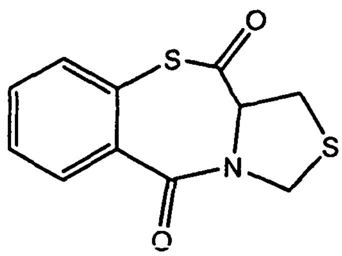
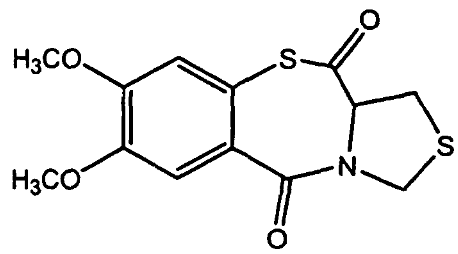

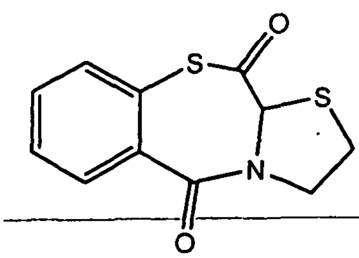
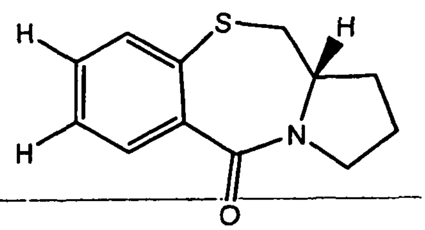

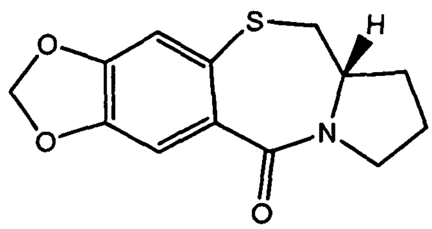
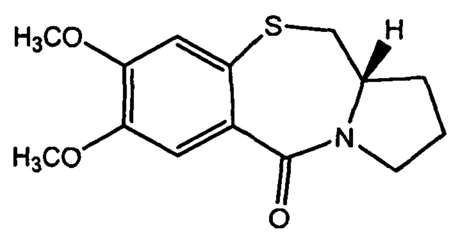

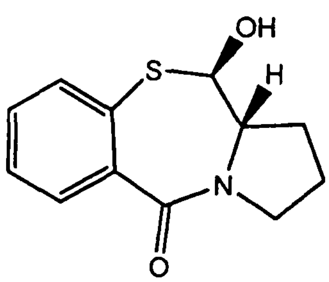
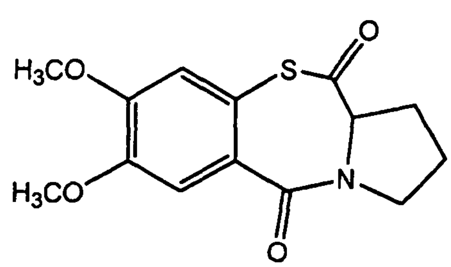

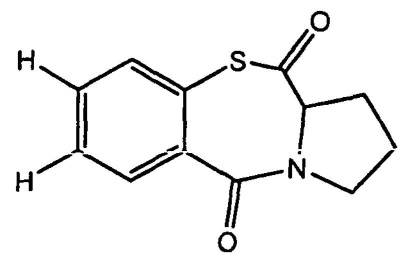

or wherein the compound is

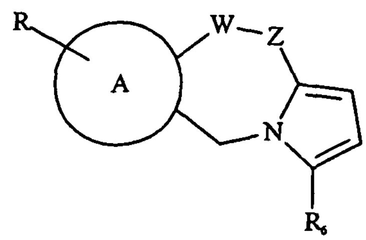

wherein

A is thiazole, benzene, naphthalene, pyridine, pyrimidine, pyrazine, or quinoline; and
R is one or more of H, $NO_2$, halogen, unsubstituted or substituted cyclic, branched or straight chain alkyl having from 1 to 5 carbon atoms, or carboxylic acid;
$R_6$ is H, unsubstituted cyclic, branched or straight chain alkyl, cyclic, branched or straight chain alkyl substituted with one or more of halogen, alkyl, alkoxy, alkylthio, trifluoromethyl, acyloxy, hydroxy, mercapto, carboxy, aryloxy, aryloxy, aryl, arylalkyl, hereroaryl, amino, alkylamino, dialkylamino, morpholino, piperidino, pyrrolidin-1-yl, or piperazin-1-yl or amino;
W is S; and
Z is S, O, $CH_2$, $CH_2CH_2$, or C=O
with the proviso that the compound is not

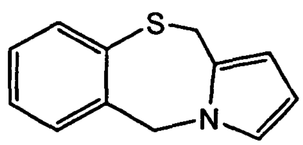

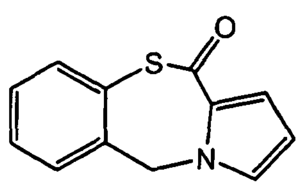

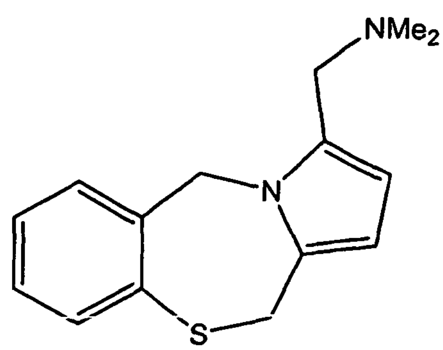

for prophylactic or therapeutic treatment of HIV infection in a subject.

The present invention also provides an HIV integrase inhibitor compound having the following formula, or a pharmaceutically acceptable salt thereof:

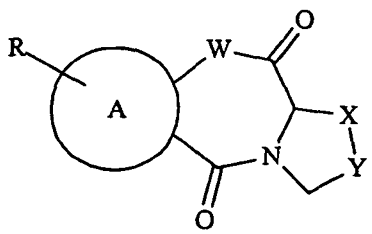

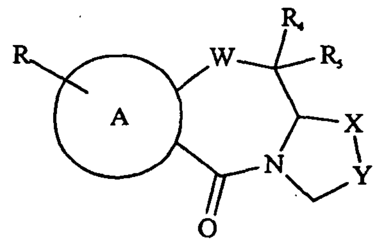

wherein

A is thiazole, benzene, naphthalene, pyridine, pyrimidine, pyrazine, or quinoline;
R is one or more of halogen or $NO_2$ ;
X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$;
$R_4$ is H or hydroxy;
$R_5$ is H, phenyl, or alkylamino; and
W is S or O

or wherein the compound is

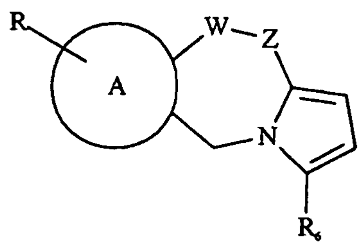

wherein

A is thiazole, benzene, naphthalene, pyridine, pyrimidine, pyrazine, or quinoline; and
R is one or more of halolgen or NO ;
$R_6$ is H, unsubstituted cyclic, branched or straight chain alkyl, cyclic, branched or straight chain alkyl substituted with one or more of halogen, unsubstituted or substituted cyclic, branched or straight chain alkyl, alkoxy, alkylthio, trifluoromethyl, acyloxy, hydroxy, mercapto, carboxy, aryloxy, aryloxy, aryl, arylalkyl, hereroaryl, amino, alkylamino, dialkylamino, morpholino, piperidino, pyrrolidin-1-yl, or piperazin-1-yl or amino;
W is S; and
Z is S, O, $CH_2$, $CH_2CH_2$, or C=O.

The present invention further provides an HN integrase inhibitor compound having the following formula or a pharmaceutically acceptable salt thereof, wherein the compound is:

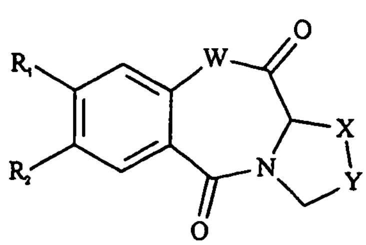

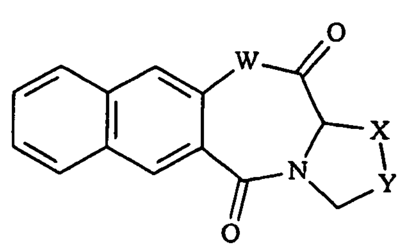

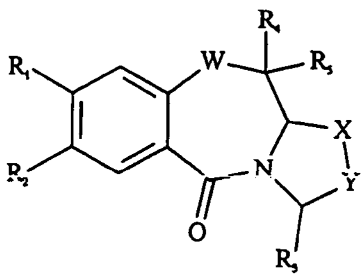

or

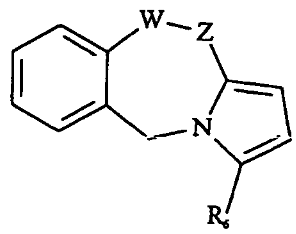

wherein

X-Y is S-$CH_2$, $CH_2$-S, S(O)-$CH_2$. $CH_2$-S(O), or $CH_2CH_2$;
W is S or O;
$R_1$ is H or $NO_2$;
$R_2$ is H, halogen, unsubstituted or substituted cyclic, branched or straight chain alkyl having from 1 to 5 carbon atoms or alkoxy having from 1 to 5 carbon atoms;
$R_3$ is H;
Z is S, O, $CH_2$, $CH_2CH_2$, or C = O;
$R_4$ is hydroxy or H;
$R_5$ is phenyl or $N(CH_2CH_2)_2NCH_3$; and
$R_6$ is $CH_2N(CH_2CH_2)_2NCH_3$,
provided that $R_1$ and $R_2$ are not both H or not both alkoxy.

There is also provided a compound having the formula

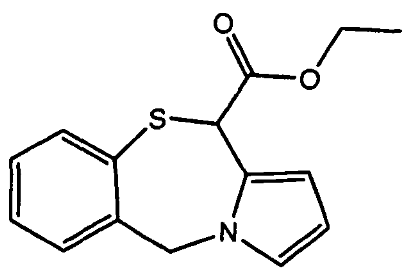

Further preferred features of the invention as set out in the appended dependent claims.

The present disclosure includes a method of inhibiting a HIV integrase by exposing the integrase inhibiting amount of one or more anti-integrase compounds, or pharmaceutically acceptable salts, of

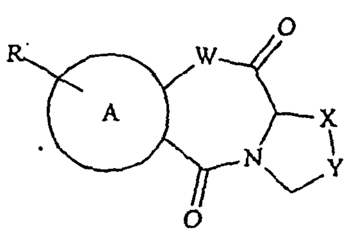

or

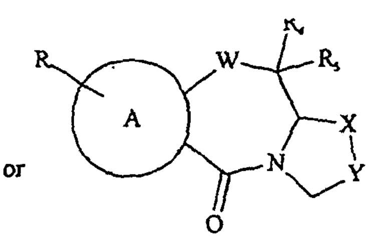

wherein A is thiazole, benzene, naphthalene, pyridine, pyrimidine, pyrazine, or quinoline; R is one or more of H, halogen, lower alkyl, lower alkoxy, $NO_2$, lower ester or carboxylic acid; X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$; $R_4$ is H or hydroxy; $R_5$ is H, phenyl, or alkylamine; and W is S or O or wherein the compound is

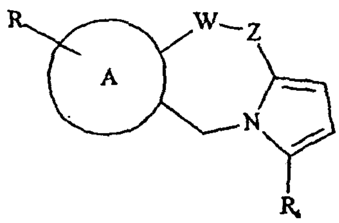

wherein A is thiazole, benzene, naphthalene, pyridine, pyrimidine, pyrazine, or quinoline; and R is one or more of H, halogen, lower alkyl, lower ester or carboxylic acid; $R_6$ is H, substituted or unsubstituted alkyl or amine; W is S or O; and Z is S, O, $CH_2$, $CH_2CH_2$, or C=O.

In yet other aspects of the disclosure, the compound is selected from the group of

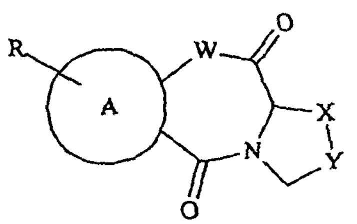

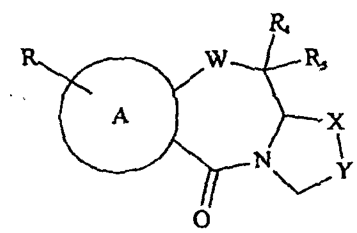

wherein X-Y is $CH_2$-S, S-$CH_2$, $CH_2$-O, or $CH_2$-$CH_2$, and W is S. In particular compounds, A is benzene, naphthalene, pyridine, pyrimidine, pyrazine, or quinoline, and in particular benzene or naphthalene. In other particular examples, R is H, halogen, lower alkoxy, or $NO_2$.

In another aspect of the disclosure, the compound is one or more of:

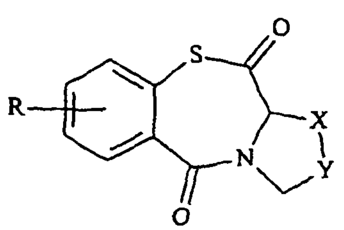

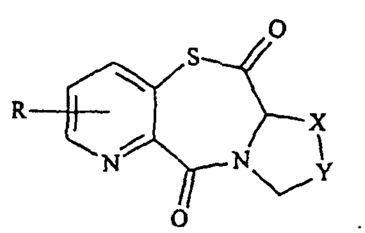

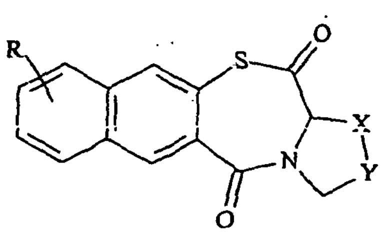

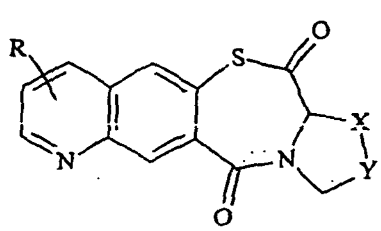

In yet other aspects, the compound is one or more of:

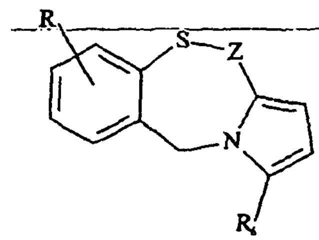

or

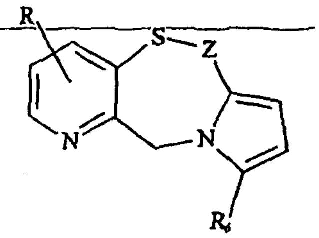

In more specific aspects, the compound is one or more of:

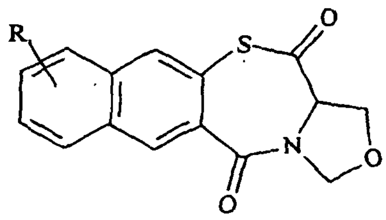

or

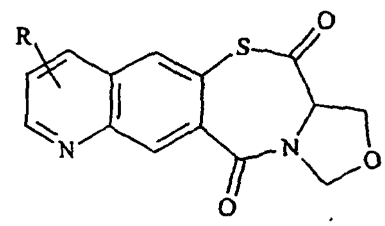

or

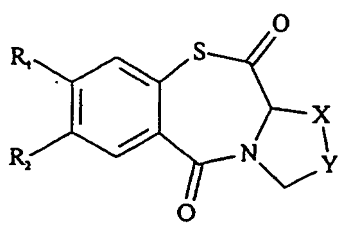

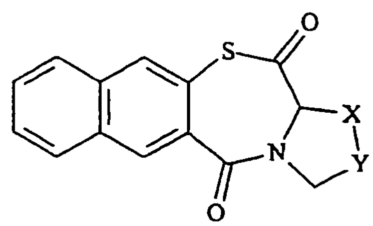

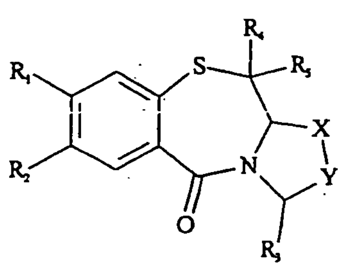

or

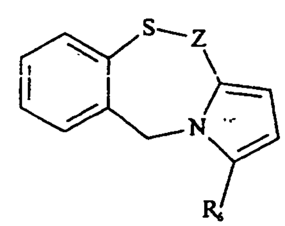

wherein X-Y is S-$CH_2$, $CH_2$-S, $CH_2$-O, $CH_2$-$CH_2$, S(O)-$CH_2$, or $CH_2$-S(O); $R_1$ and $R_2$ are independently selected from the group consisting of H, $NO_2$, halogen, lower alkyl or lower alkoxy; $R_3$ is H or phenyl; $R_4$ is H or hydroxy; $R_5$ is H, phenyl or alkylamine; and $R_6$ is H, phenyl or alkylamine.

In particular aspects, the alkylamine is $-N(CH_2CH_2)_2NCH_3$, $-CH_2NCH_2CH_3$, or $-CH_2N(CH_2CH_2)_2NCH_3$.

A particular group of compounds with superior anti-integrase activity is

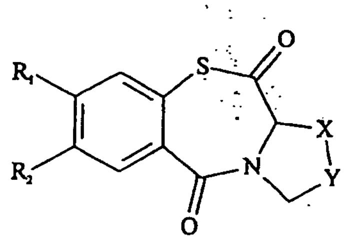

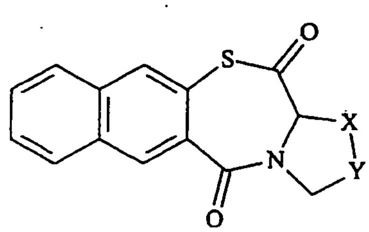

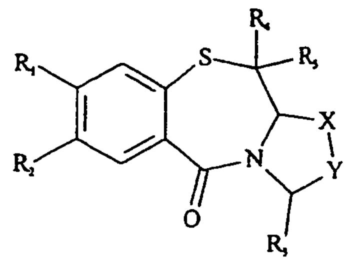

or

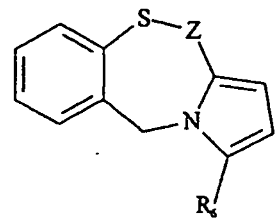

wherein X-Y is S-$CH_2$, $CH_2$-S, or $CH_2$-S(O) ; and $R_1$ and $R_2$ are independently selected from the group consisting of H, $NO_2$, halogen, lower alkyl and lower alkoxy ; $R_3$ is H ; and $R_4$, $R_5$, and $R_6$ are H. An example of such a compound is

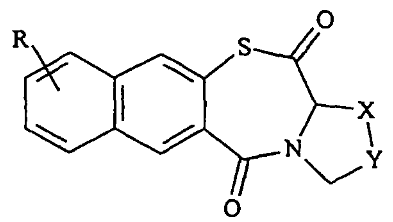

wherein X-Y is S-$CH_2$ or $CH_2$-S, and for example where R is H and X-Y is S-$CH_2$.

In other aspects, in which the compounds have particularly good antiviral activity, $R_1$ is H, $NO_2$, or lower alkoxy; $R_2$ is H, Cl, Br, lower alkyl, or lower alkoxy; $R_3$ and $R_4$ are H; $R_5$ is $N(CH_2CH_2)_2NCH_3$; and X-Y is $CH_2$-S, S-$CH_2$, or $CH_2$-$CH_2$, and particularly:

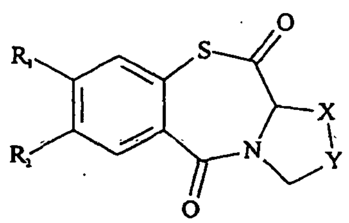

wherein $R_1$ is H, $NO_2$, or methoxy; $R_2$ is H, halogen or methoxy; and X-Y is $CH_2$-S or S-$CH_2$, especially S-$CH_2$.

The present disclosure also includes methods of administering any of the above compounds in a therapeutically effective amount to a subject, for example to treat or prevent HIV infection in the subject. In particular examples, the compound which is administered is selected from the group of

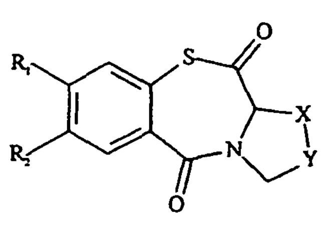

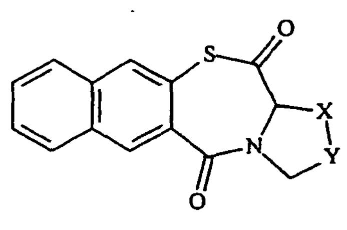

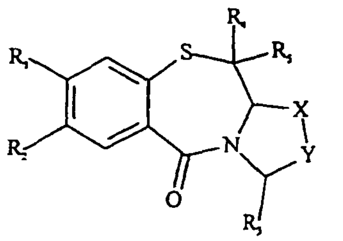

and

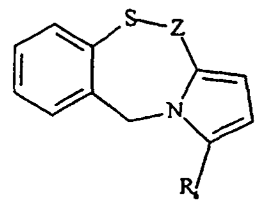

wherein X-Y is S-$CH_2$, $CH_2$-S, $CH_2CH_2$ or S(O)$CH_2$; $R_1$ is H, $NO_2$, or lower alkoxy; $R_2$ is H, Cl, Br, lower alkyl, or lower alkoxy; $R_3$ and $R_4$ are H; $R_5$ is $N(CH_2CH_2)_2NCH_3$; and $R_6$ is H, and in particular:

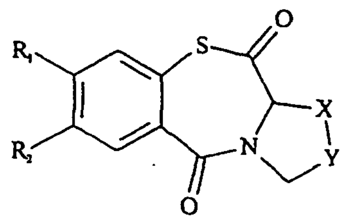

wherein $R_1$ and $R_2$ are H, and X-Y is S-$CH_2$; or $R_1$ is H, $R_2$ is Cl or Br or methyl, and X-Y is S-$CH_2$; or $R_1$ is $NO_2$, $R_2$ is H, and X-Y is $CH_2$-S; or $R_1$ and $R_2$ are methoxy, and X-Y is $CH_2$-S; or $R_1$ is H, $R_2$ is methyl, and X-Y is S(O)-$CH_2$; or

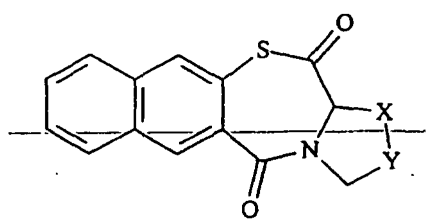

wherein X-Y is S-$CH_2$ or $CH_2$-S; or

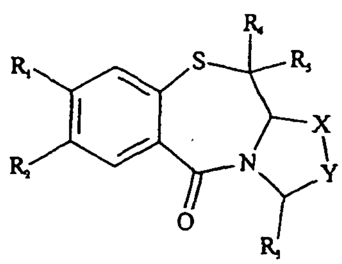

wherein X-Y is $CH_2$-$CH_2$, $R_1$, $R_2$, $R_3$ and $R_4$ are H; and $R_5$ is $N(CH_2CH)_2NCH_3$; or

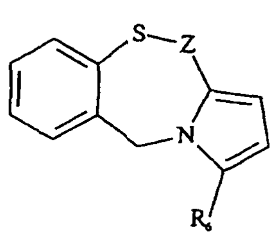

wherein $R_6$ is H and Z is C=O.

In other aspects, R is one or more of halogen or $NO_2$; X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$; $R_4$ is H or hydroxy, particularly hydroxy; $R_5$ is H, phenyl, or alkylamine; and W is S or O; $R_6$ is H, substituted or unsubstituted alkyl or amine; Z is S, O, $CH_2$, $CH_2CH_2$, or C=O, particularly C=O.

In particular aspects, the method includes administration of:

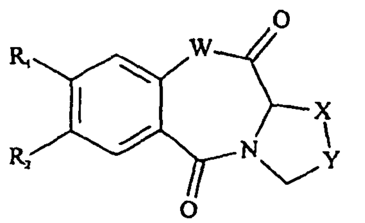

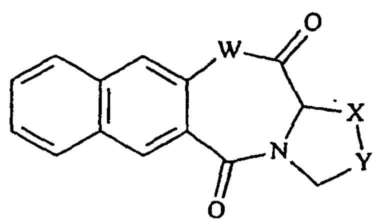

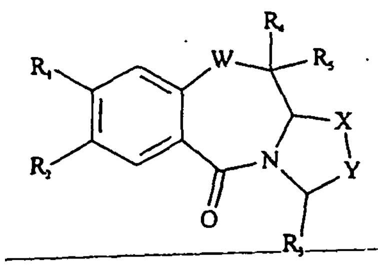

or

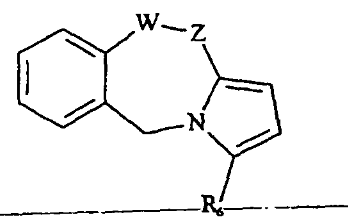

wherein X-Y is S-$CH_2$, $CH_2$-S, S(O)-$CH_2$, $CH_2$-S(O), or $CH_2CH_2$; W is S or O; $R_1$ is H or $NO_2$; $R_2$ is H, halogen, lower alkyl or lower alkoxy; $R_3$ is H; $R_4$ is hydroxy or H; $R_5$ is phenyl or $N(CH_2CH_2)_2NCH_3$; and $R_6$ is $CH_2N(CH_2CH_2)_2NCH_3$, provided that $R_1$ and $R_2$ are not both H or not both lower alkoxy (such as methoxy).

For example, the administered compound can be

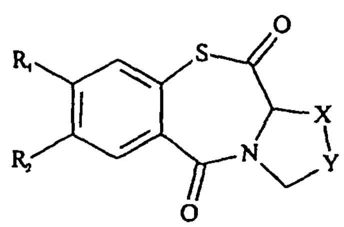

wherein $R_1$ is H or $NO_2$; $R_2$ is H, halogen, lower alkyl or lower alkoxy; provided that $R_1$ and $R_2$ are not both H or lower alkoxy; or wherein $R_1$ is H, $R_2$ is Cl, X-Y is S-$CH_2$; or $R_1$ is H, $R_2$ is Br, X-Y is S-$CH_2$; or $R_1$ is H, $R_2$ is $CH_3$, X-Y is S-$CH_2$; or $R_1$ is H, $R_2$ is H, X-Y is $CH_2$-S; or $R_1$ is H, $R_2$ is Cl, X-Y is $CH_2$-S; or $R_1$ is H, $R_2$ is Br, X-Y is $CH_2$-S; or $R_1$ is H, $R_2$ is $CH_3$, X-Y is $CH_2$-S; or $R_1$ is $NO_2$, $R_2$ is H, X-Y is $CH_2$-S; or $R_1$ is H, $R_2$ is $OCH_3$, X-Y is $CH_2$-S; or $R_1$ is H, $R_2$ is H, X-Y is $CH_2$-O; or $R_1$ is H, $R_2$ is $CH_3$, X-Y is S(O)-$CH_2$; or $R_1$ is H, $R_2$ is H, X-Y is $CH_2$-S(O); or $R_1$ is H, $R_2$ is Cl, X-Y is $CH_2$-S(O); or $R_1$ is H, $R_2$ is $OCH_3$, X-Y is $CH_2$-S(O).

In particular, X-Y may be S-$CH_2$. Alternatively, $R_1$, $R_2$ and $R_3$ are H, $R_4$ is OH or H, $R_5$ is Ph or $N(CH_2CH_2)_2CH_3$, and X-Y is $CH_2$-$CH_2$.

Alternatively, the compound can be:

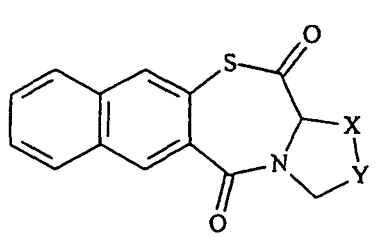

wherein X-Y is $S-CH_2$ or $CH_2-S$.

The compound can also be:

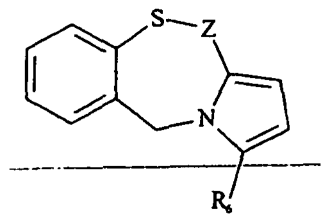

wherein $R_6$ is $CH_2N(CH_2CH_2)_2NCH_3$.

The disclosure also includes a pharmaceutical composition that includes the compound, or a pharmaceutically acceptable salt thereof, and a pharmaceutically acceptable carrier. Also included are methods of screening for an anti-HIV integrase drug, by providing an assay of HIV integrase inhibition, and using the assay to screen for drugs that are analogs or derivatives of any of the compounds, and which inhibit HIV integrase. In particular aspects, the assay detects a thiazepine compound that inhibits human immunodeficiency virus type-1 integrase (HIV-1 IN), and particularly such a compound that has no detectable effect on reverse transcriptase, protease, and virus attachment. In very particular examples; the compounds that are screened are thiazolothiazepines.

## BRIEF DESCRIPTION OF THE FIGURES

FIG. 1 is a schematic diagram illustrating an assay for integrase mediated integration of HIV DNA into the host cell genome. A 21-mer blunt-end oligonucleotide corresponding to the U5 end of the HIV-1 LTR, 5' end-labeled with $^{32}P$, is reacted with purified integrase. The enzyme causes nucleolytic cleavage of two bases from the 3'-end (3' processing), resulting in a 19-mer oligonucleotide. Subsequently, 3' ends are covalently joined to another identical oligonucleotide that serves as the target DNA (strand transfer reaction).

FIGS 2 and 3 show concentration dependent inhibition of HIV-1 IN by thiazolothiazepines **1, 19,** and **20** using $Mn^{+2}$ (FIG. 2) and $Mg^{+2}$ (FIG. 3) as a cofactor. Lane 1, DNA alone, lanes 2 and 15 DNA and integrase, lanes 3-6, 7-10, 11-14 DNA, integrase, and compounds **1, 19**, and **20** at 1000, 333, 111, and 37 μM, respectively. See Table 1 for compound identifiers **1,19,20.**

## DETAILED DESCRIPTION OF PARTICULAR EXAMPLES

### Definitions

A "benzothiazepine" refers to a benzodiazepine in which one of the ring nitrogens has been replaced with a sulfur, for example:

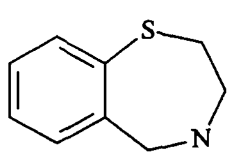

while a "thiazolothiazepine" refers to a benzothiazepine having a thiazolo ring, for example:

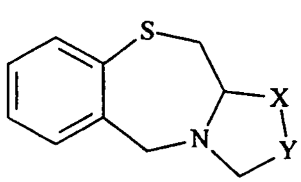

in which X-Y is $CH_2$-S or S-$CH_2$.

The term "alkyl" refers to a cyclic, branched, or straight chain alkyl group containing only carbon and hydrogen, and unless otherwise mentioned contains one to twelve carbon atoms. This term is further exemplified by groups such as methyl, ethyl, n-propyl, isobutyl, t-butyl, pentyl, pivalyl, heptyl, adamantyl, and cyclopentyl. Alkyl groups can either be unsubstituted or substituted with one or more substituents, e.g. halogen, alkyl, alkoxy, alkylthio, trifluoromethyl, acyloxy, hydroxy, mercapto, carboxy, aryloxy, aryloxy, aryl, arylalkyl, heteroaryl, amino, alkylamino, dialkylamino, morpholino, piperidino, pyrrolidin-1-yl, piperazin-1-yl, or other functionality.

The term "lower alkyl" refers to a cyclic, branched or straight chain monovalent alkyl radical of one to five carbon atoms. This term is further exemplified by such radicals as methyl, ethyl, n-propyl, i-propyl, n-butyl, t-butyl, i-butyl (or 2-methylpropyl), cyclopropylmethyl, i-amyl, and n-amyl. Lower alkyl groups can also be unsubstituted or substituted, where a specific example of a substituted alkyl is 1,1-dimethyl propyl.

"Carboxyl" refers to the radical -COOH, and substituted carboxyl refers to -COR where R is alkyl, lower alkyl or a carboxylic acid or ester.

The term "aryl" refers to a monovalent unsaturated aromatic carbocyclic group having a single ring (e.g. phenyl) or multiple condensed rings (e.g. naphthyl or anthryl), which can optionally be unsubstituted or substituted with, e.g., halogen, alkyl, alkoxy, mercapto (-SH), alkylthio, trifluoromethyl, acyloxy, hydroxy, mercapto, carboxy, aryloxy, aryl, arylalkyl, heteroaryl, amino, alkylamino, dialkylamino, morpholino, piperidino, pyrrolidin-1-yl, piperazin-1-yl, or other functionality.

The term "alkoxy" refers to a substituted or unsubstituted alkoxy, where an alkoxy has the structure -O-R, where R is substituted or unsubstituted alkyl. In an unsubstituted alkoxy, the R is an unsubstituted alkyl. The term "substituted alkoxy" refers to a group having the structure -O-R, where R is alkyl which is substituted with a non-interfering substituent. "Lower alkoxy" refers to any alkoxy in which R is a lower alkyl.

The term "heterocycle" refers to a monovalent saturated, unsaturated, or aromatic carbocyclic group having a single ring (e.g. benzyl, morpholino, pyridyl or furyl) or multiple condensed rings (e.g. naphthyl, quinolinyl, indolizinyl or benzo[b]thienyl) and having at least one heteroatom, defined as N, O, P, or S, within the ring, which can optionally be unsubstituted or substituted with, e.g. halogen, alkyl, alkoxy, alkylthio, trifluoromethyl, acyloxy, hydroxy, mercapto, carboxy, aryloxy, aryl, arylalkyl, heteroaryl, amino, alkylamino, dialkylamino, morpholino, piperidino, pyrrolidin-1-yl, piperazin-1-yl, or other functionality.

The term "halogen" refers to fluoro, bromo, chloro and iodo substituents.

The term "amino" refers to a chemical functionality $-NR_1R_2$ where $R_1$ and $R_2$ are independently hydrogen, alkyl, or aryl.

A "pharmaceutical agent" or "drug" refers to a chemical compound or composition capable of inducing a desired therapeutic or prophylactic effect when properly administered to a subject.

The pharmaceutically acceptable salts of the compounds of this invention include those formed from cations such as sodium, potassium, aluminum, calcium, lithium, magnesium, zinc, and from bases such as ammonia, ethylenediamine, N-methyl-glutamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, and tetramethylammonium hydroxide. These salts may be prepared by standard procedures, for example by reacting the free acid with a suitable organic or inorganic base. Any chemical compound recited in this specification may alternatively be administered as a pharmaceutically acceptable salt thereof.

All chemical compounds include both the (+) and (-) stereoisomers, as well as either the (+) or (-) stereoisomer.

An analog is a molecule, that differs in chemical structure from a parent compound, for example a homolog (differing by an increment in the chemical structure, such as a difference in the length of an alkyl chain), a molecular fragment, a structure that differs by one or more functional groups, or a change in ionization. Structural analogs are often found using quantitative structure activity relationships (QSAR), with techniques such as those disclosed in Remington: The Science and Practice of Pharmacology, 19th Edition (1995), chapter 28. A derivative is a biologically active molecule derived from the base structure. A mimetic is a biomolecule that mimics the activity of another biologically active molecule. Biologically active molecules can include both chemical structures and peptides of protein entities that mimic the biological activities of the mercaptosalicylhydrazides of the present invention.

Other chemistry terms herein are used according to conventional usage in the art, as exemplified by The McGraw-Hill Dictionary of Chemical Terms (1985) and The Condensed Chemical Dictionary (1981).

A "mammal" includes both human and non-human mammals. Similarly, the term "subject" includes both human and veterinary subjects.

An animal is a living multicellular vertebrate organism, a category which includes, for example, mammals and birds.

"HIV disease" refers to a well recognized constellation of signs and symptoms (including the development of opportunistic infections) in persons who are infected by an HIV virus, as determined by antibody or western blot studies. Laboratory findings associated with this disease are a progressive decline in T-helper cells.

## Materials and Methods

Where necessary, solvents were dried and purified according to the recommended procedures. All reactions were carried out under an argon atmosphere. Progress of the reaction was monitored by TLC on silica gel plates (Riedel-de-Haen, Art. 37341). Organic solutions were dried over $MgSO_4$, evaporation refers to removal of solvent on a rotary evaporator under reduced pressure. Melting points were determined using an Electrothermal 8103 apparatus and are uncorrected. Optical rotations were measured on an Optical Activity AA-5 polarimeter. IR spectra were recorded as thin films on Perkin-Elmer 398 and FT 1600 spectrophotometers. $^1$H NMR spectra were recorded on a Bruker 200 MHz spectrometer with TMS as internal standard; the values of chemical shifts (δ) are given in ppm and coupling constants (*J*) in Hz. Mass spectral data were determined by direct insertion at 70 eV with a VG70 spectrometer. Merck silica gel (Kieselgel 60/230-400 mesh) was used for flash chromatography columns. Dowex 50x2 200 resin (Aldrich) was used for ion-exchange chromatography. Elemental analyses were performed on a Perkin-Elmer 240C elemental analyzer, and the results are within ±0.4% of the theoretical values. Yields refer to purified products and are not optimized.

## Compound Reference Numbers

**[0049]** Compounds are identified throughout this detailed description using reference numerals in bold, which correspond to the identification of the compounds in Table 1, and in the following examples.

## EXAMPLE 1

### Synthesis of Thiazolothiazepines

The synthesis of compounds **1, 5, 11, 13-14, 21-25** and **28-30** (see Table 1 for an identification of these compounds) has been accomplished according to general or similar procedures reported elsewhere. 15-20 Compounds **2-4, 6-10, 12** and **19-20** (Table 1) have been prepared following the general method outlined in Scheme 1:

Scheme 1

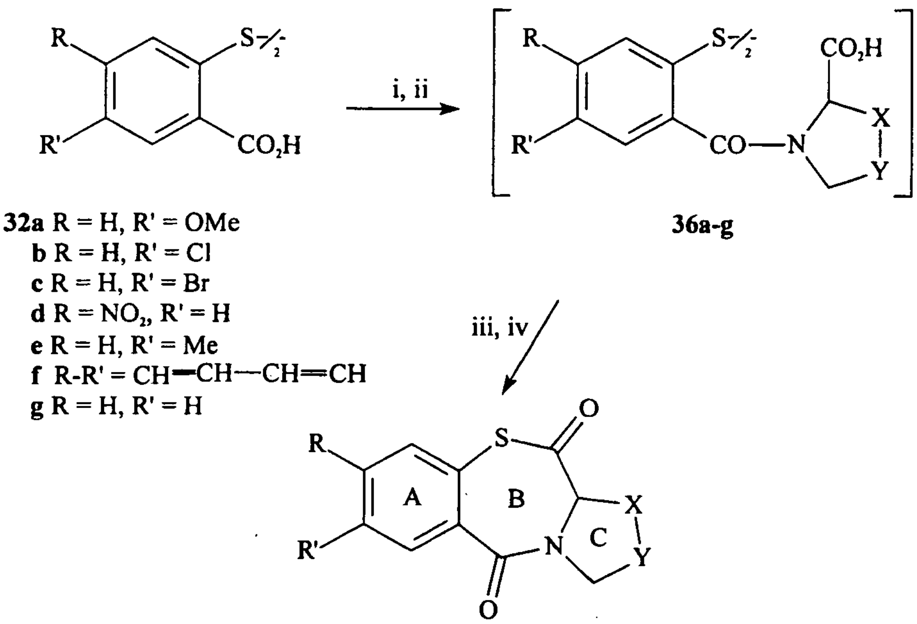


**32a** R = H, R' = OMe
**b** R = H, R' = Cl
**c** R = H, R' = Br
**d** R = $NO_2$, R' = H
**e** R = H, R' = Me
**f** R-R' = CH=CH—CH=CH
**g** R = H, R' = H

**36a-g**

**1-14, 19-20**

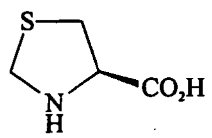
**33**

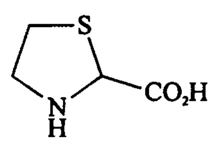
**34**

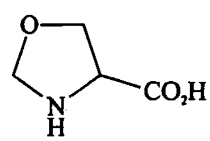
**35**

**Reagents: (i) $SOCl_2$/reflux; (ii) 33 or 34 or 35 /$Na_2CO_3$/THF/$H_2O$/rt; (iii) $NaBH_4$/EtOH/reflux; (iv) CDI/THF/reflux.**

Schotten-Baumann reaction between the (un)substituted 2,2'-dithiobis(benzoic acid chloride) or 3,3'-dithiobis(2,2'-naphtoic acid chloride), in turn obtained from corresponding acids **32a-g** [21-25] and thionyl chloride, and L-thiaproline (Aldrich) (33) or thiazolidine-2-carboxylic acid (**34**) or 1,3-oxazolidine-4-carboxylic acid (35) (Chart 1) gave disulfides **36**. $NaBH_4$ reduction of the crude disulfides gave the corresponding thiophenols in very good yield. Disulfides 36 and subsequent thiophenols were obtained as amorphous solids by ion-exchange chromatography and were then used without a thorough characterization. The eventual cyclization reaction was carried out using N,N'-carbonyldiimidazole (CDI) in dry THF leading to optically inactive tricyclics **2-4, 6-10, 12** and **19-20** because of racemization at C-11a (C-13a in the case of compounds **19** and **20**).

Controlled 3-chloroperbenzoic acid (MCPBA) oxidation of the tricyclic sulfides **4-6** and **10** gave sulfoxides **15-18** (Scheme 2) which were tentatively assigned a *cis* configuration on the basis of $^1$H NMR experiments and Dreiding stereomodels inspection. This would reflect a preferred approach of the electrophile from the less hindered face of the tricyclic ring, namely the one taken up by the C-.11a proton. Example 4 provides more details about synthesis of the sulfoxides 15-18.

Scheme 2

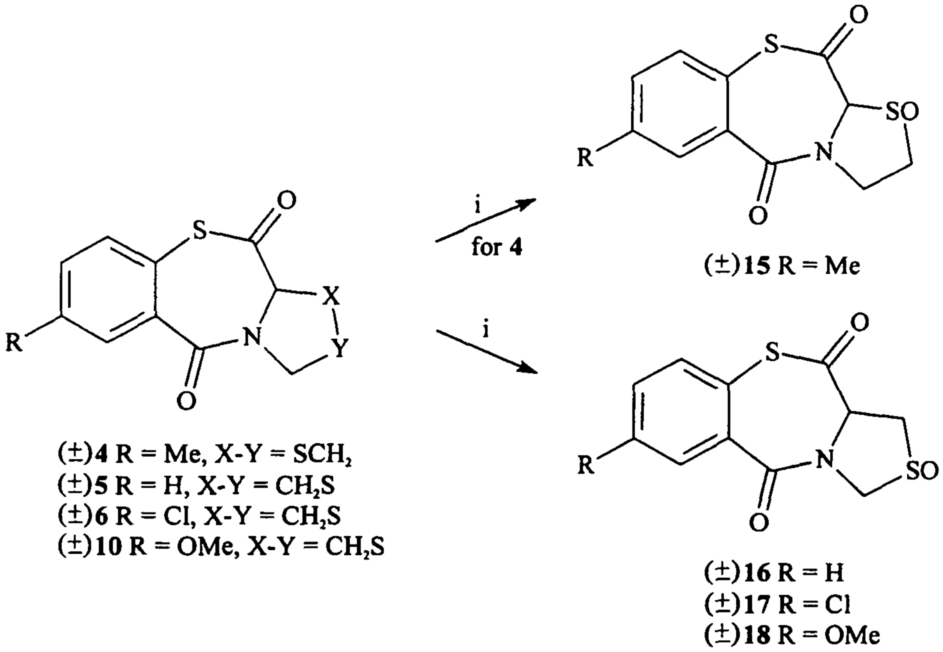


(±)4 R = Me, X-Y = $SCH_2$
(±)5 R = H, X-Y = $CH_2S$
(±)6 R = Cl, X-Y = $CH_2S$
(±)10 R = OMe, X-Y = $CH_2S$

(±)15 R = Me

(±)16 R = H
(±)17 R = Cl
(±)18 R = OMe

Reagents: (i) MCPBA/$CH_2Cl_2$/0 °C

Compound 26 was obtained as a racemic mixture by reaction of previously described (±)-1,2,3,11a-tetrahydro-5*H*,11*H*-pyrrolo[2,1-*c*][1,4]benzothiazepine-5,11-dione **(37)** with phenyl magnesium bromide in dry conditions (Scheme 3).

Scheme 3

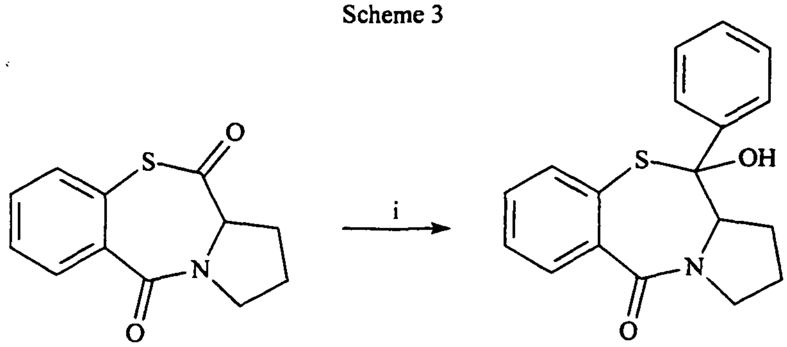


(±) 37

(±) 26

Reagents: (i) PhMgBr/$Et_2O$.

The basic side chain of compound **27** was installed by means of reaction of 1-methylpiperazine on optically active chloro derivative **38** (Scheme 4).

Scheme 4

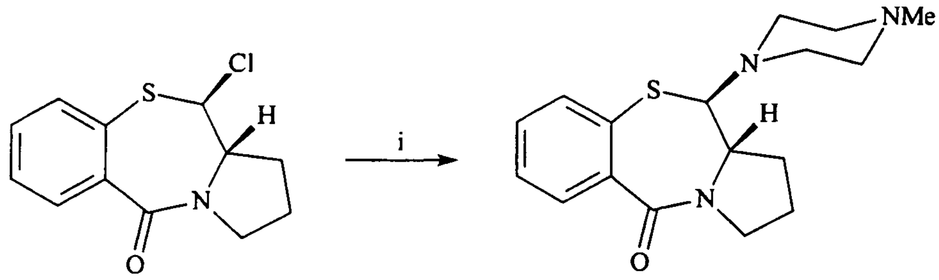

(±) 38

(±) 27

Reagents: (i) $HN(CH_2CH_2)_2NMe/Na_2CO_3/NaI/MeCN$/reflux/20 h.

In the reaction conditions used, a racemic mixture of *cis* **27** was the sole product obtained. Finally, the synthesis of compound 31 was carried out by Mannich reaction on the α-pyrrole position of (±)-11-phenyl-5*H*,11*H*-pyrrolo[2,1-c][1,4]benzothiazepine **(39)** by means of paraformaldehyde and 1-methylpiperazine dihydrochloride in methanol (Scheme 5).

Scheme 5

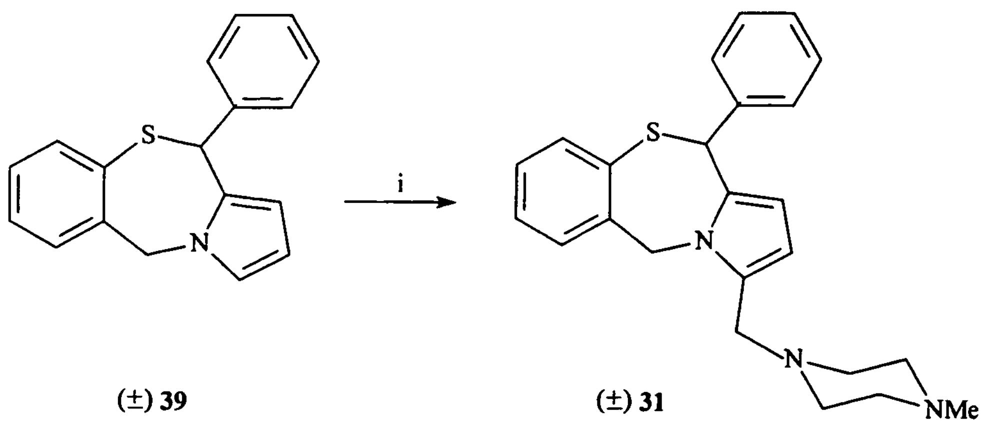

(±) 39

(±) 31

Reagents: (i) $CH_2O/HN(CH_2CH_2)_2NMe._2HCl/MeOH$.reflux 48h.

**EXAMPLE 2**

**Procedure for the Preparation of Thiazepine 10**

This example illustrates the preparation of (±)-1,11a-dihydro-7-methoxy-3*H,*5*H,*11*H*-thiazolo[4,3-c][1,4]benzothiazepine-5,11-dione **(10).** A mixture of 2,2'-dithiobis(5-methoxybenzoic acid) (5.5 g, 15 mmol) (32a) and thionyl chloride (40 mL) was refluxed for 1.5 hours. After cooling, the excess of thionyl chloride was removed under vacuum using dry benzene (2 x 5 mL). The resulting solid was dissolved in dry THF (40 mL) and the solution was added dropwise to a mixture of L-thiaproline (33) (4.0 g, 30.0 mmol) and sodium carbonate (3.2 g, 15.0 mmol) in water (50 mL). Additional sodium carbonate was added from time to time to maintain a weakly alkaline pH. The mixture was stirred overnight, then concentrated and made acidic (pH 3-4) by adding concentrated HCl. The gummy solid was extracted into ethyl acetate and the resulting solution was washed with water, dried, and evaporated to give foam. Ion-exchange column chromatography using methanol as the eluent gave almost pure disulfide **36a** as an amorphous solid.

The solid (8.95 g, 15 mmol) was dissolved in 85% ethanol (100 mL) containing NaOH (1.2 g, 30 mmol), and to this was added a solution of $NaBH_4$ (1.14 g, 30 mmol) in ethanol (50 mL). The mixture was gently refluxed for 0.5 hour, then concentrated and diluted with chilled-water. The cold solution was left for 15 minutes before being filtered

and made acidic (pH 3-4) by concentrated HCl. The gummy solid was treated exactly as described before for purification of compound **36a** and then thoroughly dried under vacuum before being subjected to the successive reaction without further manipulation. Crude thiophenol derivative (6.0 g, 20.0 mmol) was dissolved into dry THF (80 mL) and N,N'-carbonyldiimidazole (3.24 g, 20.0 mmol) was added in portions. The solution was stirred for 24 hours, and then for 2 hours under reflux. The solvent was evaporated and the residue was partitioned between $CHCl_3$ and 0.5 N HCl. The organic solution was separated and washed with $NaHCO_3$ saturated solution and water.

After drying and evaporation of the solvent, a pasty residue was obtained and purified by flash chromatography (8% methanol in EtOAc) to give 4.2 g (74% yield from the thiophenol precursor) of 10 as a white powder. Analysis revealed mp 145-147 °C (benzene); IR (KBr) 1700, 1640 $cm^{-1}$; $^1H$ NMR ($CDCl_3$) δ7.46 (d, 1 H, *J* = 2.8 Hz), 7.35 (m, 1 H), 7.04 (dd, 1 H, *J* = 8.1, 2.8 Hz), 4.79 (AB q, 2 H, *J* = 10.2 Hz), 4.60 (dd, 1 H, J = 6.6, 1.5 Hz), 3.87 (s, 3 H), 3.64 (dd, 1 H, *J* = 12.7, 1.5 Hz), 3.15 (dd, 1 H, J = 12.7, 6.6 Hz). Anal. ($C_{12}H_{11}NO_3S_2$) C, H, N.

**EXAMPLE 3**

**Synthesis and Analytical Results for Compounds 2-4, 6-9, 12, 19-20, 26-27, and 31**

**(±)-7-Chloro-2,3-dihydro-5*H*-thiazolo[2,3-*c*][1,4]benzothiazepine-5,11 (11a*H*)-dione (2).** Starting from 2,2'-dithiobis(5-chlorobenzoic acid) (32b) (5.6 g, 15 mmol), the title compound 2 was obtained (3.6 g, 63% yield from the thiophenol precursor) adopting the same procedure as for 10, but using thiazolidine-2-carboxylic acid **34** instead of L-thiaproline 33: mp 231-232 °C (benzene); IR (KBr) 1695, 1640 $cm^{-1}$; $^1H$ NMR ($CDCl_3$) δ7.98 (d, 1 H, *J* = 2.4 Hz), 7.52-7.38 (m, 2 H), 5.35 (s, 1 H), 4.03 (m, 2 H), 3.16 (m, 2 H). Anal. ($C_{11}H_8ClNO_2S_2$) C, H, N.

**(±)-7-Bromo-2,3-dihydro-5*H*-thiazolo[2,3-*c*][1,4]benzothiazepine-5,11 (11a*H*)-dione (3).** Starting from 2,2'-dithiobis(5-bromobenzoic acid) **(32c)** [22] (7.0 g, 15 mmol), the compound 3 was obtained (4.0 g, 70% yield from the thiophenol precursor) adopting the same procedure as for 10, but using thiazolidine-2-carboxylic acid **34** instead of L-thiaproline **33:** mp 219-220 °C (benzene); IR (KBr) 1695, 1640 $cm^{-1}$; $^1H$ NMR ($CDCl_3$) δ8.11 (d, 1 H, *J* = 2.2 Hz), 7.62 (dd, 1 H, *J* = 8.3, 2.2 Hz), 7.32 (dd, 1 H, *J* = 8.3, 2.2 Hz), 5.33 (s, 1 H), 4.00 (m, 2 H), 3.18 (m, 2 H). Anal. ($C_{11}H_8BrNO_2S_2$) C, H, N.

**(±)-2,3-Dihydro-7-methyt-5*H*-thiazolo[2,3-*c*][1,4]benzothiazepine-5,11 (11a*H*)-dione (4).** Starting from 2,2'-dithiobis(5-methylbenzoic acid) **(32e)** (5.0 g, 15.0 mmol), the compound **4** was obtained (5.6 g, 76% yield from the thiophenol precursor) adopting the same procedure as for **10**, but using thiazolidine-2-carboxylic acid **34** instead of L-thiaproline **33**: mp 197-199 °C (benzene - petroleum ether); IR (KBr) 1690, 1645 $cm^{-1}$; $^1H$ NMR ($CDCl_3$) δ 7.79 (s, 1H,), 7.29 (m, 2 H), 5.36 (s, 1 H), 4.02 (m, 2 H), 3.12 (m, 2 H), 2.42 (s, 3 H). Anal. ($C_{12}H_{11}NO_2S_2$) C, H, N.

**(±)-7-Chloro-1,11a-dihydro-3*H*,5*H*,11*H*-thiazolo[4,3-*c*][1,4] benzothiazepine-5,11-dione (6).** Starting from 2,2'-dithiobis(5-chlorobenzoic acid) (32b) (5.6 g, 15 mmol), the compound 7 was obtained (4.0 g, 70% yield from the thiophenol precursor) using the same procedure as for **10:** mp 204-205 °C (benzene). IR (KBr) 1705, 1640 $cm^{-1}$; $^1H$ NMR ($CDCl_3$) δ 7.96 (d, 1 H, *J* = 2.0), 7.51-7.38 (m, 2 H), 4.80 (AB q, 2 H, *J* = 10.7 Hz), 4.57 (dd, 1 H, *J* = 6.9, 1.7 Hz), 3.67 (dd, 1 H, *J* = 11.9, 1.7 Hz), 3.19 (dd, 1 H, *J* = 11.9, 6.9 Hz). Anal. ($C_{11}H_8ClNO_2S_2$) C, H, N.

(±)-7-**Bromo-1,11a-dihydro-3*H*,5*H*,11*H*-thiazolo [4,3- c] [1,4] benzothiazepine- 5,11- dione** (7). Starting from 2,2'-dithiobis(5-bromobenzoic acid) **(32c)** (7.0 g, 15 mmol), the compound **8** was obtained (4.1 g, 63% yield from the thiophenol precursor) using the same procedure as for 10: mp 213-214 °C (benzene). IR (KBr) 1700, 1635 $cm^{-1}$; $^1H$ NMR ($CDCl_3$) δ 8.10 (t, 1 H, *J* = 1.0 Hz), 7.63 (dd, 1 H, *J* = 8.0, 1.0), 7.34 (d, 1 H, *J* = 8.0), 4.81 (AB q, 2 H, *J* = 10.6 Hz), 4.55 (dd, 1 H, *J* = 6.0, 1.9 Hz), 3.67 (dd, 1 H, *J* = 11.8, 1.9 Hz), 3.18 (dd, 1 H, *J* = 11.8, 6.0 Hz). Anal. ($C_{11}H_8BrNO_2S_2$) C, H, N.

(±)**-1,11a-Dihydro-7-methyl-3*H*,5*H*,11*H*-thiazolo[4,3-*c*][1,4] benzothiazepine-5,11-dione (8).** Starting from 2,2'-dithiobis(5-methylbenzoic acid) (32e) (5.0 g, 15.0 mmol), the compound 8 (6.1 g, 82% yield from the thiophenol precursor) was obtained as a thick oil, using the same procedure as for 10: IR (KBr) 1695, 1640 $cm^{-1}$; $^1H$ NMR ($CDCl_3$) δ 7.74 (s, 1H,), 7.30 (m, 2 H), 4.77 (AB q, 2 H, *J* = 10.2 Hz), 4.60 (dd, 1 H, *J* = 6.8, 1.7 Hz), 3.62 (dd, 1 H, *J* = 12.1, 1.7 Hz), 3.23 (dd, 1 H, *J* = 12.1, 6.8 Hz), 2.40 (s, 3 H). Anal. ($C_{12}H_{11}NO_2S_2$) C, H, N.

**(±)-1,11a-Dihydro-8-nitro-3*H*,5*H*,11*H*-thiazolo[4,3-*c*][1,4] benzothiazepine-5,11-dione (9).** Starting from 2,2'-dithiobis(4-nitrobenzoic acid) **(32d)** (5.9 g, 15 mmol), the compound **9** was obtained (2.0 g, 34% from the thiophenol precursor) using the same procedure as for **10:** mp 231-232 °C (benzene - petroleum ether); IR (KBr) 1715, 1645 $cm^{-1}$; $^1H$ NMR ($CDCl_3$) δ8.35 (s, 1H,), 8.32 (dd, 1 H, *J* = 8.1, 2.0 Hz), 8.16 (d, 1 H, *J* = 8.1 Hz), 4.84 (AB q, 2 H, *J* = 10.5 Hz), 4.54 (dd, 1 H, *J* = 6.4, 1.5 Hz), 3.70 (dd, 1 H, *J* = 11.9, 1.5 Hz), 3.23 (dd, 1 H, *J* = 11.9, 6.4 Hz). Anal. ($C_{11}H_8N_2O_4S_2$) C, H, N.

**(±)-1,11a-Dihydro-3*H*,5*H*,11*H*-oxazolo[4,3-*c*][1,4]benzothiazepine-5,11-dione (12).** Starting from commercial 2,2'-dithiodibenzoic acid **(32g)** (4.6 g, 15.0 mmol), the compound **12** was obtained as a thick oil (3.0 g, 42% yield from the thiophenol precursor) adopting the same procedure as for 10, but using freshly prepared 1,3-oxazolidine-4-carboxylic acid **32** [27] (3.5 g, 30.0 mmol) instead of L-thiaproline **33:** IR (KBr) 1690, 1635 $cm^{-1}$; $^1H$ NMR ($CDCl_3$) δ7.50

(m, 1H,), 7.22 (m, 3H), 5.22 (AB q, 2 H, *J* = 5.3 Hz), 4.81 (dd, 1 H, *J* = 8.8, 1.7 Hz), 4.25 (dd, 1 H, *J* = 5.9, 1.6 Hz), 3.98 (dd, 1 H, *J* = 8.9, 5.9 Hz). MS *mlz* 235 ($M^+$), 207, 177, 150, 136 (100), 108. Anal. ($C_{11}H_9NO_3S$) C, H, N.

**(±)-2,3-Dihydro-5*H*-naphto[2,3-*f*]thiazolo[2,3-*c*][1,4]thiazepine-5,13(13a*H*)-dione (19).** Starting from 3,3'-dithiobis(2,2'-naphtoic acid) (32f) (1.22 g, 1.5 mmol), the compound **19** (0.27 g, 30% yield from the thiophenol precursor) was obtained, adopting the same procedure as for 10, but using thiazolidine-2-carboxylic acid 34 instead of L-thiaproline 33 and carrying out the $NaBH_4$ reduction of the disulfide overnight at room temperature: mp 196-198 ˚C; IR (KBr) 1700, 1630 $cm^{-1}$; $^1H$ NMR ($CDCl_3$) δ8.52 (s, 1 H), 7.99 (s, 1 H), 7.93 (m, 1 H), 7.84 (m, 1 H,), 7.63 (m, 2 H), 5.40 (s, 1 H), 4.11 (m, 2 H), 3.16 (m, 2 H). MS m/z 301 ($M^+$), 273 (100), 186, 158, 142, 114. Anal. ($C_{15}H_{11}NO_2S_2$) C, H, N.

**(±)-1,13a-Dihydro-3*H*,5*H*,11*H*-naphto[2,3-*f*]thiazolo[4,3-*c*][1,4] thiazepine-5,13-dione (20).** Starting from 3,3'-dithiobis(2,2'-naphtoic acid) **(32f)** (1.22 g, 1.5 mmol), the compound **20** (0.34 g, 38% yield from the thiophenol precursor) was obtained, using the same procedure as for 10, but carrying out the $NaBH_4$ reduction of the disulfide overnight at room temperature: mp 200-203 ˚C; IR (KBr) 1695, 1630 $cm^{-1}$; $^1H$ NMR ($CDCl_3$) δ8.51 (s, 1 H), 7.98 (s, 1 H), 7.94 (m, 1 H), 7.84 (m, 1 H,), 7.58 (m, 2 H), 4.85 (AB q, 2 H, *J* = 10.6 Hz), 4.66 (dd, 1 H, *J* = 6.9, 1.6 Hz), 3.65 (dd, 1 H, *J* = 11.8, 1.5 Hz), 3.14 (dd, 1 H, *J* = 12.1, 6.7 Hz). MS *m*/*z* 301 ($M^+$), 273 (100), 186, 158, 142, 114. Anal. ($C_{15}H_{11}NO_2S_2$) C, H, N.

**(±)-2,3,11,11a-Tetrahydro-11-hydroxy-11-phenyl-1*H*,5*H*-pyrrolo [2,1-*c*][1,4]benzothiazepin-5-one (26).** A solution of compound **37**[16] (0.88 g, 3.8 mmol) in dry THF (10 mL) was dropwise added to a solution of PhMgBr in ethyl ether [obtained from 1.23 g of PhBr, 0.185 g of Mg turnings and 8 mL of $Et_2O$]. The mixture was refluxed for 30 min, then cooled to rt and quenched by the addition of $NH_4Cl$ saturated solution. Chloroform extraction and evaporation gave a residue which was purified by column chromatography to give the title compound **26** (0.7 g, 59% yield) along with some other unidentified by-products. The title compound was obtained as colorless crystals by crystallization: mp 137-141˚C (ethanol); IR (KBr) 3210 broad, 1620 $cm^{-1}$; $^1H$ NMR ($CDCl_3$) δ8.02 (m, 1 H), 7.85 (m, 1 H), 7.60-7.10 (m, 7 H), 3.87 (m, 1 H), 3.55 (m, 2 H), 3.08 (br s, 1 H), 1.95 (m, 4 H). Anal. ($C_{18}H_{17}NO_2S$) C, H, N.

**(±)-*cis*-2,3,11,11a-Tetrahydro-11-(4-methylpiperazin-1-yl)-1*H*,5*H*-pyrrolo[2,1-c][1,4]benzothiazepin-5-one (27).** A mixture of (+)-11-chloro-2,3,11,11a-tetrahydro-1*H*,5*H*,-pyrrolo[2,1-*c*][1,4]benzothiazepin-5-one **(38)**[15] (0.6 g, 2.4 mmol), freshly distilled 1-methylpiperazine (0.34 mL, 3.1 mmol), $Na_2CO_3$ (1.06 g, 10.0 mmol), NaI (0.36 g, 2.4 mmol) in dry $CH_3CN$ (30 mL) was gently refluxed for 20 hours. The solvent was evaporated and the residue was partitioned between $CH_2Cl_2$ and water. The organic phase was successively washed with a 2 % solution of $Na_2S_2O_3$, water and brine. The residue obtained after evaporation was chromatographed on silica gel eluting with 5 % methanol in $CH_2Cl_2$. The title compound **27** was obtained as a white solid (0.55 g, 73% yield): mp 188-190˚C (2-propanol - isopropyl ether); IR (KBr) 1630 $cm^{-1}$; $^1H$ NMR ($CDCl_3$) δ7.71 (m, 1 H), 7.50 (m, 2 H), 7.61 (m, 1 H), 7.33 (m, 2 H), 4.36 (d, 1 H, *J* = 11.2 Hz), 3.70 (m, 3 H), 2.58 (m, 4 H), 2.38 (m, 4 H), 2.27 (s, 3 H), 2.02 (m, 4 H). MS *m*/*z* 317 (100, $M^+$), 217, 180, 139, 99, 70. Anal. ($C_{17}H_{23}N_3OS$) C, H, N.

**(±)-3-[(4-Methylpiperazin-1-yl)methyl]-11-phenyl-5*H*,11*H*-pyrrolo [2,1-*c*][1,4]benzothiazepine (31).** A solution of compound **39**[20] (0.55 g, 2.0 mmol), paraformaldehyde (0.1 g) and 1-methylpiperazine dihydrochloride (0.52 g, 3.0 mmol) in $CH_3OH$ (20 mL) was refluxed for 48 hours. After cooling, the mixture was diluted with water and made alkaline (pH 9-10) by dropwise addition of 1N NaOH. The oil formed was extracted with ethyl acetate. The organic layer was washed with water and dried. After evaporation of the solvent, the residue obtained was chromatographed (5 % $CH_3OH$ in $CH_2Cl_2$) to give the title compound 31 as a white solid (0.7 g, 91 % yield): mp 161-162˚C (2-propanol); $^1H$ NMR ($CDCl_3$) δ7.50-7.00 (m, 9 H), 5.97 (s, 1 H), 5.86 (d, 1 H, *J* = 3.5 Hz), 5.53 (d, 1 H, *J* = 3.5 Hz), 5.36 (AB q, 2 H, *J* = 14.5 Hz), 3.50 (AB q, 2 H, *J* = 13.5 Hz), 2.52 (m, 8 H), 2.30 (s, 3 H). Anal. ($C_{24}H_{27}N_3S$) C, H, N.

**EXAMPLE 4**

**Procedure for the Preparation of Sulfoxides 15-18**

This procedure is illustrated for the preparation of (±)-*cis*- 1,11a-Dihydro-3*H,*5*H,*11*H* thiazolo[4,3-*c*][1,4]benzothiazepine-5,11-dione 2-oxide **(16)**. To a stirred and cooled (0˚C) solution of compound 5[15] (0.5 g, 2.0 mmol) in dry dichloromethane (5 mL) ~80% 3-chloroperbenzoic acid (0.43 g, ~2 mmol) in 8 mL of the same solvent was added dropwise over about 15 minutes. After an additional 2 hours at 0˚C, the reaction mixture was filtered and the filter cake was rinsed with dichloromethane. The combined solution was washed twice with 5% aqueous $K_2CO_3$, dried, and evaporated to give the crude **16** (0.47 g, 89% yield), which solidified on trituration with hexane: mp 191-194˚C (benzene); IR (KBr) 1695, 1670 $cm^{-1}$; $^1H$ NMR ($CDCl_3$) δ8.04 (m, 1 H,), 7.55 (m, 3 H,), 5.75 (dd, 1 H, *J* = 13.1, 3.0 Hz), 5.14 (t, 1 H, *J* = 7.6 Hz), 3.91 (d, 1 H, *J* = 13.1 Hz), 3.72 (dd, 1 H, *J* = 14.6, 7.6 Hz), 3.23 (ddd, 1 H, *J* = 14.6, 7.6, 3.0 Hz). Anal. ($C_{11}H_9NO_3S_2$) C, H, N.

**(±)-*cis*-2,3-Dihydro-7-methyl-5*H*-thiazolo[2,3-*c*][1,4]benzothiazepme-5,11 (11a*H*)-dione 1-Oxide (15).** Starting from 4 (0.53 g, 2.0 mmol), the compound **15** (0.39 g, 73 % yield) was obtained using an identical procedure as for **16**: mp 211-212˚C (benzene); IR (KBr) 1690, 1670 $cm^{-1}$; $^1H$ NMR ($CDCl_3$) δ7.79 (d, 1 H, *J* = 2.5 Hz), 7.38 (m, 2 H),

5.25 (s, 1 H), 4.52 (m, 1 H), 3.40 (m, 1 H), 3.10 (m, 1 H), 2.47 (t, 3 H). Anal. ($C_{12}H_{11}NO_3S_2$) C, H, N.

**(±)-*cis*-7-Chloro-1,11a-dihydro-3*H*,5*H*,11*H*-thiazolo[4,3-*c*][1,4] benzothiazepine-5,11-dione 2-Oxide (17).** Starting from 6 (0.57 g, 2.0 mmol), the compound 17 (0.41 g, 68% yield) was obtained using an identical procedure as for 16: mp 213-215°C (benzene); IR (KBr) 1695, 1660 $cm^{-1}$; $^1H$ NMR ($CDCl_3$) δ8.04 (d, 1 H, *J* = 2.7 Hz), 7.50 (m, 2 H), 5.75 (dd, 1 H, *J* = 12.9, 2.9 Hz), 5.13 (t, 1 H, *J* = 7.8 Hz), 3.91 (d, 1 H, *J* = 13.0 Hz), 3.73 (dd, 1 H, *J* = 14.7, 7.2 Hz), 3.23 (ddd, 1 H, *J* = 14.7, 7.8, 2.9 Hz). Anal. ($C_{11}H_8ClNO_3S_2$) C, H, N.

**(±)-*cis*-1,11a-Dihydro-7-methoxy-3*H*,5*H*,11*H*-thiazolo[4,3-*c*][1,4] benzothiazepine-5,11-dione 2-Oxide (18).** Starting from **10** (0.56 g, 2.0 mmol), the compound **18** (0.42 g, 71% yield) was obtained using an identical procedure as for **16:** mp 172-174°C (benzene); IR (KBr) 1690, 1660 $cm^{-1}$; $^1H$ NMR ($CDCl_3$) δ7.55 (d, 1 H, *J* = 2.7 Hz), 7.40 (d, 1 H, *J* = 8.3 Hz), 7.09 (dd, 1 H, *J* = 8.3, 2.7 Hz), 5.76 (dd, 1 H, *J* = 12.9, 2.8 Hz), 5.17 (t, 1 H, *J* = 7.6 Hz), 3.91 (d, 1 H, *J* = 12.9 Hz), 3.89 (s, 3 H), 3.73 (dd, 1 H, *J* = 14.7, 7.1 Hz), 3.23 (ddd, 1 H, *J* = 14.7, 7.6, 2.8 Hz). Anal. ($C_{12}H_{11}NO_4S_2$) C, H, N.

**EXAMPLE 5**

**Integrase Assays**

The HPLC purified oligonucleotides
AE117 (5'-ACTGCTAGAGATTTTCCACAC-3');
AE118 (5'-GTGTGGAAAATCTCTAGCAGT-3');
AE157 (5'-GAAAGCGACCGCGCC-3');
AE146 (5'-GGACGCCATAGCCCCGGCGCGGTCGCTTTC-3');
AE156 (5'-GTGTGGAAAATCTCTAGCAGGGGCTATGGCGTCC-3');
RM22M (5'-TACTGCTAGAGATTTTCCACAC-3'); and
RMAB2 (5'-GTGTGGAAAATCTCTAGCUGT-3') were purchased from Midland Certified Reagent Company (Midland, TX). An expression system for the wild-type integrase and the $IN^{50-212}$ (F185K) were obtained from the Laboratory of Molecular Biology, NIDDK, NIH, Bethesda, MD.

To analyze the extent of 3'-processing and strand transfer using 5'-end labeled substrates, AE118 was 5'-end labeled using $T_4$ polynucleotide kinase (Gibco BRL) and $\gamma[^{32}P]$-ATP (Dupont-NEN). To determine the extent of 30-mer target strand generation during disintegration, AE157 was 5'-end labeled and annealed to AE156, AE146, and AE117. The kinase was heat-inactivated and AE117 was added to the same final concentration. The mixture was heated at 95°C, allowed to cool slowly to room temperature, and run through a G-25 Sephadex quick spin column (Boehringer Mannheim, Indianapolis, IN) to separate annealed double-stranded oligonucleotide from unincorporated label.

To analyze the extent of site-specific cleavage of 3'-end-labeled substrate by integrase, AE118 was 3'-end-labeled using $\gamma[^{32}P]$-cordycepin triphosphate (Dupont-NEN) and terminal transferase (Boehringer Mannheim). The transferase was heat-inactivated, and RM22M was added to the same final concentration. The mixture was heated at 95°C, allowed to cool slowly to room temperature, and run through a G-25 spin column as before.

To determine the extent of Schiff base formation, RMAB2 was 5'-end labeled and reacted with AE117 as described above. The uracil was removed from duplex oligonucleotide containing deoxyuridine by incubation of 40 μl of end-labeled DNA (500 nM stock solution) with 1 unit of uracil DNA glycosylase (Life Technologies, Inc.) for 90 minutes at 30 C. The reaction was then loaded on a G-25 Sephadex quick spin column to remove the unincorporated label and the uracil.

To determine the extent of 3'-processing and strand transfer, integrase was preincubated at a final concentration of 200 nM with the inhibitor in a reaction buffer (50 mM NaCl, 1 mM HEPES, pH 7.5, 50 μM EDTA, 50 μM dithiothreitol, 10% glycerol (w/v), 7.5 mM $MnCl_2$, 0.1 mg/ml bovine serum albumin, 10 mM 2-mercaptoethanol, 10% dimethyl sulfoxide, and 25 mM MOPS, pH 7.2) at 30°C for 30 minutes. Then, 20 nM of the 5'-end $^{32}P$-labeled linear oligonucleotide substrate was added, and incubation was continued for an additional one hour. Reactions were quenched by the addition of an equal volume (16 μl) of loading dye (98% deionized formamide, 10 mM EDTA, 0.025% xylene cyanol and 0.025% bromophenol blue). An aliquot (5 μl) was electrophoresed on a denaturing 20% polyacrylamide gel (0.09 M tris-borate pH 8.3, 2 mM EDTA, 20% acrylamide, 8M urea).

Gels were dried, exposed in a PhosphorImager cassette, and analyzed using a Molecular Dynamics PhosphorImager (Sunnyvale, CA). Percent inhibition was calculated using the following equation:

$$\%I = 100 \times [1 - (D - C)/(N - C)]$$

where C, N, and D are the fractions of 21-mer substrate converted to 19-mer (3'-processing product) or strand transfer products for DNA alone (C), DNA plus integrase (N), and integrase plus drug (D). All $IC_{50}$ values were determined by

plotting the drug concentration versus percent inhibition, and determining the concentration which produced 50% inhibition.

To determine the effects of drugs on the choice of nucleophile in the 3'-processing, reactions were performed essentially as described above with a 3'-end labeled oligonucleotide. Disintegration reactions were performed as above with a Y oligonucleotide (i.e., the branched substrate in which the U5 end was "integrated" into target DNA).

**EXAMPLE 6**

**HIV-1 Cell and Target-based Assays**

The cell-based p24 attachment assay has been described in detail elsewhere.[33] Assays for activity against HIV-1 reverse transcriptase rAdT (template/primer) and rCdG (template/primer) using recombinant HIV-1 reverse transcriptase, have been previously described.[34] The substrate cleavage of recombinant HIV-1 protease in the presence of test compounds was quantified using an HPLC-based methodology with the artificial substrate Ala-Ser-Glu-Asn-Try-Pro-Ile-Val-amide (Multiple Peptide Systems, San Diego, CA) as has been previously described. [33,35]

**EXAMPLE 7**

**Anti-HIV Assays in Cultured Cell Lines**

The anti-HIV drug testing was performed at NCI based on a protocol described by Weislow et al.[36] All compounds were dissolved in dimethyl sulfoxide and diluted 1:100 in cell culture medium. Exponentially growing T4 lymphocytes (CEM cell line) were added at 5000 cells per well. Frozen virus stock solutions were thawed immediately before use, suspended in complete medium to yield the desired multiplicity of infection (m. o. i. ≈ 0.1), and added to the microtiter wells, resulting in a 1:200 final dilution of the compound. Uninfected cells with the compound served as a toxicity control, and infected and uninfected cells without the compound served as basic controls.

Cultures were incubated at 37˚C in a 5% $CO_2$ atmosphere for 6 days. The tetrazolium salt, XTT was added to all wells, and cultures were incubated to allow formazan color development by viable cells. Individual wells were analyzed spectrophotometrically to quantitate formazan production, and in addition were viewed microscopically for detection of viable cells and confirmation of protective activity.

All positive control compounds for individual assays except AZTTP were obtained from the NCI chemical repository. The reference reagents for the individual assays were as follows:

attachment: Farmatalia (NSC 65016)[29] and dextran sulfate (NSC 620255);
reverse transcriptase inhibition: rAdT Template/primer-AZTEC (Sierra BioResearch, Tuscon, AZ), rCdG Template/primer-UC38[30] (NSC 629243);
protease inhibition: KNI-272[31] (NSC 651714).

**EXAMPLE 8**

**Identification of Anti-Integrase and Anti-Viral Compounds**

The thiazepines of the present invention were tested for anti-integrase and anti-viral activity, and the results are shown in Table 1.

**Table 1**

**Anti-HIV-1 Integrase Activities of Thiazepines 1-31**

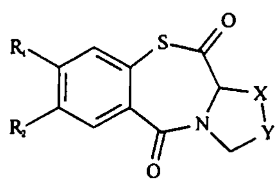

**1-16**

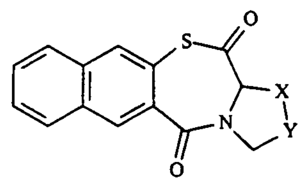

**19-20**

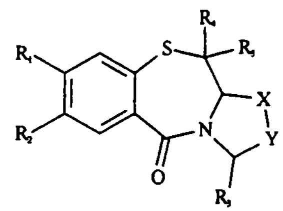

**21-27**

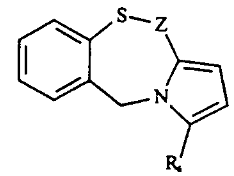

**28-31**

| | | | | | | | | | Integrase Assay $IC_{50}$ (μM) | | Cell data (μM) | |
|---|---|---|---|---|---|---|---|---|---|---|---|---|
| Compd | $R_1$ | $R_2$ | $R_3$ | $R_4$ | $R_5$ | $R_6$ | -X-Y- | Z | 3'-Processing | 3'-end joining | [a]$EC_{60}$ | [b]$CC_{50}$ |
| **1** | H | H | | | | | $-S-CH_2-$ | | 110±12 | 146±79 | 107 | >200 |
| **2** | H | Cl | | | | | $-S-CH_2-$ | | 151; 105 | 120; 60 | >50 | >50 |
| **3** | H | Br | | | | | $-S-CH_2-$ | | 58±15 | 48±18 | | |
| **4** | H | Me | | | | | $-S-CH_2-$ | | 64±47 | 55±29 | | |
| **5** | H | H | | | | | $-CH_2-S-$ | | 208±24 | 227±104 | > 200 | > 200 |
| **6** | H | Cl | | | | | $-CH_2-S-$ | | 158; 111 | 160; 110 | > 100 | > 100 |
| **7** | H | Br | | | | | $-CH_2-S-$ | | 87±24 | 74±32 | >200 | >200 |
| **8** | H | Me | | | | | $-CH_2-S-$ | | 52 | 63 | NR | 35 |
| **9** | $NO_2$ | H | | | | | $-CH_2-S-$ | | 90±27 | 100±36 | > 50 | >50 |
| **10** | H | OMe | | | | | $-CH_2-S-$ | | 155; 275 | 155; 245 | > 200 | > 200 |
| **11** | OMe | OMe | | | | | $-CH_2-S-$ | | 670; 630 | 333; 330 | > 50 | > 50 |
| **12** | H | H | | | | | $-CH_2-O-$ | | > 333 | > 333 | | |
| **13** | OMe | OMe | | | | | $-(CH_2)_2-$ | | > 1000 | > 1000 | > 200 | > 200 |
| **14** | H | H | | | | | $-(CH_2)_3-$ | | 406; 495 | 343±109 | > 200 | > 200 |
| **15** | H | Me | | | | | $-S(O)-CH_2-$ | | 590±350 | 590±350 | > 100 | > 100 |
| **16** | H | H | | | | | $-CH_2-S(O)-$ | | 200; 185 | 215; 222 | | |

(continued)

| Compd | $R_1$ | $R_2$ | $R_3$ | $R_4$ | $R_5$ | $R_6$ | -X-Y- | Z | Integrase Assay $IC_{50}$ (μM) 3'-Processing | 3'-end joining | Cell data (μM) $^aEC_{60}$ | $^bCC_{50}$ |
|---|---|---|---|---|---|---|---|---|---|---|---|---|
| **17** | H | Cl | | | | | $-CH_2-S(O)-$ | | 260; 215 | 280; 200 | | |
| **18** | H | OMe | | | | | $-CH_2-S(O)-$ | | 84.5 | 142 | | |
| **19** | | | | | | | $-S-CH_2-$ | | 40±10 | 47±6 | 60 | >316 |
| **20** | | | | | | | $-CH_2-S$ | | 92±30 | 100±40 | 280 | >316 |
| **21** | H | H | H | OAc | H | | $-(CH_2)_2-$ | | > 1000 | > 1000 | > 200 | > 200 |
| **22** | H | H | H | OMe | H | | $-(CH_2)_2-$ | | > 1000 | > 1000 | > 200 | > 200 |
| **23** | H | H | Ph | H | H | | $-CH_2-S-$ | | 372; 111 | 376; 288 | | |
| **24** | OMe | OMe | H | H | H | | $-(CH_2)_2-$ | | > 1000 | > 1000 | > 200 | > 200 |
| **25** | H | H | H | OH | H | | $-(CH_2)_2-$ | | > 1000 | > 1000 | > 200 | > 200 |
| **26** | H | H | H | OH | Ph | | $-(CH_2)_2-$ | | > 1000 | > 1000 | | |
| **27** | H | H | H | H | | $N(CH_2CH_2)_2NMe$ | $-(CH_2)_2-$ | | > 1000 | > 1000 | > 125 | > 125 |
| **28** | | | | | | H | | > CO | 590 | 300 | 146 | > 200 |
| **29** | | | | | | H | | $> CHCO_2Et$ | > 1000 | > 1000 | | |
| **30** | | | | | | $CH_2NMe_2$ | | $>CHC_6H_4$-*p*F | >1000 | > 1000 | | |
| **31** | | | | | | $CH_2N(CH_2CH_2)_2NMe$ | | >CHPh | >1000 | > 1000 | | |

$^aEC_{60}$: 50% effective concentration
$^bCC_{50}$: 50% cytotoxic concentration

Benzothiazepine 1 was identified as an IN inhibitor ($IC_{50}$ values for 3'-processing and 3'-end joining: 110 and 146 μM, respectively) by showing it to have antiviral activity in the NCI Antiviral Drug Screen against CEM cells (Example 7). Compound **1** with a therapeutic index (TI = $CC_{50}/EC_{50}$) value of > 1.8 (50% effective concentration [$EC_{50}$] value of 107±26 and a 50% cytotoxic concentration [$CC_{50}$] value of > 200 μM) is moderately active in HIV-1 infected CEM cells. This compound was also found to be relatively non-cytotoxic, such that it would more specifically affect HIV than normal host cells. Hence once the inventors recognized the anti-integrase activity of this compound, benzothiazepine 1 served as a "lead" compound for the design and testing of more potent derivatives.

In order to establish a structure-activity relationship, 30 analogs were prepared and tested in an assay specific for IN as well as against HIV-1-infected CEM cells (see Table 1). Several modifications were made to determine the biological effect of each ring and the substituents. The first modifications were aimed at the A and B rings (compounds **1-18**). In a second class of compounds, an extra benzene ring was added, and the effect of the position of the sulfur in the thiazolo ring was analyzed (19 and 20). The third group contains substitutions on all the rings (**21-27**) and the last group (**28-31**) was designed to determine the effect of the thiazepine ring system on biological activity.

As illustrated in Table 1, the chloro-, bromo-, and the methyl-substituted derivatives **2-4**, exhibited potency similar to the parent compound 1 against purified IN. Compound **2** was tested for antiviral activity only at 50 μM and lower. Comparable results were obtained when sulfur was moved to position 10 as in compounds **5-11** (Table 1). However, the removal of sulfur (compounds **12-14**) substantially reduced anti-IN and antiviral activities. Oxidation of the sulfur atom also generally reduced potency (compounds **15-18**), but with compound 18 showing the best anti-IN activity. Both of the naphtho-derivatives 19 and 20 showed antiviral as well as anti-IN activity. Compound **19** with an $IC_{50}$value of 40 μM against IN was more active than the parent compound **1** against HIV-1 infected cells. Compound 19 with a therapeutic index (TI) value of > 5 was the best in this series ($EC_{50}$: 60 and $CC_{50}$: > 316 μM). Moreover, the naphthalene substituted compound 20 was active both against purified IN ($IC_{50}$ values of 92 and 100 μM against 3'-processing and strand transfer, respectively) and HIV-1 infected cells ($EC_{50}$: 279-286 and $IC_{50}$: > 316 μM). Other modifications of the ring system (compounds **21-31**) reduced both antiviral and anti-IN activities.

In particular embodiments, but without limiting the invention, an EC50 of 200 μM or less identifies a compound that is particularly suitable for further investigation. A $CC_{50}$ as high as possible is desired, for example at least 100, 200, 300 or even 1000. A TI value of at least 1 (for example 1.5 or greater) also helps identify compounds for further investigation.

## EXAMPLE 9

### Role of divalent metals

Divalent metal ions, such as $Mn^{+2}$ or $Mg^{+2}$, coordinate with the acidic residues (D, D, E) of IN's active site.[27] Metals are involved in the catalytic functioning because the enzyme is unable to perform 3' processing and strand transfer without $Mn^{+2}$ or $Mg^{+2}$. Previous studies have implied that hydroxylated aromatic inhibitors of IN are potentially metal chelators. [8,9,28] Thus, chelating metals at the active site of IN has been proposed to be responsible for the inhibition of IN function. However, hydroxylated aromatics are generally active only when $Mn^{+2}$ is used as a cofactor.[8]

In contrast, the thiazolothiazepines of the present invention are equally active in $Mg^{+2}$-based assays. Figure 1 shows a representative gel comparing the inhibition of IN in the presence of $Mn^{+2}$ or $Mg^{+2}$. A 21-mer oligonucleotide corresponding to the last 21 bases on the HIV-1 viral DNA in the U5 region of long terminal repeat (LTR) was radiolabeled with $^{32}P$. This is shown in the Figs. 2 and 3 as a band corresponding to DNA alone. Integrase will cleave the last two bases of this DNA in the presence of $Mn^{+2}$ or $Mg^{+2}$ to give a 19-mer DNA substrate (lane 2) which runs faster on the gel and is indicated as 19-mer on the Figures. When an inhibitor is added this process is blocked, therefore the original 21-mer DNA would not be cut. When an inhibitor is added at various concentrations, percent inhibition at every concentration will be calculated by quantification of the intensity of each band using an Imagequant program.

The gels are run using polyacrylamide according to the standard procedures. Upon completion of electrophoresis run, the gels are dried and exposed to a phosphorimager cassette. The cassettes are analyzed by the phosphorimager, and the bands are shown on a computer screen. The intensity of each band is quantitated, plotted against concentrations of drugs, and the $IC_{50}$ values are calculated from the graphs.

Compounds **1, 19** and **20** were active in the presence of $Mg^{+2}$ within the same concentration range as in $Mn^{+2}$, thus indicating that these compounds differ from hydroxylated aromatics and perhaps act at different sites on IN. Inhibition of IN in the presence of $Mg^{+2}$ by thiazolothiazepines sets this class of compounds apart from other IN inhibitors. The activity in $Mg^{+2}$ is believed to be related to the antiviral activity of the thiazolothiazepines and derivatives, because $Mg^{+2}$ has been proposed to be the metal used *in vivo* by IN. Hence compounds having activity that is inhibited in the presence of $Mg^{2+}$ would be expected to have less antiviral activity in the presence of the viral $Mg^{2+}$.

## EXAMPLE 10

### Selectivity of Compounds

The selectivity of compounds **1, 19,** and **20** was examined against other sites on the HIV replication cycle. Assays for activity against HIV-1 reverse transcriptase rAdT (template/primer) and rCdG (template/primer) using recombinant HIV-1 reverse transciptase (from ABL Basic Research NCI-FCRDC, Frederick, MD) have been previously described.[30] A cell-based viral p24 attachment assay, and a nucleoprotein zinc finger assay, have also been described in detail in Rice et al.[33] The substrate cleavage of recombinant HIV-1 protease in the presence of test compounds was quantified using an HPLC-based methodology with the artificial substrate Ala-Ser-Glu-Asn-Tyr-Pro-Ile-Val-amide (Multiple Peptide Systems, San Diege, CA) as previously described.[30]

When tested against reverse transcriptase, protease, virus attachment, or nucleocapsid protein zinc fingers, none of the compounds exhibited any detectable activities at 100 $\mu$M, indicating moderate selectivity against IN. Thus, thiazolothiazepines are a group of selective inhibitors of HIV IN, that can serve as therapeutic compounds or lead compounds in the development of analogs and other derivatives.

The fact that thiazolothiazepines are equally potent in $Mg^{+2}$- and $Mn^{+2}$-based assays indicates that the IN binding site of these compounds differs from the binding site of previously reported inhibitors. Testing these compounds against other viral proteins (reverse transcriptase, protease, virus attachment, or nucleocapsid zinc fingers) shows selectivity of the compounds for IN, as compared to other steps in the retroviral life cycle. Another advantage of this class of compounds is that they are amenable for preparation of chemical libraries using recent advances in combinatorial chemistry, and can be used as novel lead compounds for designing drugs against IN and HIV replication.

**Reference Reagents for Mechanistic and Target-based Assays.** All positive control compounds for individual assays except AZTTP were obtained from the NCI chemical repository. The reference reagents for the individual assays are as follows:

attachment: Farmitalia (NSC 65016) and dextran sulfate (NSC 620255);
reverse transcriptase inhibition: rAdT Template/primer-AZTEC (Sierra BioResearch, Tuscon, AZ), rCdG Template/primer-UC38 (NSC 629243);
protease inhibition: KNI-272 (NSC 651714);
IN inhibitor: ISIS 5320 (NSC 665353) and DIBA-1 (NSC 654077) a NCp7 Zn finger inhibitor.

## EXAMPLE 11

### Methods of Treatment

The present invention includes a treatment for HIV disease and associated diseases, in a subject such as an animal, for example a rat or human. The method includes administering the compound of the present invention, or a combination of the compound and one or more other pharmaceutical agents, to the subject in a pharmaceutically compatible carrier and in an amount effective to inhibit the development or progression of HIV disease. Although the treatment can be used prophylactically in any patient in a demographic group at significant risk for such diseases, subjects can also be selected using more specific criteria, such as a definitive diagnosis of the condition.

The vehicle in which the drug is delivered can include pharmaceutically acceptable compositions of the drugs, using methods well known to those with skill in the art. Any of the common carriers, such as sterile saline or glucose solution, can be utilized with the drugs provided by the invention. Routes of administration include but are not limited to oral and parenteral rountes, such as intravenous (iv), intraperitoneal (ip), rectal, topical, ophthalmic, nasal, and transdermal.

The drugs may be administered intravenously in any conventional medium for intravenous injection, such as an aqueous saline medium, or in blood plasma medium. The medium may also contain conventional pharmaceutical adjunct materials such as, for example, pharmaceutically acceptable salts to adjust the osmotic pressure, lipid carriers such as cyclodextrins, proteins such as serum albumin, hydrophilic agents such as methyl cellulose, detergents, buffers, preservatives and the like. A more complete explanation of parenteral pharmaceutical carriers can be found in Remington: The Science and Practice of Pharmacy (19th Edition, 1995) in chapter 95.

Embodiments of other pharmaceutical compositions can be prepared with conventional pharmaceutically acceptable carriers, adjuvants and counterions as would be known to those of skill in the art. The compositions are preferably in the form of a unit dose in solid, semi-solid and liquid dosage forms such as tablets, pills, powders, liquid solutions or suspensions.

The compounds of the present invention are ideally administered as soon as possible after potential or actual exposure to HIV infection. For example, once HIV infection has been confirmed by laboratory tests, a therapeutically

effective amount of the drug is administered. The dose can be given by frequent bolus administration.

Therapeutically effective doses of the compounds of the present invention can be determined by one of skill in the art, with a goal of achieving tissue concentrations that are at least as high as the $IC_{50}$ of each drug tested in the foregoing examples. The low toxicity of the compound makes it possible to administer high doses, for example 100 mg/kg, although doses of 10 mg/kg, 20 mg/kg, 30 mg/kg or more are contemplated. An example of such a dosage range is 0.1 to 200 mg/kg body weight orally in single or divided doses. Another example of a dosage range is 1.0 to 100 mg/kg body weight orally in single or divided doses. For oral administration, the compositions are, for example, provided in the form of a tablet containing 1.0 to 1000 mg of the active ingredient, particularly 1, 5, 10, 15, 20, 25, 50, 100, 200, 400, 500, 600, and 1000 mg of the active ingredient for the symptomatic adjustment of the dosage to the subject being treated.

The specific dose level and frequency of dosage for any particular subject may be varied and will depend upon a variety of factors, including the activity of the specific compound, the metabolic stability and length of action of that compound, the age, body weight, general health, sex, diet, mode and time of administration, rate of excretion, drug combination, and severity of the condition of the host undergoing therapy.

The pharmaceutical compositions can be used in the treatment of a variety of retroviral diseases caused by infection with retroviruses that require integrase activity for infection and viral replication. Examples of such diseases include HIV-1, HIV-2, the simian immunodeficiency virus (SIV), the feline immunodeficiency virus (FIV), HTLV-1, HTLV-2, spumavirus (human foamy virus) and feline infectious leukemia.

## EXAMPLE 12

### Combination Therapy

The present invention also includes combinations of HIV integrase inhibitor compounds with one or more agents useful in the treatment of HIV disease. For example, the compounds of this invention may be administered, whether before or after exposure to the virus, in combination with effective doses of other anti-virals, immunomodulators, anti-infectives, or vaccines. The term "administration" refers to both concurrent and sequential administration of the active agents.

Example of antivirals that can be used in combination with the integrase inhibitors of the invention are: AL-721 (from Ethigen of Los Angeles, CA), recombinant human interferon beta (from Triton Biosciences of Alameda, CA), Acemannan (from Carrington Labs of Irving, TX), gangiclovir (from Syntex of Palo alto, CA), didehydrodeoxythymidine or d4T (from Bristol-Myers-Squibb), EL10 (from Elan Corp. of Gainesville, GA), dideoxycytidine or ddC (from Hoffman-LaRoche), Novapren (from Novaferon labs, Inc. of Akron, OH), zidovudine or AZT (from Burroughs Wellcome), ribaririn (from Viratek of Costa Mesa, CA), alpha interferon and acyclovir (from Burroughs Wellcome), Indinavir (from Merck & Co.), 3TC (from Glaxo Wellcome), Ritonavir (from Abbott), Saquinavir (from Hoffmann-LaRoche), and others.

Examples of immuno-modulators that can be used in combination with the integrase inhibitors of the invention are AS-101 (Wyeth-Ayerst Labs.), bropirimine (Upjohn), gamma interferon (Genentech), GM-CSF (Genetics Institute), IL-2 (Cetus or Hoffinan-LaRoche), human immune globulin (Cutter Biological), IMREG (from Imreg of New Orleans, La.), SK&F106528, and TNF (Genentech).

Examples of some anti-infectives with which the integrase inhibitors can be used include clindamycin with primaquine (from Upjohn, for the treatment of pneumocystis pneumonia), fluconazlone (from Pfizer for the treatment of cryptococcal meningitis or candidiasis), nystatin, pentamidine, trimethaprim-sulfamethoxazole, and many others.

The combination therapies are of course not limited to the lists provided in these examples, but includes any composition for the treatment of HIV disease (including treatment of AIDS).

## EXAMPLE 13

### Obtaining HIV Integrase Inhibitors by Combinatorial Chemistry

Combinatorial chemistry allows generation of large numbers of unique molecules with a small number of chemical reactions. Rather than using the traditional approach of synthesizing novel compounds one at a time, compounds are synthesized by performing chemical reactions in stages, and reacting all of the molecules formed in stage n-1 with each reactant in stage n. Such techniques can be used to obtain analogs and other variants of the thiazepines of the present invention, and to test them for anti-integrase activity.

In view of the many possible embodiments to which the principles of the invention may be applied, it should be recognized that the illustrated embodiments are only particular examples of the invention and should not be taken as a limitation on the scope of the invention. Rather, the scope of the invention is defined by the following claims. We therefore claim as our invention all that comes within the scope and spirit of these claims.

**References**

1) Brown, P. O. Integration; Cold Spring Harbor Press: Cold Spring Harbor, 1998.

2) Mazumder, A.; Neamati, N.; Sunder, S.; Owen, J.; Pommier, Y. Retroviral Integrase: A Novel Target in Antiviral Development; Basic In Vitro Assays with the Purified Enzyme; Mazumder, A. ; Neamati, N. ; Sunder, S.; Owen, J.; Pommier, Y., Ed.; The Humana Press, Inc.: Totowa, NJ, 1998, pp 1998.

3) Chow, S. A. In vitro assays for activities of retroviral integrase. Methods 1997, 12, 306-17.

4) Chow, S. A.; Vincent, K. A.; Ellison, V.; Brown, P. O. Reversal of integration and DNA splicing mediated by integrase of human immunodeficiency virus. Science 1992, 255, 723-6.

5) Pommier, Y.; Pilon, A.; Bajaj, K.; Mazumder, A.; Neamati, N. HIV-1 integrase as a target for antiviral drugs. Antiviral Chem Chemother 1997, 8, 483-503.

6) Neamati, N.; Sunder, S.; Pommier, Y. Design and discovery of HIV-1 integrase inhibitors. Drug Discovery Today 1997, 2, 487-498.

7) Stanwell, C.; Ye, B.; Yuspa, S. H.; Burke, T. R., Jr. Cell protein crosslinking by erbstatin and related compounds. Biochem Pharmacol 1996, 52, 475-80.

8) Neamati, N.; Hong, H.; Owen, J. M.; Sunder, S.; Winslow, H. E.; Christensen, J. L.; Zhao, H.; Burke, J. T. R.; Milne, G. W. A.; Pommier, Y. Salicylhydrazine-containing inhibitors of HIV-1 integrase: implication for a selective chelation in the integrase active site. J Med Chem 1998, 41, 3202-3209.

9) Neamati, N.; Hong, H.; Mazumder, A.; Wang, S.; Sunder, S.; Nicklaus, M. C.; Milne, G. W.; Proksa, B.; Pommier, Y. Depsides and depsidones as inhibitors of HIV-1 integrase: discovery of novel inhibitors through 3D database searching. J Med Chem 1997, 40, 942-51.

10) Nicklaus, M. C.; Neamati, N.; Hong, H.; Mazumder, A.; Sunder, S.; Chen, J.; Milne, G. W.; Pommier, Y. HIV-1 integrase pharmacophore: discovery of inhibitors through three- dimensional database searching. J Med Chem 1997, 40, 920-9.

11) Lubkowski, J.; Yang, F.; Alexandratos, J.; Wlodawer, A.; Zhao, H.; Burke, T. R., Jr.; Neamati, N.; Pommier, Y.; Merkel, G.; Skalka, A. M. Structure of the catalytic domain of avian sarcoma virus integrase with a bound HIV-1 integrase-targeted inhibitor. Proc Natl Acad Sci U S A 1998, 95, 4831-6.

12) Neamati, N.; Mazumder, A.; Zhao, H.; Sunder, S.; Burke, T. R., Jr.; Schultz, R. J.; Pommier, Y. Diarylsulfones, a novel class of human immunodeficiency virus type 1 integrase inhibitors. Antimicrob Agents Chemother 1997, 41, 385-93.

13) Neamati, N.; Mazumder, A.; Sunder, S.; Owen, J. M.; Schultz, R. J.; Pommier, Y. 2-Mercaptobenzenesulfonamides as novel class of human immunodeficiency type 1 (HIV-1) integrase and HIV-1 replication. Antimicrob Agents Chemother 1997, 8, 485-495.

14) Garofalo, A.; Balconi, G.; Botta, M.; Corelli, F.; D'Incalci, M.; Fabrizi, G.; Fiorini, I.; Lamba, D.; Nacci, V. Thioanalogues of anti-tumor antibiotics. II. Synthesis and preliminary in vitro cytotoxicity evaluation of tricyclic [1,4] benzothiazepine derivatives. Eur J Med Chem 1993, 29, 213-220.

15) Nacci, V.; Garofalo, A.; Anzini, M. Thioanalogues of antitumor antibiotics. I. Synthesis of 7,8-disubstituted 5,11-dioxo-1,2,3,11a-tetrahydro-5H,11H- and 5-oxo-2,3,11,11a-tetrahydro-1H,5H-pyrrolo[2,1-c][1,4]benzothiazepine. Farmaco 1989, 44, 423-433.

16) Nacci, V.; Garofalo, A.; Fiorini, I. Polycondensed heterocycles. II. A new preparative route to 11-oxo-5H,11H-pyrrolo[2,1-c][1,4]benzothiazepine. J Heterocycl Chem 1986, 23, 769-773.

17) Garofalo, A.; Nacci, V.; Corelli, F.; Campiani, G. Polycondensed heterocycles. V. Synthesis of 5H,11H-pyrrolo [2,1-c][1,4]benzothiazepine. Heterocycles 1990, 31, 1291-1300.

18) Garofalo, A.; Campiani, G.; Ciani, S. M.; Fiorini, I.; Nacci, V. Polycondensed heterocycles. IX. Pyrrolo[2,1-c] [1,4]benzothiazepines Synthesis of 3-(dimethylamino)methyl derivatives. Tetrahedron 1996, 52, 7745-7754.

19) Garofalo, A.; Campiani, G.; Nacci, V.; Fiorini, I. Polycondensed heterocycles. VIII. Synthesis of 11-aryl-5H,11H-pyrrolo[2,1-c][1,4] benzothiazepines by Pummerer rearrangement-cyclization reaction. Heterocycles 1992, 34, 51-60.

20) Archer, S.; Miller, J. K.; Rej, R.; Periana, C.; Fricker, L. Ring-hydroxylated analogues of lucantone as antitumor agents. J Med Chem 1982, 25, 220-227.

21) Katz, L.; Karger, L. S.; Schroeder, W.; Cohen, M. S. Hydrazine derivatives. I. Benzalthio- and bisbenzaldithiosalicylhydrazides. J Org Chem 1953, 18, 1380-1402.

22) Pendergast, W.; Dickerson, S. H.; Dev, I. K.; Ferone, R.; Duch, D. S.; Smith, G. K. Benzo[f]quinazoline inhibitors of thymidylate synthase: methyleneamino-linked aroylglutamate derivatives. J Med Chem 1994, 37, 838-844.

23) Guise, G. B.; Ollis, W. D.; Peacock, J. E.; Stephanatou, J. S.; Stoddart, J. F. Dithiosalicylides and trithiosalicylides. Their conformational behavior in solution. Tetrahedron Lett 1980, 21, 4203-4206.

24) Vitali, T.; Mossini, F.; Plazzi, P. V. Sintesi ed attivita' antifungine di bis-(3-carbossiamido-2-naftil)disolfuri e di nafto[3,2-d]isotiazolin-3-oni. Il Farmaco Ed Sc 1974, 29, 27-36.
25) Lalezari, I.; Schwartz, E. L. Synthesis and antineoplastic activity of 5-Aryl-2,3-dihydropyrrolo[2,1-b]thiazole-6,7-dimethanol 6,7-bis(isopropylcarbamates). J Med Chem 1988, 31, 1427-1429.
26) Falorni, M.; Conti, S.; Giacomelli, G.; Cossu, S.; F., S. Optically active 4-oxaproline derivatives: new useful chiral synthons derived from serine and threonine. Tetrahedron: Asymmetry 1995, 6, 287-294.
27) Goldgur, Y.; Dyda, F.; Hickman, A. B.; Jenkins, T. M.; Craigie, R.; Davies, D. R. Three new structures of the core domain of HIV-1 integrase: An active site that binds magnesium. Proc Natl Acad Sci U S A 1998, 95, 9150-4.
28) Fesen, M. R.; Pommier, Y.; Leteurtre, F.; Hiroguchi, S.; Yung, J.; Kohn, K. W. Inhibition of HIV-1 integrase by flavones, caffeic acid phenethyl ester (CAPE) and related compounds. Biochem Pharmacol 1994, 48, 595-608.
29) Clanton, D. J.; Buckheit, R. W., Jr.; Terpening, S. J.; Kiser, R.; Mongelli, N.; Borgia, A. L.; Schultz, R.; Narayanan, V.; Bader, J. P.; Rice, W. G. Novel sulfonated and phosphonated analogs of distamycin which inhibit the replication of HIV. Antiviral Res 1995, 27, 335-54.
30) Bader, J. P.; McMahon, J. B.; Schultz, R. J.; Narayanan, V. L.; Pierce, J. B.; Harrison, W. A.; Weislow, O. S.; Midelfort, C. F.; Stinson, S. F.; Boyd, M. R. Oxathiin carboxanilide, a potent inhibitor of human immunodeficiency virus reproduction. Proc Natl Acad Sci U S A 1991, 88, 6740-4.
31) Kageyama, S.; Mimoto, T.; Murakawa, Y.; Nomizu, M.; Ford, H., Jr.; Shirasaka, T.; Gulnik, S.; Erickson, J.; Takada, K.; Hayashi, H.; et al. In vitro anti-human immunodeficiency virus (HIV) activities of transition state mimetic HIV protease inhibitors containing allophenylnorstatine. Antimicrob Agents Chemother 1993, 37, 810-7.
32) Buckheit, R. W., Jr.; Roberson, J. L.; Lackman-Smith, C.; Wyatt, J. R.; Vickers, T. A.; Ecker, D. J. Potent and specific inhibition of HIV envelope-mediated cell fusion and virus binding by G quartet-forming oligonucleotide (ISIS 5320). AIDS Res Hum Retroviruses 1994, 10, 1497-506.
33) Rice, W. G.; Supko, J. G.; Malspeis, L.; Buckheit, R. W., Jr.; Clanton, D.; Bu, M.; Graham, L.; Schaeffer, C. A.; Turpin, J. A.; Domagala, J.; et al. Inhibitors of HIV nucleocapsid protein zinc fingers as candidates for the treatment of AIDS. Science 1995, 270, 1194-7.
34) Turpin, J. A.; Terpening, S. J.; Schaeffer, C. A.; Yu, G.; Glover, C. J.; Felsted, R. L.; Sausville, E. A.; Rice, W. G. Inhibitors of human immunodeficiency virus type 1 zinc fingers prevent normal processing of gag precursors and result in the release of noninfectious virus particles. J Virol 1996, 70, 6180-9.
35) Rice, W. G.; Baker, D. C.; Schaeffer, C. A.; Graham, L.; Bu, M.; Terpening, S.; Clanton, D.; Schultz, R.; Bader, J. P.; Buckheit, R. W., Jr.; Field, L.; Singh, P. K.; Turpin, J. A. Inhibition of multiple phases of human immunodeficiency virus type 1 replication by a dithiane compound that attacks the conserved zinc fingers of retroviral nucleocapsid proteins. Antimicrob Agents Chemother 1997, 41, 419-26.
36) Weislow, O. W.; Kiser, R.; Fine, D.; Bader, J.; Shoemaker, R. H.; Boyd, M. R. New soluble-formazan assay for HIV-1 cytopathic effects: application to high-flux screening of synthetic and natural products for AIDS antiviral activity. J Natl Cancer Inst 1989, 81, 577-586.

## Claims

**1.** Use of a compound selected from the group consisting of:

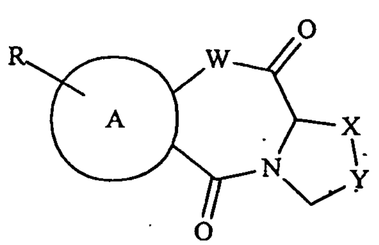

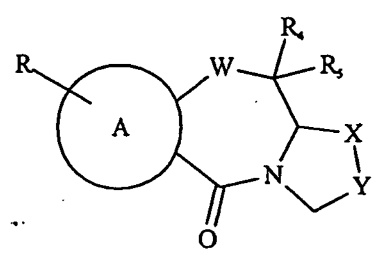

wherein

A is thiazole, benzene, naphthalene, pyridine, pyrimidine, pyrazine, or quinoline;
R is one or more of H, halogen, unsubstituted or substituted cyclic, branched or straight chain alkyl having from 1 to 5 carbon atoms, alkoxy having from 1 to 5 carbon atoms, $NO_2$, or carboxylic acid;
X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$ ;

$R_4$ is H or hydroxy;
$R_5$ is H, phenyl, or alkylamino; and
W is S or O

or wherein the compound is

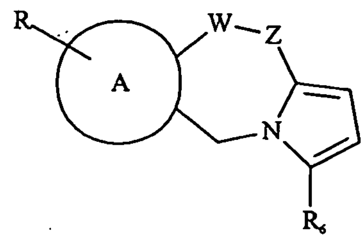

wherein

A is thiazole, benzene, naphthalene, pyridine, pyrimidine, pyrazine, or quinoline; and
R is one or more of H, $NO_2$, halogen, unsubstituted or substituted cyclic, branched or straight chain alkyl having from 1 to 5 carbon atoms, or carboxylic acid;
$R_6$ is H, unsubstituted cyclic, branched or straight chain alkyl or cyclic, branched or straight chain alkyl substituted with one or more of halogen, alkyl, alkoxy, alkylthio, trifluoromethyl, acyloxy, hydroxy, mercapto, carboxy, aryloxy, aryloxy, aryl, arylalkyl, hereroaryl, amino, alkylamino, dialkylamino, morpholino, piperidino, pyrrolidin-1-yl, or piperazin-1-yl or amino;
W is S or O; and
Z is S, O, $CH_2$, $CH_2CH_2$, or C=O, for the manufacture of a medicament for prophylactic or therapeutic treatment of HIV infection in a subject.

**2.** The use of Claim 1, wherein the compound is selected from the group consisting of:

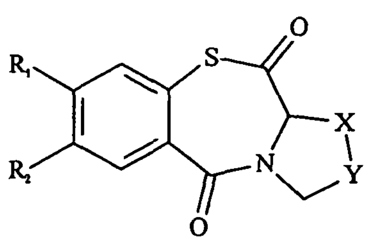

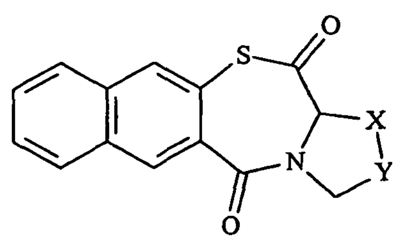

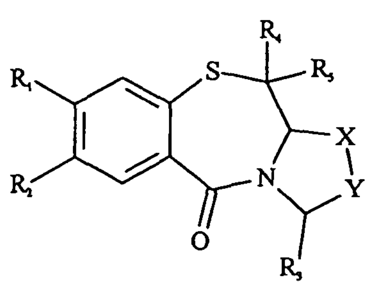

and

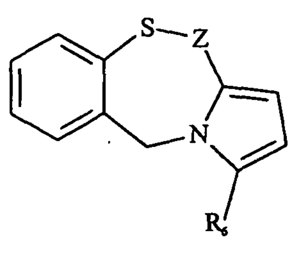

wherein

X-Y is $S-CH_2$, $CH_2-S$, $CH_2CH_2$ or $S(O)CH_2$;
$R_1$ is H, $NO_2$, or alkoxy having from 1 to 5 carbon atoms;
$R_2$ is H, Cl, Br, unsubstituted or substituted cyclic, branched or straight chain alkyl having from 1 to 5 carbon atoms, or alkoxy having from 1 to 5 carbon atoms;

$R_3$ and $R_4$ are H;
$R_5$ is $N(CH_2CH_2)_2NCH_3$; and
$R_6$ is H.

**3.** The use of Claim 1, wherein the compound is

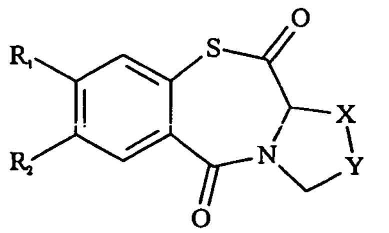

and

$R_1$ and $R_2$ are H, and X-Y is S-$CH_2$; or
$R_1$ is H, $R_2$ is Cl or Br or methyl, and X-Y is S-$CH_2$; or
$R_1$ is $NO_2$, $R_2$ is H, and X-Y is $CH_2$-S; or
$R_1$ and $R_2$ are methoxy, and X-Y is $CH_2$-S; or
$R_1$ is H, $R_2$ is methyl, and X-Y is S(O)-$CH_2$.

**4.** The use of Claim 1, wherein the compound is

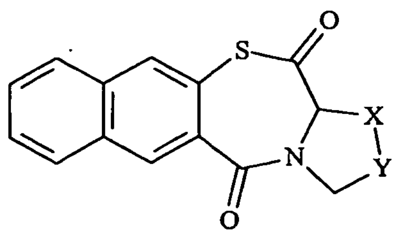

wherein X-Y is S-$CH_2$ or $CH_2$-S.

**5.** The use of Claim 1, wherein the compound is

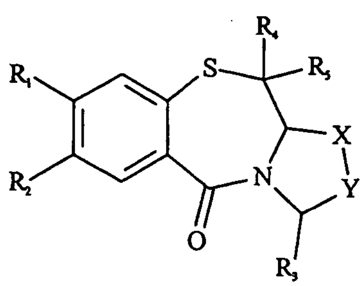

wherein

X-Y is $CH_2$-$CH_2$;
$R_1$, $R_2$, $R_3$ and $R_4$ are H; and
$R_5$ is $N(CH_2CH_2)_2NCH_3$.

**6.** The use of Claim 1, wherein the compound comprises

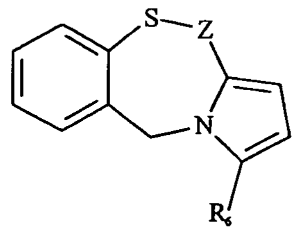

wherein $R_6$ is H and Z is C =O.

**7.** The use of Claim 1, wherein

A is thiazole, benzene, naphthalene, pyridine, pyrimidine, pyrazine, or quinoline;
R is one or more of halogen or $NO_2$;
X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$;
$R_4$ is H or hydroxy;
$R_5$ is H, phenyl, or alkylamino;
$R_6$ is H, or unsubstituted cyclic, branched or straight chain alkyl or cyclic, branched or straight chain alkyl substituted with one or more of halogen, alkyl, alkoxy, alkylthio, trifluoromethyl, acyloxy, hydroxy, mercapto, carboxy, aryloxy, aryloxy, aryl, arylalkyl, hereroaryl, amino, alkylamino, dialkylamino, morpholino, piperidino, pyrrolidin-1-yl, or piperazin-1-yl or amino; and
W is S or O.

**8.** The use of Claim 1, wherein the compound comprises

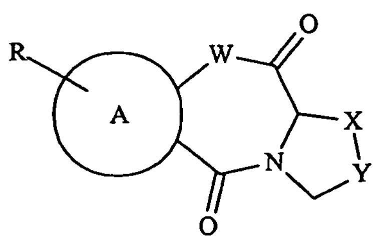

and

A is benzene or naphthalene
R is H, $NO_2$, or alkoxy having from 1 to 5 carbon atoms; and
X-Y is $CH_2$-S or S-$CH_2$.

**9.** The use of Claim 1, wherein the compound is selected from the group consisting of:

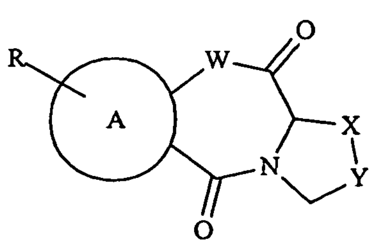
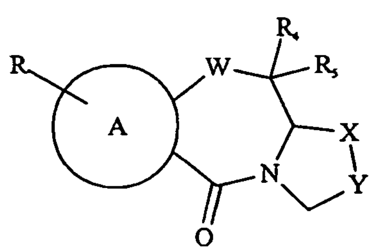

wherein X-Y is $CH_2$-S, S-$CH_2$, $CH_2$-O, or $CH_2$-$CH_2$, and W is S.

**10.** The use of Claim 9, wherein:

A is benzene, naphthalene, pyridine, pyrimidine, pyrazine, or quinoline.

**11.** The use of Claim 10, wherein A is benzene or naphthalene.

**12.** The use of Claim 11, wherein R is H, halogen, alkoxy having from 1 to 5 carbon atoms or $NO_2$.

**13.** The use of Claim 1, wherein the compound is:

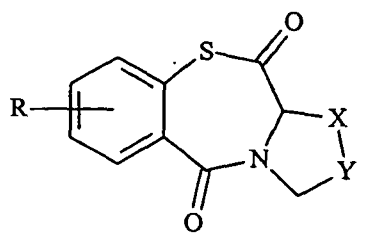

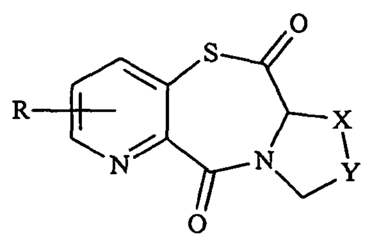

or

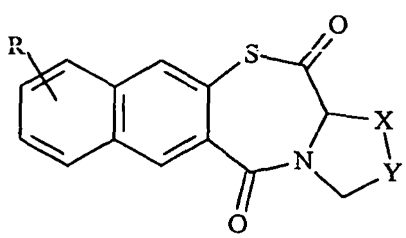

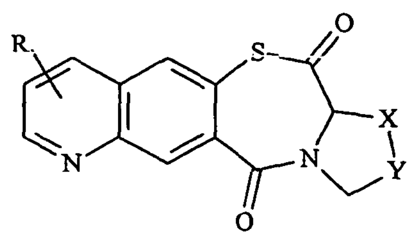

**14.** The use of Claim 1, wherein the compound is:

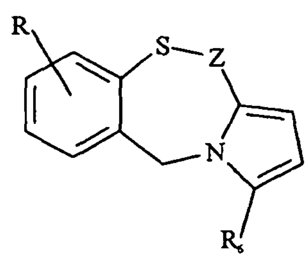

or

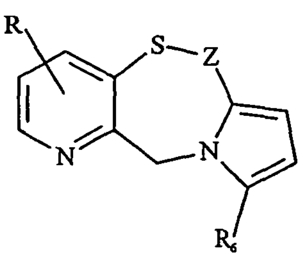

**15.** The use of Claim 13, wherein the compound is

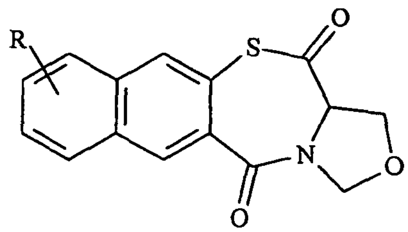

or

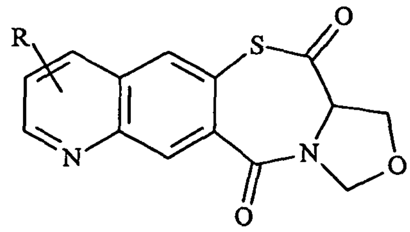

**16.** The use of Claim 1, wherein the compound is one of the following:

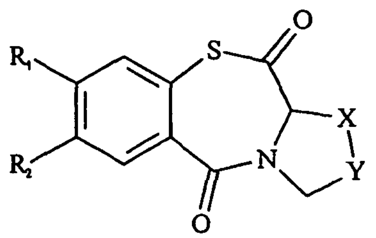

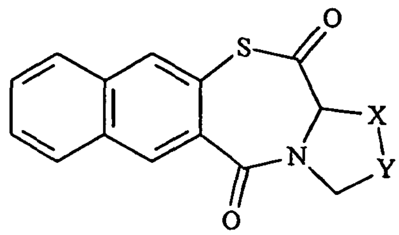

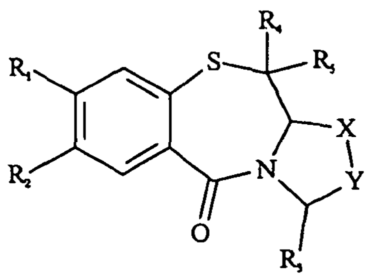

or

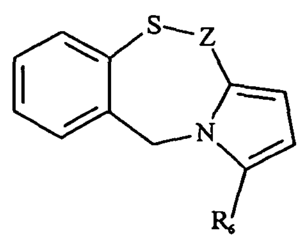

wherein

X-Y is S-$CH_2$, $CH_2$-S, $CH_2$-O, $CH_2$-$CH_2$, S(O)-$CH_2$, or $CH_2$-S(O);
$R_1$ and $R_2$ are independently selected from the group consisting of H, $NO_2$, halogen, unsubstituted or substituted cyclic, branched or straight chain alkyl having from 1 to 5 carbon atoms, or alkoxy having from 1 to 5 carbon atoms;
$R_3$ is H or phenyl;
$R_4$ is H or hydroxy;
$R_5$ is H, phenyl or alkylamino; and
$R_6$ is H, phenyl or alkylamino.

**17.** The use of Claim 16, wherein the alkylamine is $-N(CH_2CH_2)_2NCH_3$,$-CH_2NCH_2CH_3$, or $-CH_2N(CH_2CH_2)_2NCH_3$.

**18.** The use of Claim 14, wherein the compound is

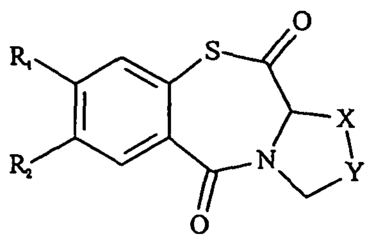

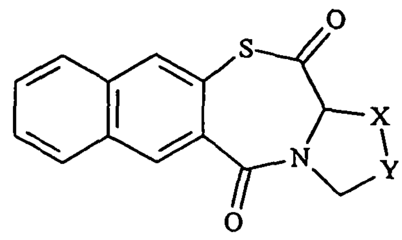

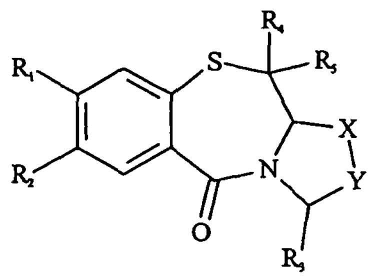

or

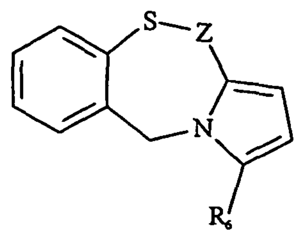

X-Y is $S-CH_2$, $CH_2-S$, or $CH_2-S(O)$;
and $R_1$ and $R_2$ are independently selected from the group consisting of H, $NO_2$, halogen, unsubstituted or substituted cyclic, branched or straight chain alkyl having from 1 to 5 carbon atoms and alkoxy having from 1 to 5 carbon atoms;
$R_3$ is H; and
$R_4$, $R_5$, and $R_6$ are H.

**19.** The use of Claim 1, wherein the compound is

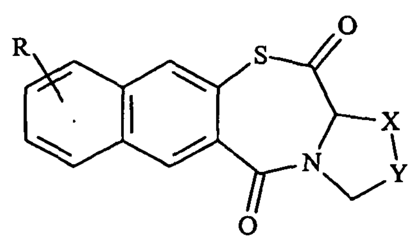

and X-Y is $S-CH_2$ or $CH_2-S$.

**20.** The use of Claim 19, wherein R is H.

**21.** The use of Claim 20, wherein X-Y is $S-CH_2$.

**22.** The use of Claim 16, wherein

$R_1$ is H, $NO_2$, or alkoxy having from 1 to 5 carbon atoms,
$R_2$ is H, Cl, Br, unsubstituted or substituted cyclic, branched or straight chain alkyl having from 1 to 5 carbon atoms, or alkoxy having from 1 to 5 carbon atoms;
$R_3$ and $R_4$ are H;
$R_5$ is $N(CH_2CH_2)_2NCH_3$; and
X-Y is $CH_2-S$, $S-CH_2$, or $CH_2-CH_2$.

**23.** The use of Claim 22, wherein the compound is

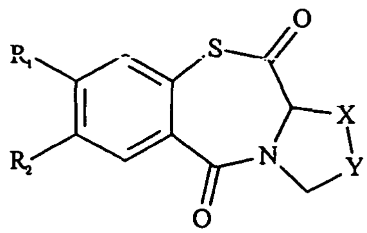

wherein

$R_1$ is H, $NO_2$, or methoxy;
$R_2$ is H, halogen or methoxy; and
X-Y is $CH_2$-S or S-$CH_2$.

**24.** The use of any preceding claim, wherein the treatment comprises inhibiting a HIV integrase by exposing the integrase to an integrase inhibiting amount of one or more of said compounds.

**25.** The use of any preceding claim, wherein the medicament is for administration in a therapeutically effective amount to the subject.

**26.** A compound selected from the group consisting of:

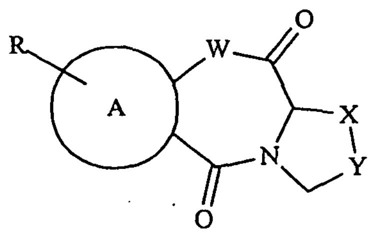

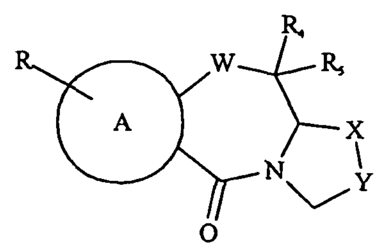

wherein

A is thiazole, benzene, naphthalene, pyridine, pyrimidine, pyrazine, or quinoline;
R is one or more of H, halogen, unsubstituted or substituted cyclic, branched or straight chain alkyl having from 1 to 5 carbon atoms, alkoxy having from 1 to 5 carbon atoms, $NO_2$, or carboxylic acid;
X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$;
$R_4$ is H or hydroxy;
$R_5$ is H, phenyl, or alkylamino; and
W is S or O; with the proviso that the compound is not selected from:

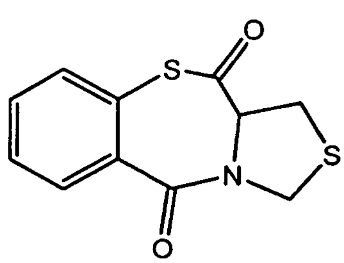

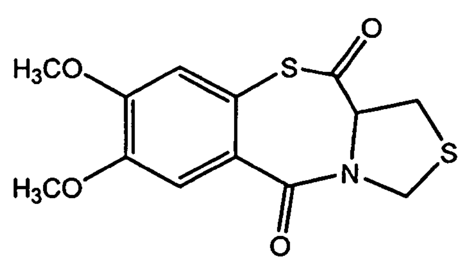

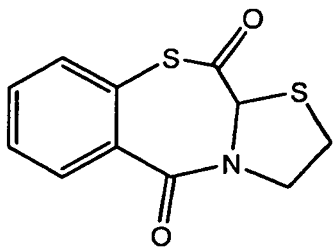

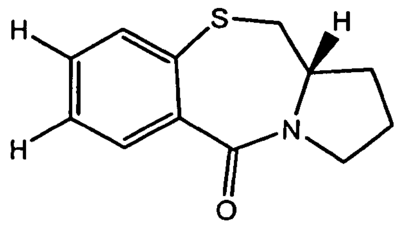

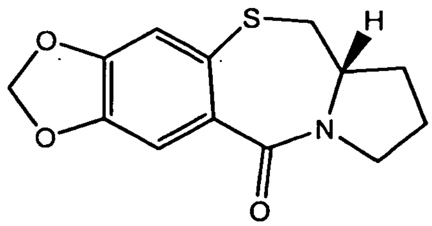

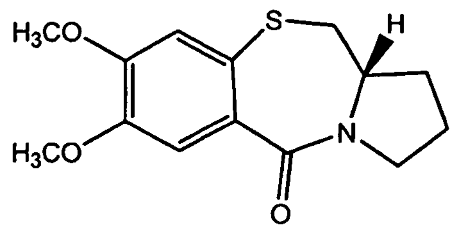

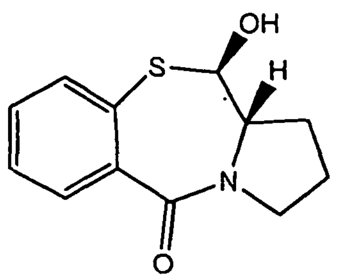

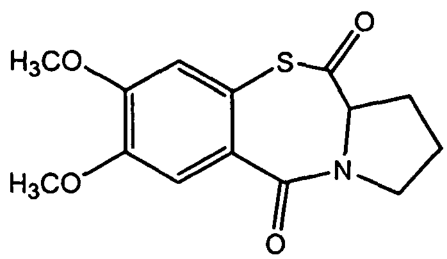

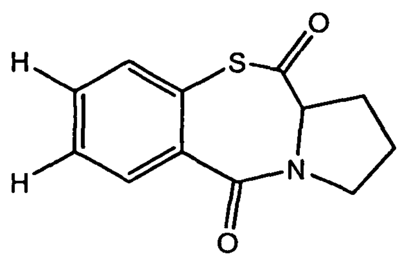

or wherein the compound is

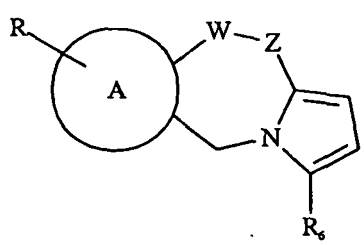

wherein

A is thiazole, benzene, naphthalene, pyridine, pyrimidine, pyrazine, or quinoline; and
R is one or more of H, $NO_2$, halogen, unsubstituted or substituted cyclic, branched or straight chain alkyl having from 1 to 5 carbon atoms, or carboxylic acid;
$R_6$ is H, unsubstituted cyclic, branched or straight chain alkyl, cyclic, branched or straight chain alkyl substituted with one or more of halogen, alkyl, alkoxy, alkylthio, trifluoromethyl, acyloxy, hydroxy, mercapto, carboxy, aryloxy, aryloxy, aryl, arylalkyl, hereroaryl, amino, alkylamino, dialkylamino, morpholino, piperidino, pyrrolidin-1-yl, or piperazin-1-yl or amino;
W is S; and
Z is S, O, $CH_2$, $CH_2CH_2$, or C=O

with the proviso that the compound is not

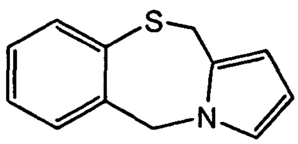
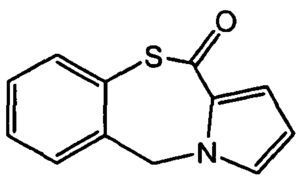
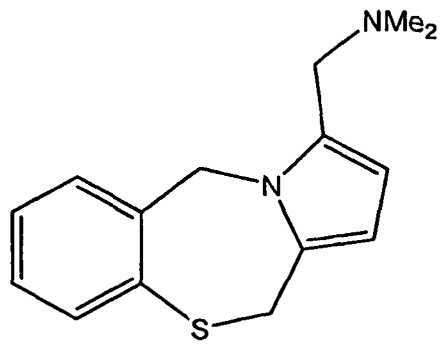

for prophylactic or therapeutic treatment of HIV infection in a subject.

**27.** The compound of Claim 26, wherein the compound is selected from the group consisting of:

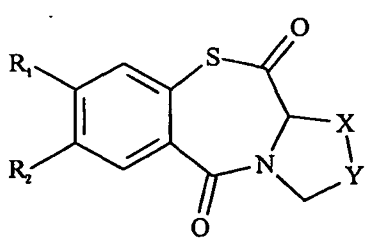
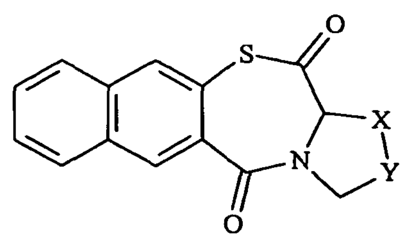

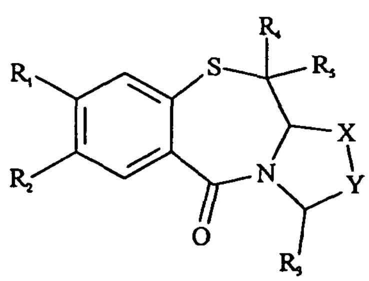
and
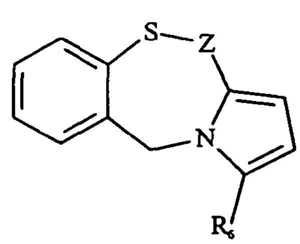

wherein

X-Y is S-$CH_2$, $CH_2$-S, $CH_2CH_2$ or S(O)$CH_2$;
$R_1$ is H, $NO_2$, or alkoxy having from 1 to 5 carbon atoms;
$R_2$ is H, Cl, Br, unsubstituted or substituted cyclic, branched or straight chain alkyl having from 1 to 5 carbon atoms, or alkoxy having from 1 to 5 carbon atoms;
$R_3$ and $R_4$ are H;
$R_5$ is N($CH_2CH_2$)$NCH_3$; and

$R_6$ is H.

**28.** The compound of Claim 26, wherein the compound is

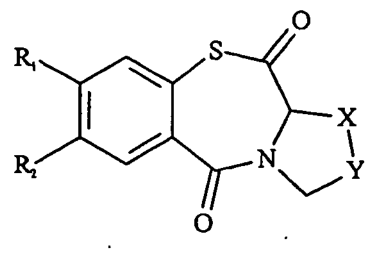

and

$R_1$ and $R_2$ are H, and X-Y is S-$CH_2$; or
$R_1$ is H, $R_2$ is Cl or Br or methyl, and X-Y is S-$CH_2$; or
$R_1$ is $NO_2$, $R_2$ is H, and X-Y is $CH_2$-S; or
$R_1$ and $R_2$ are methoxy, and X-Y is $CH_2$-S; or
$R_1$ is H, $R_2$ is methyl, and X-Y is S(O)-$CH_2$.

**29.** The compound of Claim 26, wherein the compound is

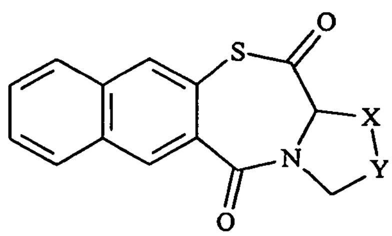

wherein X-Y is S-$CH_2$ or $CH_2$-S.

**30.** The compound of Claim 26, wherein the compound is

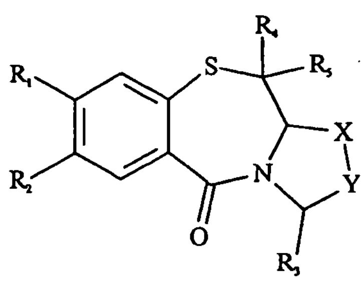

wherein

X-Y is $CH_2$-$CH_2$;
$R_1$, $R_2$, $R_3$ and $R_4$ are H; and
$R_5$ is $N(CH_2CH_2)_2NCH_3$.

**31.** The compound of Claim 26, wherein the compound comprises

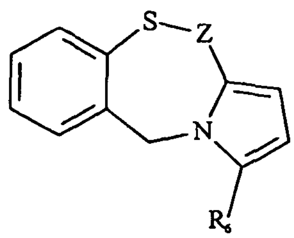

wherein $R_6$ is H and Z is C = O.

**32.** The compound of Claim 26, wherein

A is thiazole, benzene, naphthalene, pyridine, pyrimidine, pyrazine, or quinoline;
R is one or more of halogen or $NO_2$;
X-Y is $CH_2$-S, S-$CH_2$, $CH_2$-O, $CH_2$-S(O), S(O)-$CH_2$, $CH_2$-$CH_2$, $CH_2$-$CH_2$-$CH_2$, or $CH_2$-$CH_2$-$CH_2$-$CH_2$;
$R_4$ is H or hydroxy;
$R_5$ is H, phenyl, or alkylamino;
$R_6$ is H, or unsubstituted cyclic, branched or straight chain alkyl or cyclic, branched or straight chain alkyl substituted with one or more of halogen, unsubstituted or substituted cyclic, branched or straight chain alkyl having from 1 to 12 carbon atoms, alkoxy, alkylthio, trifluoromethyl, acyloxy, hydroxy, mercapto, carboxy, aryloxy, aryloxy, aryl, arylalkyl, hereroaryl, amino, alkylamino, dialkylamino, morpholino, piperidino, pyrrolidin-1-yl, or piperazin-1-yl or amino; and
W is S or O.

**33.** The compound of Claim 26, wherein the compound comprises

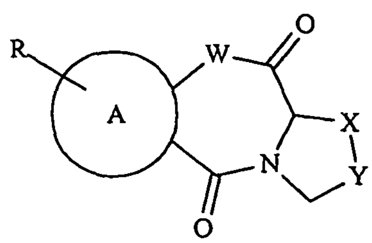

and

A is benzene or naphthalene;
R is H, $NO_2$, or alkoxy having from 1 to 5 carbon atoms; and
X-Y is $CH_2$-S or S-$CH_2$.

**34.** The compound of Claim 26, wherein the compound is selected from the group consisting of:

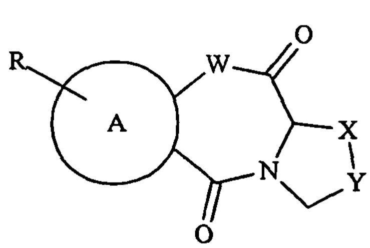 . 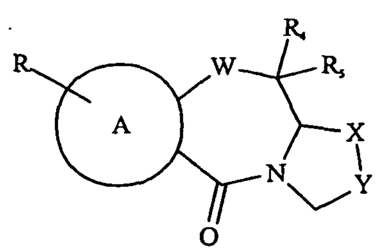

wherein X-Y is $CH_2$-S, S-$CH_2$, $CH_2$-O, or $CH_2$-$CH_2$, and W is S.

**35.** The compound of Claim 34, wherein:

A is benzene, naphthalene, pyridine, pyrimidine, pyrazine, or quinoline.

**36.** The compound of Claim 35, wherein A is benzene or naphthalene.

**37.** The compound of Claim 36, wherein R is H, halogen, alkoxy having from 1 to 5 carbon atoms or $NO_2$.

**38.** The compound of Claim 26, wherein the compound is:

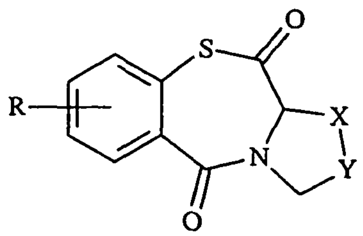

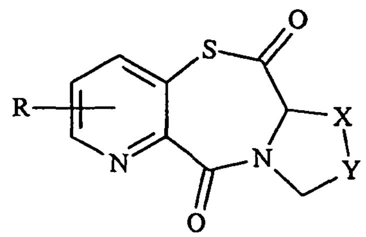

or

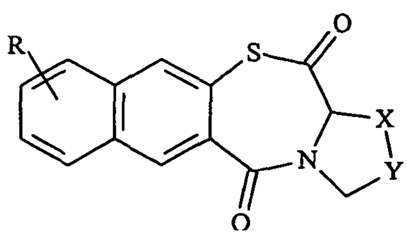

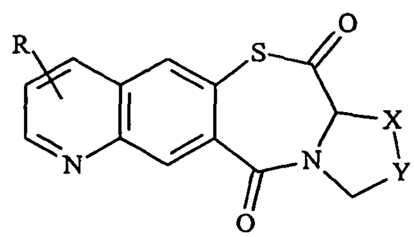

**39.** The compound of Claim 26, wherein the compound is:

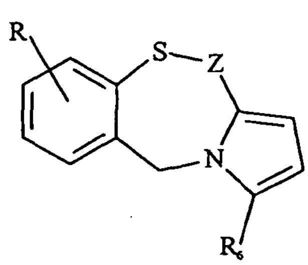

or

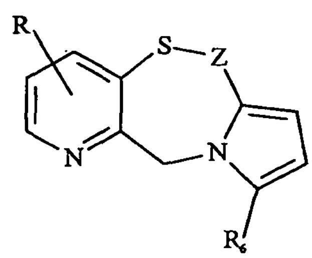

**40.** The compound of Claim 38, wherein the compound is

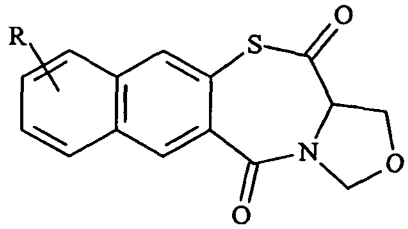

or

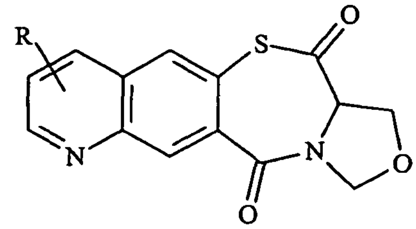

**41.** The compound of Claim 26, wherein the compound is one of the following:

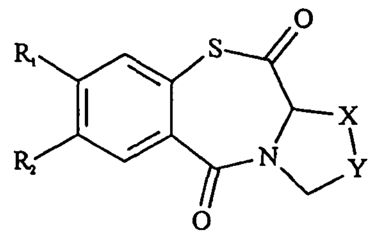

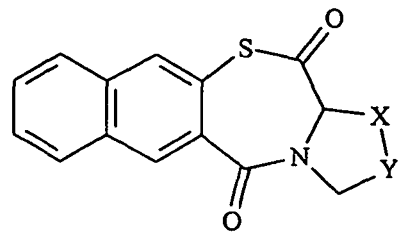

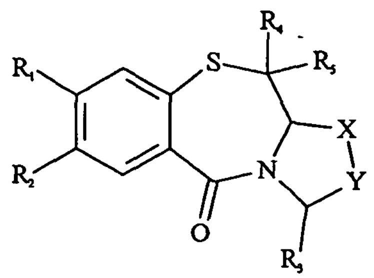

or

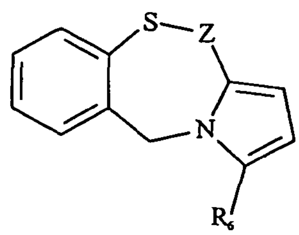

wherein

X-Y is S-$CH_2$, $CH_2$-S, $CH_2$-O, $CH_2$-$CH_2$, S(O)-$CH_2$, or $CH_2$-S(O);
$R_1$ and $R_2$ are independently selected from the group consisting of H, $NO_2$, halogen, unsubstituted or substituted cyclic, branched or straight chain alkyl having from 1 to 5 carbon atoms, or alkoxy having from 1 to 5 carbon atoms;
$R_3$ is H or phenyl;
$R_4$ is H or hydroxy;
$R_5$ is H, phenyl or alkylamino; and
$R_6$ is H, phenyl or alkylamino.

**42.** The compound of Claim 41, wherein the alkylamine is $-N(CH_2CH_2)_2NCH_3$,$-CH_2NCH_2CH_3$, or $-CH_2N(CH_2CH_2)_2NCH_3$.

**43.** The compound of Claim 39, wherein the compound is

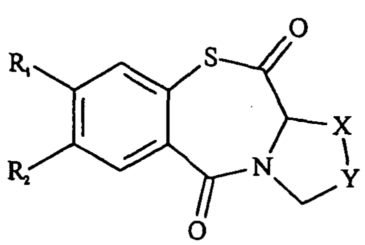

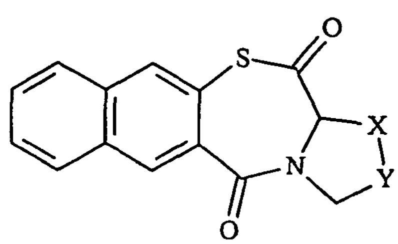

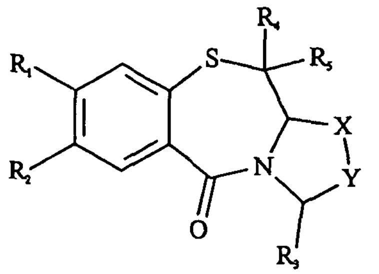

or

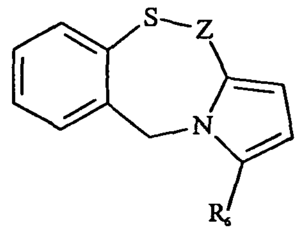

X-Y is $S\text{-}CH_2$, $CH_2\text{-}S$, or $CH_2\text{-}S(O)$;

and $R_1$ and $R_2$ are independently selected from the group consisting of H, $NO_2$, halogen, unsubstituted or substituted cyclic, branched or straight chain alkyl having from 1 to 5 carbon atoms and alkoxy having from 1 to 5 carbon atoms;

$R_3$ is H; and

$R_4$, $R_5$, and $R_6$ are H.

**44.** The compound of Claim 26, wherein the compound is

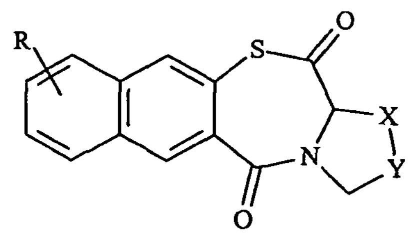

and X-Y is $S\text{-}CH_2$ or $CH_2\text{-}S$.

**45.** The compound of Claim 44, wherein R is H.

**46.** The compound of Claim 45, wherein X-Y is $S\text{-}CH_2$.

**47.** The compound of Claim 41, wherein

$R_1$ is H, $NO_2$, or alkoxy having from 1 to 5 carbon atoms,

$R_2$ is H, Cl, Br, unsubstituted or substituted cyclic, branched or straight chain alkyl having from 1 to 5 carbon atoms, or alkoxy having from 1 to 5 carbon atoms;

$R_3$ and $R_4$ are H;

$R_5$ is $N(CH_2CH_2)_2NCH_3$ ; and

X-Y is $CH_2\text{-}S$, $S\text{-}CH_2$, or $CH_2\text{-}CH_2$.

**48.** The compound of Claim 47, wherein the compound is

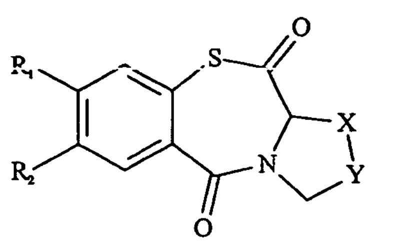

wherein

$R_1$ is H, $NO_2$, or methoxy;
$R_2$ is H, halogen or methoxy; and
X-Y is $CH_2$-S or S-$CH_2$.

**49.** The compound of any one of Claims 26 to 48, wherein the treatment comprises inhibiting a HIV integrase by exposing the integrase to an integrase inhibiting amount of one or more of said compounds.

**50.** A pharmaceutical composition comprising a compound according to any one of Claims 26 to 49.

**51.** An HIV integrase inhibitor compound having the following formula, or a pharmaceutically acceptable salt thereof:

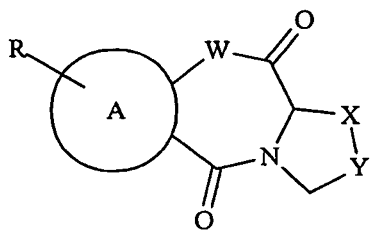

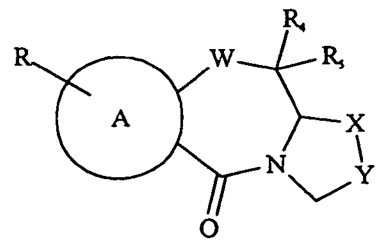

wherein

A is thiazole, benzene, naphthalene, pyridine, pyrimidine, pyrazine, or quinoline;
R is one or more of halogen or $NO_2$;
X-Y is $CH_2$-S, S-$CH_2$, $CH_2$-O, $CH_2$-S(O), S(O)-$CH_2$, $CH_2$-$CH_2$, CH,-$CH_2$-$CH_2$, or $CH_2$-$CH_2$-$CH_2$-$CH_2$;
$R_4$ is H or hydroxy;
$R_5$ is H, phenyl, or alkylamino; and
W is S or O

or wherein the compound is

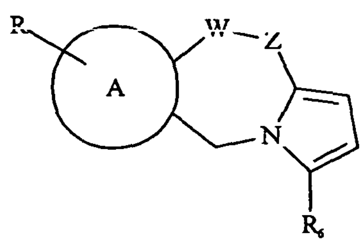

wherein

A is thiazole, benzene, naphthalene, pyridine, pyrimidine, pyrazine, or quinoline; and
R is one or more of halogen or $NO_2$;
$R_6$ is H, unsubstituted cyclic, branched or straight chain alkyl, cyclic, branched or straight chain alkyl substituted with one or more of halogen, unsubstituted or substituted cyclic, branched or straight chain alkyl, alkoxy, alkylthio, trifluoromethyl, acyloxy, hydroxy, mercapto, carboxy, aryloxy, aryloxy, aryl, arylalkyl, hereroaryl, amino, alkylamino, dialkylamino, morpholino, piperidino, pyrrolidin-1-yl, or piperazin-1-yl or amino;
W is S; and
Z is S, O, $CH_2$, $CH_2CH_2$, or C=O.

**52.** An HIV integrase inhibitor compound having the following formula, or a pharmaceutically acceptable salt thereof, wherein the compound is:

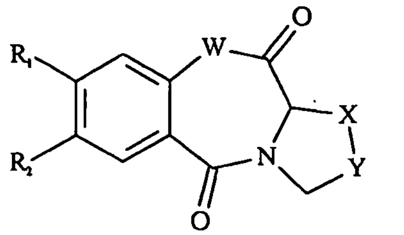

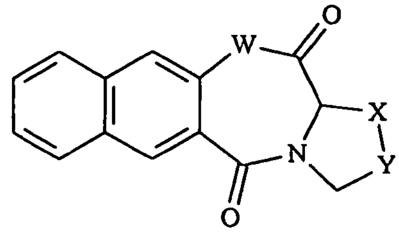

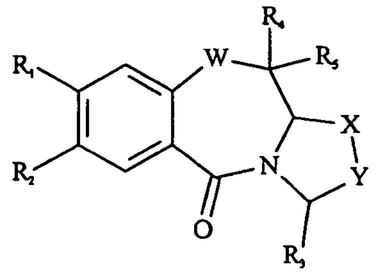

or

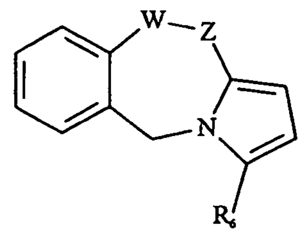

wherein

X-Y is S-$CH_2$, $CH_2$-S, S(O)-$CH_2$, $CH_2$-S(O), or $CH_2CH_2$;
W is S or O;
$R_1$ is H or $NO_2$;
$R_2$ is H, halogen, unsubstituted or substituted cyclic, branched or straight chain alkyl having from 1 to 5 carbon atoms or alkoxy having from 1 to 5 carbon atoms;
$R_3$ is H;
Z is S, O, $CH_2$, $CH_2CH_2$, or C = O;
$R_4$ is hydroxy or H;
$R_5$ is phenyl or $N(CH_2CH_2)_2NCH_3$; and
$R_6$ is $CH_2N(CH_2CH_2)_2NCH_3$,
provided that $R_1$ and $R_2$ are not both H or not both alkoxy.

**53.** The compound of Claim 52, wherein the compound is

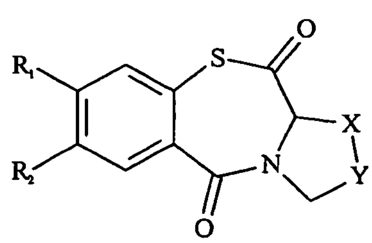

and $R_1$ is H or $NO_2$;
$R_2$ is H, halogen, unsubstituted or substituted cyclic, branched or straight chain, alkyl having from 1 to 5 carbon atoms or alkoxy having from 1 to 5 carbon atoms;
provided that $R_1$ and $R_2$ are not both H or not both alkoxy.

**54.** The compound of Claim 52, wherein

$R_1$ is H, $R_2$ is Cl, X-Y is S-$CH_2$; or
$R_1$ is H, $R_2$ is Br, X-Y is S-$CH_2$; or
$R_1$ is H, $R_2$ is $CH_3$, X-Y is S-$CH_2$; or

$R_1$ is H, $R_2$ is Cl, X-Y is $CH_2$-S; or
$R_1$ is H, $R_2$ is Br, X-Y is $CH_2$-S; or
$R_1$ is H, $R_2$ is $CH_3$, X-Y is $CH_2$-S; or
$R_1$ is $NO_2$, $R_2$ is H, X-Y is $CH_2$-S; or
$R_1$ is H, $R_2$ is $OCH_3$, X-Y is $CH_2$-S; or
$R_1$ is H, $R_2$ is $CH_3$, X-Y is S(O)-$CH_2$; or
$R_1$ is H, $R_2$ is Cl, X-Y is $CH_2$-S(O); or
$R_1$ is H, $R_2$ is $OCH_3$, X-Y is $CH_2$-S(O).

**55.** The compound of Claim 52, wherein the compound is

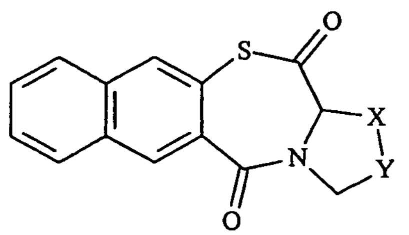

and X-Y is S-$CH_2$ or $CH_2$-S.

**56.** The compound of Claim 55, wherein X-Y is S-$CH_2$.

**57.** The compound of Claim 52, wherein the compound is

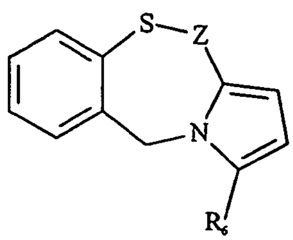

and $R_6$ is $CH_2N(CH_2CH)_2NCH_3$.

**58.** A compound having the formula

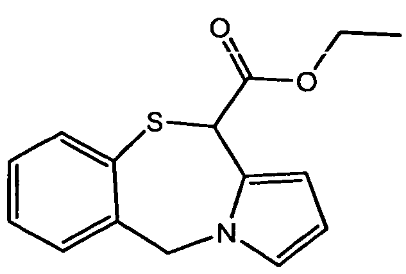

**59.** A compound according to Claim 58, for prophylactic or therapeutic treatment of HIV infection in a subject.

**60.** Use of a compound according to Claim 58, for the manufacture of a medicament for prophylactic or therapeutic treatment of HIV infection in a subject.

**Patentansprüche**

**1.** Verwendung einer Verbindung, die ausgewählt ist aus der Gruppe, bestehend aus:

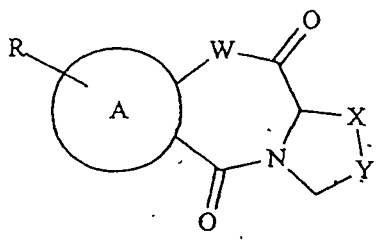

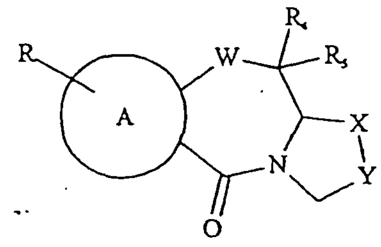

worin

A Thiazol, Benzol, Naphthalin, Pyridin, Pyrimidin, Pyrazin oder Chinolin ist;
R eines oder mehrere von H, Halogen, unsubstituiertem oder substituiertem cyclischen, verzweigten oder geradkettigen Alkyl mit von 1 bis 5 Kohlenstoffatomen, Alkoxy mit von 1 bis 5 Kohlenstoffatomen, $NO_2$ oder Carbonsäure ist;
X-Y $CH_2$-S, S-$CH_2$, $CH_2$-O, $CH_2$-S(O), S(O)-$CH_2$, $CH_2$-$CH_2$, $CH_2$-$CH_2$-$CH_2$ oder $CH_2$-$CH_2$-$CH_2$-$CH_2$ ist;
$R_4$ H oder Hydroxy ist;
$R_5$ H, Phenyl oder Alkylamino ist; und
W S oder O ist;

oder wobei die Verbindung

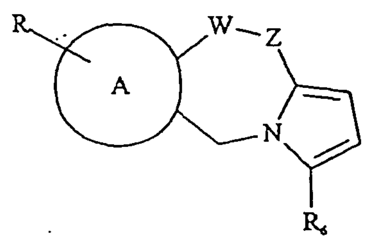

ist, worin

A Thiazol, Benzol, Naphthalin, Pyridin, Pyrimidin, Pyrazin oder Chinolin ist; und
R eines oder mehrere von H, $NO_2$, Halogen, unsubstituiertem oder substituiertem cyclischen, verzweigten oder geradkettigen Alkyl mit von 1 bis 5 Kohlenstoffatomen oder Carbonsäure ist;
$R_6$ H, unsubstituiertes cyclisches, verzweigtes oder geradkettiges Alkyl oder cyclisches, verzweigtes oder geradkettiges Alkyl, substituiert mit einem oder mehreren von Halogen, Alkyl, Alkoxy, Alkylthio, Trifluormethyl, Acyloxy, Hydroxy, Mercapto, Carboxy, Aryloxy, Aryloxy, Aryl, Arylalkyl, Heteroaryl, Amino, Alkylamino, Dialkylamino, Morpholino, Piperidino, Pyrrolidin-1-yl oder Piperazinyl-1-yl, oder Amino ist;
W S oder O ist; und
Z S, O, $CH_2$, $CH_2CH_2$ oder C=O ist,

zur Herstellung eines Arzneimittels zur prophylaktischen oder therapeutischen Behandlung von HIV-Infektion bei einem Patienten.

**2.** Verwendung nach Anspruch 1, **dadurch gekennzeichnet, daß** die Verbindung ausgewählt ist aus der Gruppe, bestehend aus:

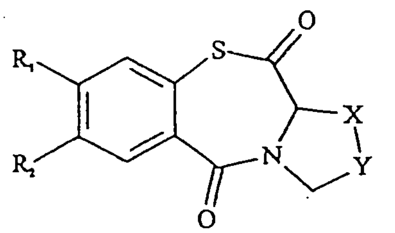

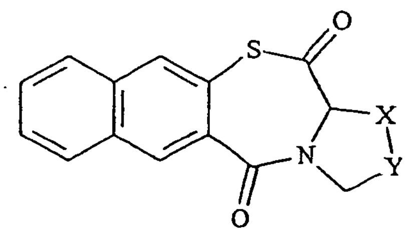

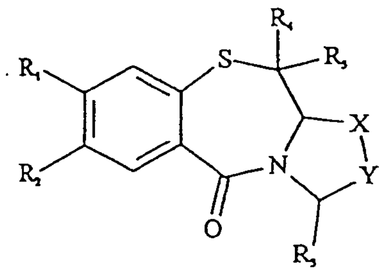

und

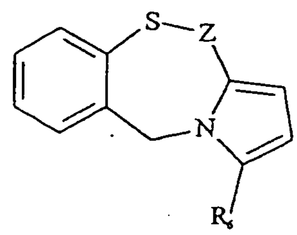

worin

X-Y $S\text{-}CH_2$, $CH_2\text{-}S$, $CH_2CH_2$ oder $S(O)CH_2$ ist;
$R_1$ H, $NO_2$ oder Alkoxy mit von 1 bis 5 Kohlenstoffatomen ist;
$R_2$ H, Cl, Br, unsubstituiertes oder substituiertes cyclisches, verzweigtes oder geradkettiges Alkyl mit von 1 bis 5 Kohlenstoffatomen oder Alkoxy mit von 1 bis 5 Kohlenstoffatomen ist;
$R_3$ und $R_4$ H sind;
$R_5$ $N(CH_2CH_2)_2NCH_3$ ist; und
$R_6$ H ist.

**3.** Verwendung nach Anspruch 1, **dadurch gekennzeichnet, daß** die Verbindung

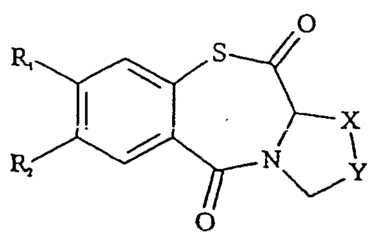

ist und

$R_1$ und $R_2$ H sind und X-Y $S\text{-}CH_2$ ist; oder
$R_1$ H ist, $R_2$ Cl oder Br oder Methyl ist und X-Y $S\text{-}CH_2$ ist; oder
$R_1$ $NO_2$ ist, $R_2$ H ist und X-Y $CH_2\text{-}S$ ist; oder
$R_1$ und $R_2$ Methoxy sind und X-Y $CH_2\text{-}S$ ist; oder
$R_1$ H ist, $R_2$ Methyl ist und X-Y $S(O)\text{-}CH_2$ ist.

**4.** Verwendung nach Anspruch 1, **dadurch gekennzeichnet, daß** die Verbindung

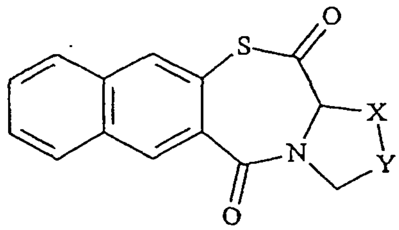

ist, worin X-Y S-$CH_2$ oder $CH_2$-S ist.

**5.** Verwendung nach Anspruch 1, **dadurch gekennzeichnet, daß** die Verbindung

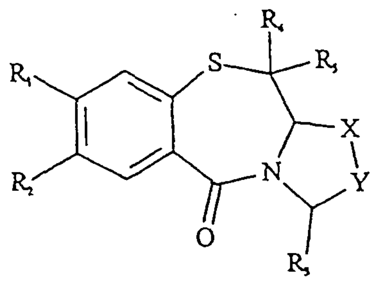

ist,

worin X-Y $CH_2$-$CH_2$ ist;
$R_1$, $R_2$, $R_3$ und $R_4$ H sind; und
$R_5$ $N(CH_2CH_2)_2NCH_3$ ist.

**6.** Verwendung nach Anspruch 1, **dadurch gekennzeichnet, daß** die Verbindung

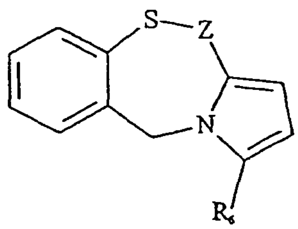

umfaßt, worin $R_6$ H ist und Z C=O ist.

**7.** Verwendung nach Anspruch 1, **dadurch gekennzeichnet, daß**

A Thiazol, Benzol, Naphthalin, Pyridin, Pyrimidin, Pyrazin oder Chinolin ist;
R eines oder mehrere von Halogen oder $NO_2$ ist;
X-Y $CH_2$-S, S-$CH_2$, $CH_2$-O, $CH_2$-S(O), S(O)-$CH_2$, $CH_2$-$CH_2$, $CH_2$-$CH_2$-$CH_2$ oder $CH_2$-$CH_2$-$CH_2$-$CH_2$ ist;
$R_4$ H oder Hydroxy ist;
$R_5$ H, Phenyl oder Alkylamino ist;
$R_6$ H ist oder unsubstituiertes cyclisches, verzweigtes oder geradkettiges Alkyl oder cyclisches, verzweigtes oder geradkettiges Alkyl, substituiert mit einem oder mehreren von Halogen, Alkyl, Alkoxy, Alkylthio, Trifluormethyl, Acyloxy, Hydroxy, Mercapto, Carboxy, Aryloxy, Aryloxy, Aryl, Arylalkyl, Heteroaryl, Amino, Alkylamino, Dialkylamino, Morpholino, Piperidino, Pyrrolidin-1-yl oder Piperazin-1-yl, oder Amino ist; und
W S oder O ist.

**8.** Verwendung nach Anspruch 1, **dadurch gekennzeichnet, daß** die Verbindung

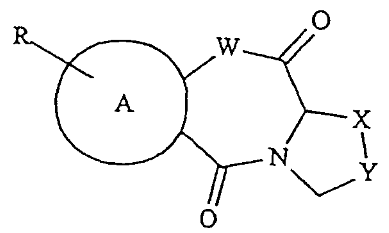

umfaßt und

A Benzol oder Naphthalin ist;
R H, $NO_2$ oder Alkoxy mit von 1 bis 5 Kohlenstoffatomen ist; und
X-Y $CH_2$-S oder S-$CH_2$ ist.

**9.** Verwendung nach Anspruch 1, **dadurch gekennzeichnet, daß** die Verbindung ausgewählt ist aus der Gruppe, bestehend aus:

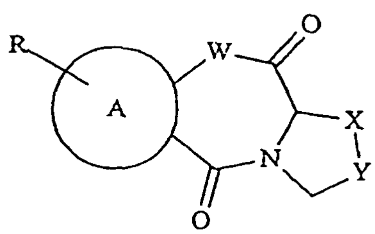

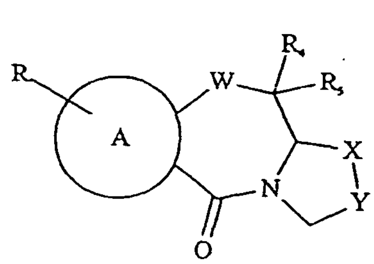

worin X-Y $CH_2$-S, S-$CH_2$, $CH_2$-O oder $CH_2$-$CH_2$ ist und W S ist.

**10.** Verwendung nach Anspruch 9, **dadurch gekennzeichnet, daß**:

A Benzol, Naphthalin, Pyridin, Pyrimidin, Pyrazin oder Chinolin ist.

**11.** Verwendung nach Anspruch 10, **dadurch gekennzeichnet, daß** A Benzol oder Naphthalin ist.

**12.** Verwendung nach Anspruch 11, **dadurch gekennzeichnet, daß** R H, Halogen, Alkoxy mit von 1 bis 5 Kohlenstoffatomen oder $NO_2$ ist.

**13.** Verwendung nach Anspruch 1, **dadurch gekennzeichnet, daß** die Verbindung:

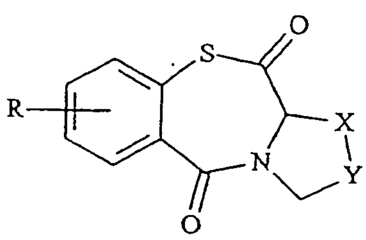

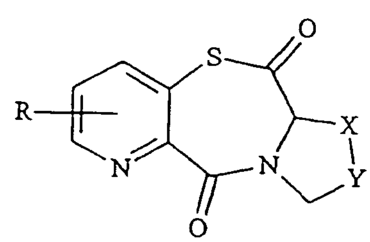

oder

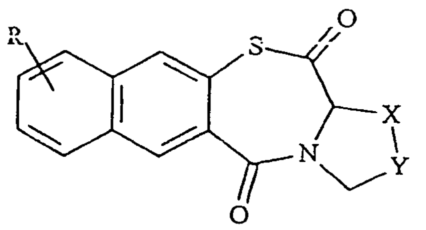
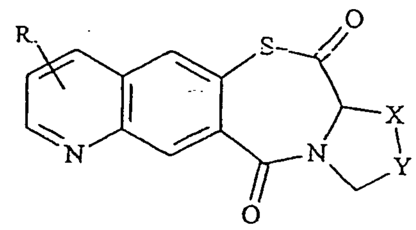

ist.

**14.** Verwendung nach Anspruch 1, **dadurch gekennzeichnet, daß** die Verbindung:

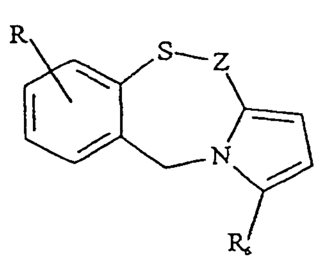
oder
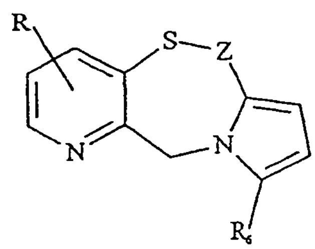

ist.

**15.** Verwendung nach Anspruch 13, **dadurch gekennzeichnet, daß** die Verbindung

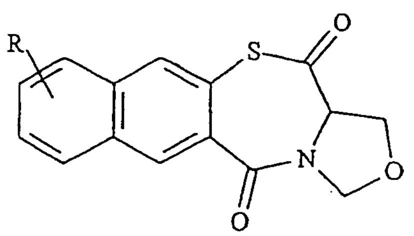
oder
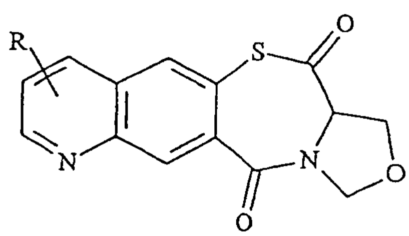

ist.

**16.** Verwendung nach Anspruch 1, **dadurch gekennzeichnet, daß** die Verbindung eine der folgenden ist:

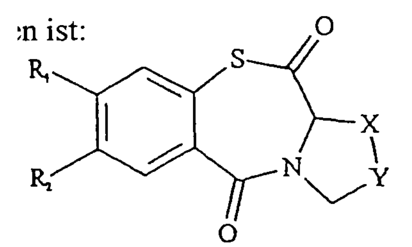
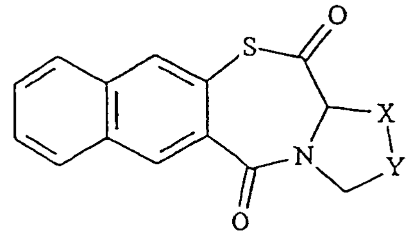

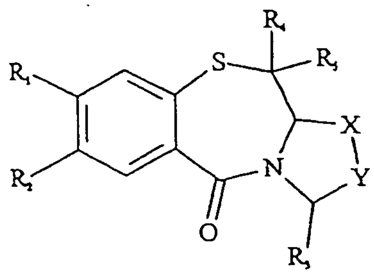

oder

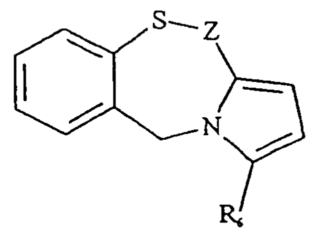

worin

X-Y S-$CH_2$, $CH_2$-S, $CH_2$-O, $CH_2$-$CH_2$, S(O)-$CH_2$ oder $CH_2$-S(O) ist;
$R_1$ und $R_2$ unabhängig ausgewählt sind aus der Gruppe, bestehend aus H, $NO_2$, Halogen, unsubstituiertem oder substituiertem cyclischen, verzweigten oder geradkettigen Alkyl mit von 1 bis 5 Kohlenstoffatomen oder Alkoxy mit von 1 bis 5 Kohlenstoffatomen;
$R_3$ H oder Phenyl ist;
$R_4$ H oder Hydroxy ist;
$R_5$ H, Phenyl oder Alkylamino ist; und
$R_6$ H, Phenyl oder Alkylamino ist.

**17.** Verwendung nach Anspruch 16, **dadurch gekennzeichnet, daß** das Alkylamin -$N(CH_2CH_2)_2NCH_3$, -$CH_2NCH_2CH_3$ oder -$CH_2N(CH_2CH_2)_2NCH_3$ ist.

**18.** Verwendung nach Anspruch 14, **dadurch gekennzeichnet, daß** die Verbindung

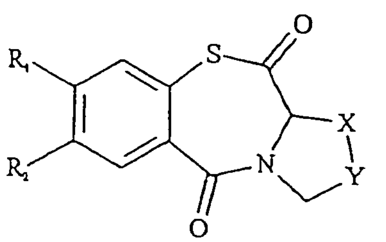

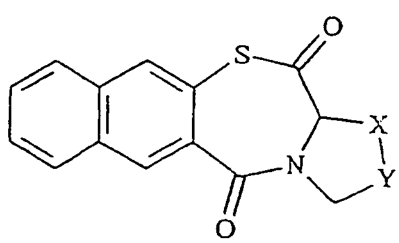

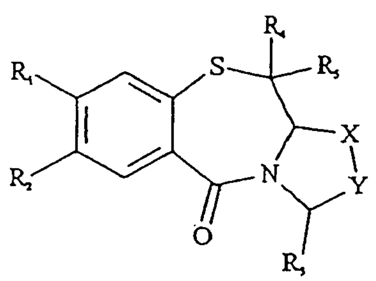

oder

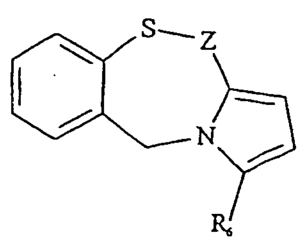

ist, worin

X-Y S-$CH_2$, $CH_2$-S oder $CH_2$-S(O) ist;
und $R_1$ und $R_2$ unabhängig ausgewählt sind aus der Gruppe, bestehend aus H, $NO_2$, Halogen, unsubstituiertem oder substituiertem cyclischen, verzweigten oder geradkettigen Alkyl mit von 1 bis 5 Kohlenstoffatomen und Alkoxy mit von 1 bis 5 Kohlenstoffatomen;
$R_3$ H ist; und
$R_4$, $R_5$ und $R_6$ H sind.

**19.** Verwendung nach Anspruch 1, **dadurch gekennzeichnet, daß** die Verbindung

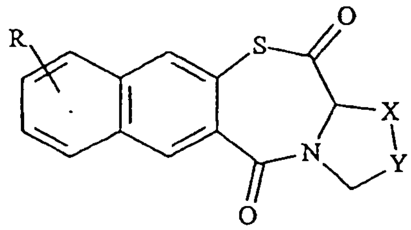

ist und X-Y S-$CH_2$ oder $CH_2$-S ist.

**20.** Verwendung nach Anspruch 19, **dadurch gekennzeichnet, daß** R H ist.

**21.** Verwendung nach Anspruch 20, **dadurch gekennzeichnet, daß** X-Y S-$CH_2$ ist.

**22.** Verwendung nach Anspruch 16, **dadurch gekennzeichnet, daß**

$R_1$ H, $NO_2$ oder Alkoxy mit von 1 bis 5 Kohlenstoffatomen ist;
$R_2$ H, Cl, Br, unsubstituiertes oder substituiertes cyclisches, verzweigtes oder geradkettiges Alkyl mit von 1 bis 5 Kohlenstoffatomen oder Alkoxy mit von 1 bis 5 Kohlenstoffatomen ist;
$R_3$ und $R_4$ H sind;
$R_5$ $N(CH_2CH_2)_2NCH_3$ ist; und
X-Y $CH_2$-S, S-$CH_2$ oder $CH_2$-$CH_2$ ist.

**23.** Verwendung nach Anspruch 22, **dadurch gekennzeichnet, daß** die Verbindung

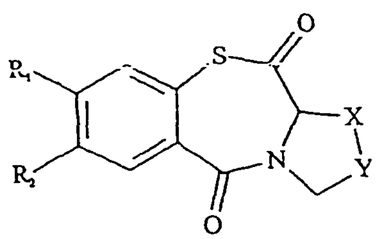

ist,

worin $R_1$ H, $NO_2$ oder Methoxy ist;
$R_2$ H, Halogen oder Methoxy ist; und
X-Y $CH_2$-S oder S-$CH_2$ ist.

**24.** Verwendung nach irgendeinem vorangehenden Anspruch, **dadurch gekennzeichnet, daß** die Behandlung die Hemmung einer HIV-Integrase umfaßt, indem die Integrase einer Integrase-hemmenden Menge einer oder mehrerer von besagten Verbindungen ausgesetzt wird.

**25.** Verwendung nach irgendeinem vorangehenden Anspruch, **dadurch gekennzeichnet, daß** das Arzneimittel zur Verabreichung in einer therapeutisch wirksamen Menge an den Patienten ist.

**26.** Verbindung, ausgewählt aus der Gruppe, bestehend aus:

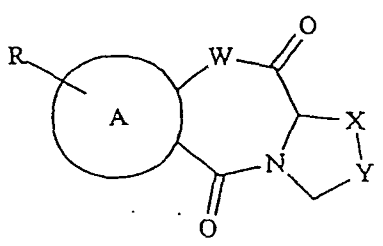

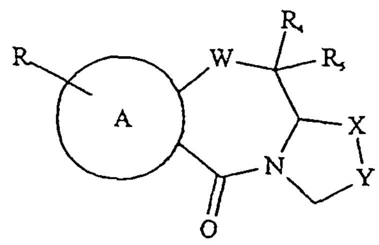

worin

A Thiazol, Benzol, Naphthalin, Pyridin, Pyrimidin, Pyrazin oder Chinolin ist;
R eines oder mehrere von H, Halogen, unsubstituiertem oder substituiertem cyclischen, verzweigten oder geradkettigen Alkyl mit von 1 bis 5 Kohlenstoffatomen, Alkoxy mit von 1 bis 5 Kohlenstoffatomen, $NO_2$ oder Carbonsäure ist;
X-Y $CH_2$-S, S-$CH_2$, $CH_2$-O, $CH_2$-S(O), S(O)-$CH_2$, $CH_2$-$CH_2$, $CH_2$-$CH_2$-$CH_2$ oder $CH_2$-$CH_2$-$CH_2$-$CH_2$ ist;
$R_4$ H oder Hydroxy ist;
$R_5$ H, Phenyl oder Alkylamino ist; und
W S oder O ist; mit der Maßgabe, daß die Verbindung nicht ausgewählt ist aus:

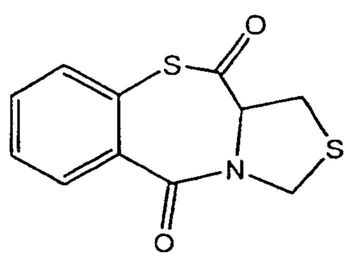

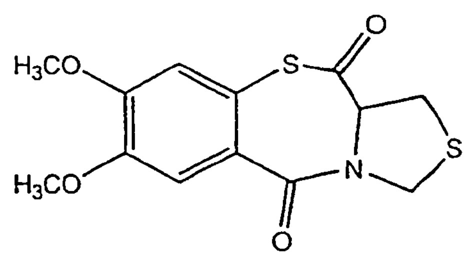

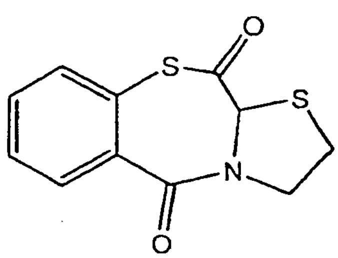

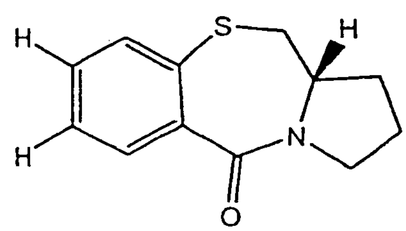

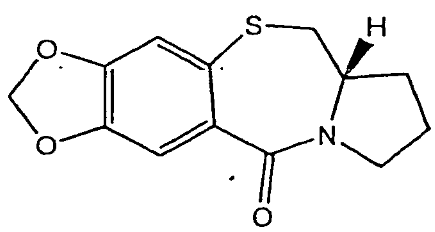

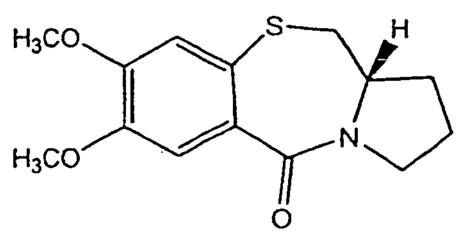

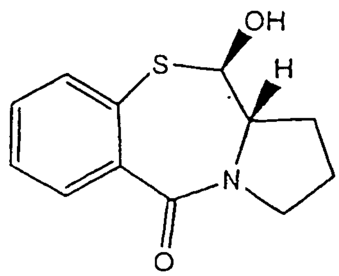

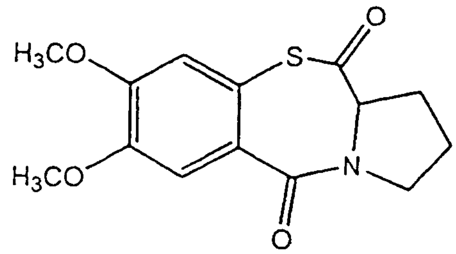

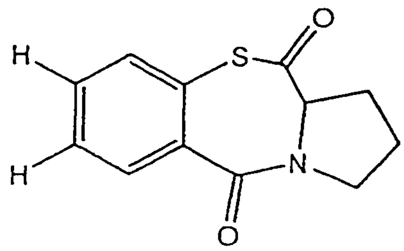

oder wobei die Verbindung

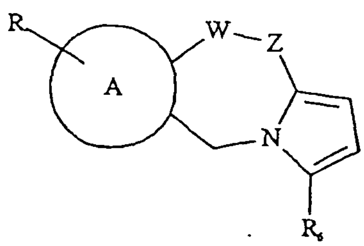

ist,
worin

A Thiazol, Benzol, Naphthalin, Pyridin, Pyrimidin, Pyrazin oder Chinolin ist; und
R eines oder mehrere von H, $NO_2$, Halogen, unsubstituiertem oder substituiertem cyclischen, verzweigten oder geradkettigen Alkyl mit von 1 bis 5 Kohlenstoffatomen oder Carbonsäure ist;
$R_6$ H, unsubstituiertes cyclisches, verzweigtes oder geradkettiges Alkyl oder cyclisches, verzweigtes oder geradkettiges Alkyl, substituiert mit einem oder mehreren von Halogen, Alkyl, Alkoxy, Alkylthio, Trifluormethyl, Acyloxy, Hydroxy, Mercapto, Carboxy, Aryloxy, Aryloxy, Aryl, Arylalkyl, Heteroaryl, Amino, Alkylamino, Dialkylamino, Morpholino, Piperidino, Pyrrolidin-1-yl oder Piperazinyl-1-yl, oder Amino ist;
W S ist; und
Z S, O, $CH_2$, $CH_2CH_2$ oder C=O ist,

mit der Maßgabe, daß die Verbindung nicht

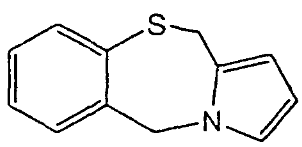
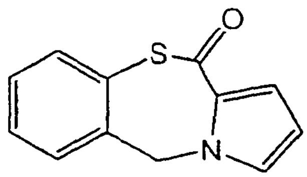
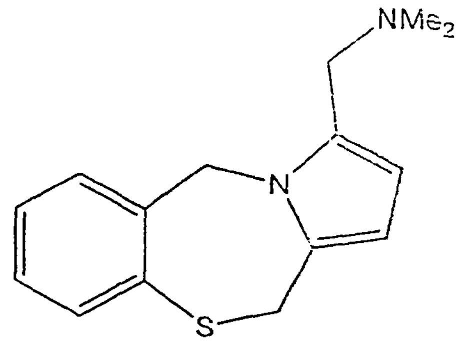

ist, zur prophylaktischen oder therapeutischen Behandlung von HIV-Infektion bei einem Patienten.

**27.** Verbindung nach Anspruch 26, **dadurch gekennzeichnet, daß** die Verbindung ausgewählt ist aus der Gruppe, bestehend aus:

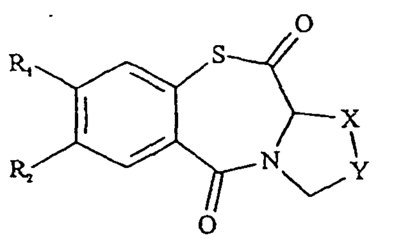
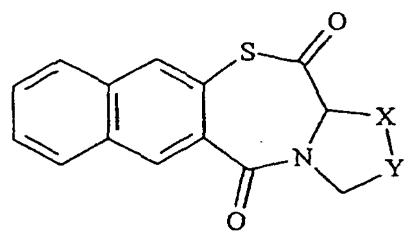

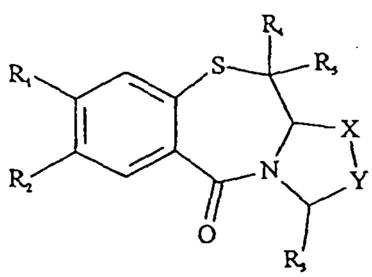
und
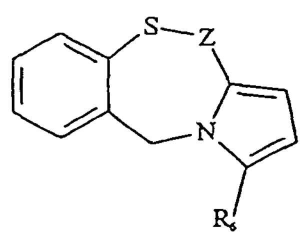

worin

X-Y S-$CH_2$, $CH_2$-S, $CH_2CH_2$ oder S(O)$CH_2$ ist;
$R_1$ H, $NO_2$ oder Alkoxy mit von 1 bis 5 Kohlenstoffatomen ist;
$R_2$ H, Cl, Br, unsubstituiertes oder substituiertes cyclisches, verzweigtes oder geradkettiges Alkyl mit von 1 bis 5 Kohlenstoffatomen oder Alkoxy mit von 1 bis 5 Kohlenstoffatomen ist;
$R_3$ und $R_4$ H sind;
$R_5$ $N(CH_2CH_2)_2NCH_3$ ist; und
$R_6$ H ist.

**28.** Verbindung nach Anspruch 26, **dadurch gekennzeichnet, daß** die Verbindung

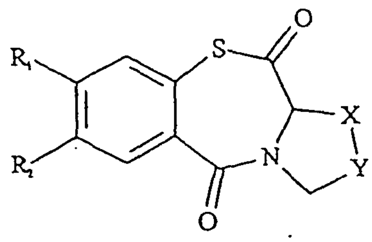

ist und

$R_1$ und $R_2$ H sind und X-Y S-$CH_2$ ist; oder
$R_1$ H ist, $R_2$ Cl oder Br oder Methyl ist und X-Y S-$CH_2$ ist; oder
$R_1$ $NO_2$ ist, $R_2$ H ist und X-Y $CH_2$-S ist; oder
$R_1$ und $R_2$ Methoxy sind und X-Y $CH_2$-S ist; oder
$R_1$ H ist, $R_2$ Methyl ist und X-Y S(O)-$CH_2$ ist.

**29.** Verbindung nach Anspruch 26, **dadurch gekennzeichnet, daß** die Verbindung

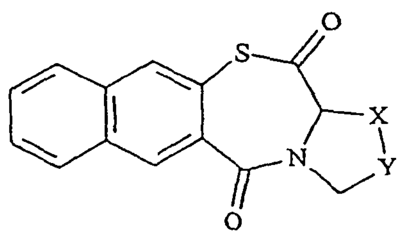

ist, worin X-Y S-$CH_2$ oder $CH_2$-S ist.

**30.** Verbindung nach Anspruch 26, daß die Verbindung

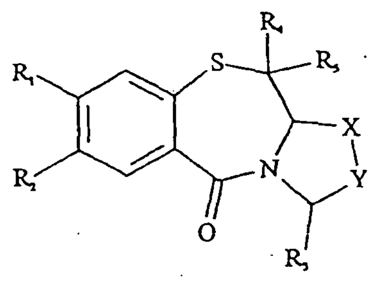

ist,
worin

X-Y $CH_2$-$CH_2$ ist;
$R_1$, $R_2$, $R_3$ und $R_4$ H sind; und
$R_5$ $N(CH_2CH_2)_2NCH_3$ ist.

**31.** Verbindung nach Anspruch 26, **dadurch gekennzeichnet, daß** die Verbindung

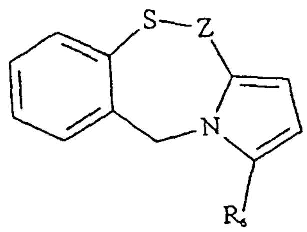

umfaßt, worin $R_6$ H ist und Z C=O ist.

**32.** Verbindung nach Anspruch 26, **dadurch gekennzeichnet, daß**

A Thiazol, Benzol, Naphthalin, Pyridin, Pyrimidin, Pyrazin oder Chinolin ist;
R eines oder mehrere von Halogen oder $NO_2$ ist;
X-Y $CH_2$-S, S-$CH_2$, $CH_2$-O, $CH_2$-S(O), S(O)-$CH_2$, $CH_2$-$CH_2$, $CH_2$-$CH_2$-$CH_2$ oder $CH_2$-$CH_2$-$CH_2$-$CH_2$ ist;
$R_4$ H oder Hydroxy ist;
$R_5$ H, Phenyl oder Alkylamino ist;
$R_6$ H oder unsubstituiertes cyclisches, verzweigtes oder geradkettiges Alkyl oder cyclisches, verzweigtes oder geradkettiges Alkyl, substituiert mit einem oder mehreren von Halogen, unsubstituiertem oder substituiertem cyclischen, verzweigten oder geradkettigen Alkyl mit von 1 bis 12 Kohlenstoffatomen, Alkoxy, Alkylthio, Trifluormethyl, Acyloxy, Hydroxy, Mercapto, Carboxy, Aryloxy, Aryloxy, Aryl, Arylalkyl, Heteroaryl, Amino, Alkylamino, Dialkylamino, Morpholino, Piperidino, Pyrrolidin-1-yl oder Piperazin-1-yl, oder Amino ist; und
W S oder O ist.

**33.** Verbindung nach Anspruch 26, **dadurch gekennzeichnet, daß** die Verbindung

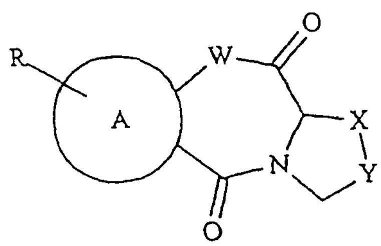

umfaßt und

A Benzol oder Naphthalin ist;
R H, $NO_2$ oder Alkoxy mit von 1 bis 5 Kohlenstoffatomen ist; und
X-Y $CH_2$-S oder S-$CH_2$ ist.

**34.** Verbindung nach Anspruch 26, **dadurch gekennzeichnet, daß** die Verbindung ausgewählt ist aus der Gruppe, bestehend aus:

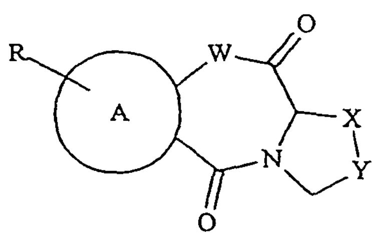

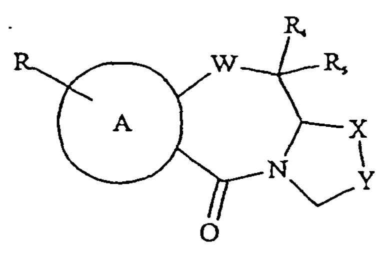

worin X-Y $CH_2$-S, S-$CH_2$, $CH_2$-O oder $CH_2$-$CH_2$ ist und W S ist.

**35.** Verbindung nach Anspruch 34, **dadurch gekennzeichnet, daß**:

A Benzol, Naphthalin, Pyridin, Pyrimidin, Pyrazin oder Chinolin ist.

**36.** Verbindung nach Anspruch 35, **dadurch gekennzeichnet, daß** A Benzol oder Naphthalin ist.

**37.** Verbindung nach Anspruch 36, **dadurch gekennzeichnet, daß** R H, Halogen, Alkoxy mit von 1 bis 5 Kohlenstoffatomen oder $NO_2$ ist.

**38.** Verbindung nach Anspruch 26, **dadurch gekennzeichnet, daß** die Verbindung:

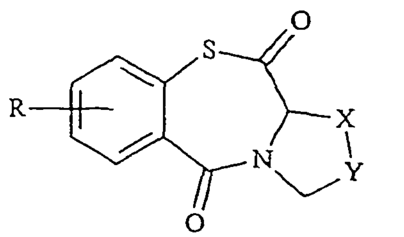

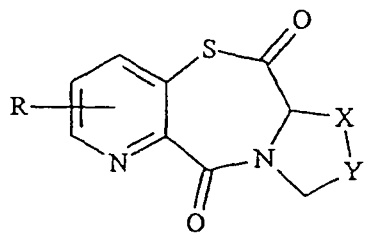

oder

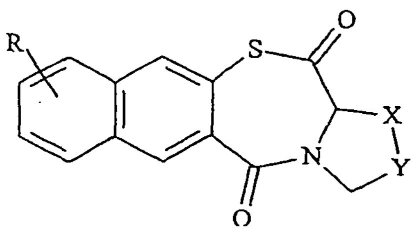

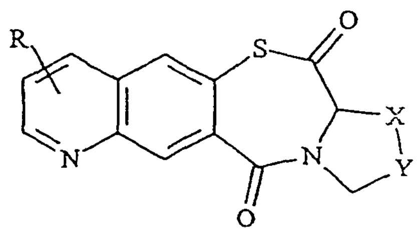

ist.

**39.** Verbindung nach Anspruch 26, **dadurch gekennzeichnet, daß** die Verbindung:

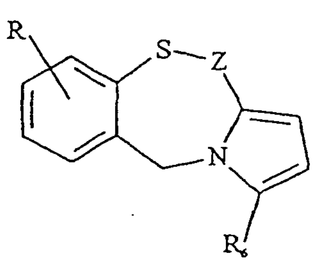

oder

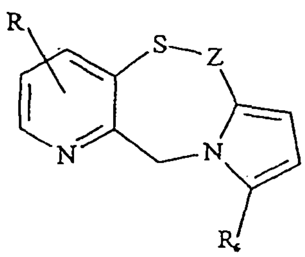

ist.

**40.** Verbindung nach Anspruch 38, **dadurch gekennzeichnet, daß** die Verbindung

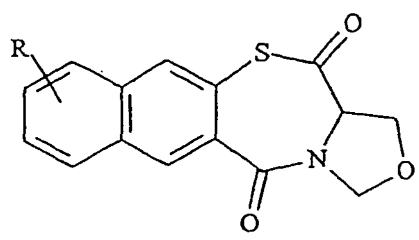

oder

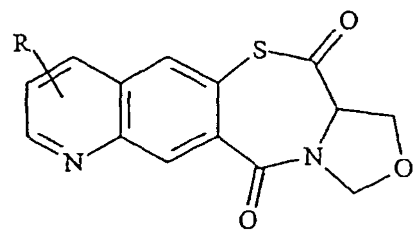

ist.

**41.** Verbindung nach Anspruch 26, **dadurch gekennzeichnet, daß** die Verbindung eine der folgenden ist:

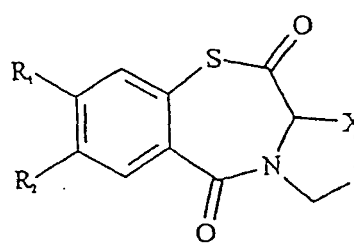

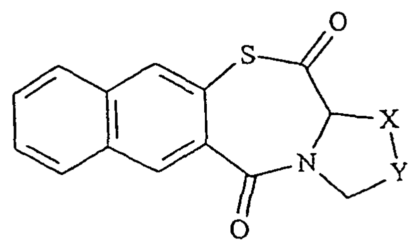

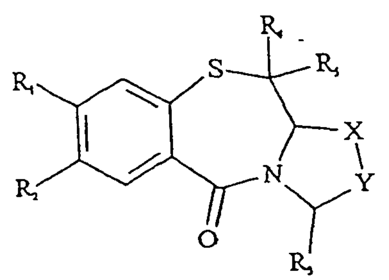

oder

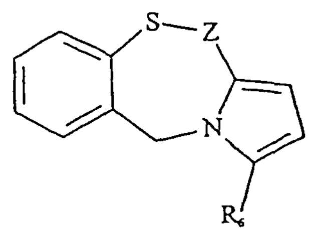

worin

X-Y S-$CH_2$, $CH_2$-S, $CH_2$-O, $CH_2$-$CH_2$, S(O)-$CH_2$ oder $CH_2$-S(O) ist;
$R_1$ und $R_2$ unabhängig ausgewählt sind aus der Gruppe, bestehend aus H, $NO_2$, Halogen, unsubstituiertem oder substituiertem cyclischen, verzweigten oder geradkettigen Alkyl mit von 1 bis 5 Kohlenstoffatomen oder Alkoxy mit von 1 bis 5 Kohlenstoffatomen;
$R_3$ H oder Phenyl ist;
$R_4$ H oder Hydroxy ist;
$R_5$ H, Phenyl oder Alkylamino ist; und
$R_6$ H, Phenyl oder Alkylamino ist.

**42.** Verbindung nach Anspruch 41, **dadurch gekennzeichnet, daß** das Alkylamin -$N(CH_2CH_2)_2NCH_3$, -$CH_2NCH_2CH_3$ oder -$CH_2N(CH_2CH_2)_2NCH_3$ ist.

**43.** Verbindung nach Anspruch 39, **dadurch gekennzeichnet, daß** die Verbindung

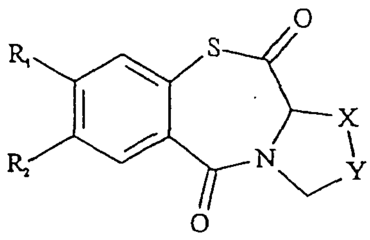

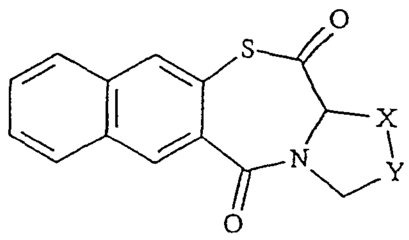

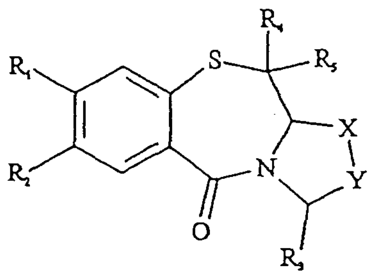

oder

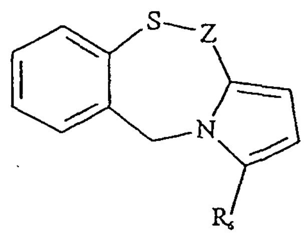

ist, worin

X-Y S-$CH_2$, $CH_2$-S oder $CH_2$-S(O) ist;
und $R_1$ und $R_2$ unabhängig ausgewählt sind aus der Gruppe, bestehend aus H, $NO_2$, Halogen, unsubstituiertem oder substituiertem cyclischen, verzweigten oder geradkettigen Alkyl mit von 1 bis 5 Kohlenstoffatomen und Alkoxy mit von 1 bis 5 Kohlenstoffatomen;
$R_3$ H ist; und
$R_4$, $R_5$ und $R_6$ H sind.

**44.** Verbindung nach Anspruch 26, **dadurch gekennzeichnet, daß** die Verbindung

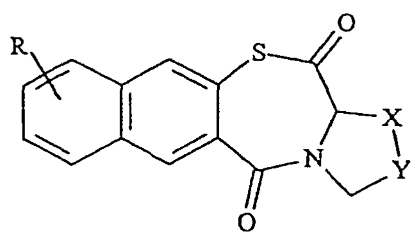

ist und X-Y S-$CH_2$ oder $CH_2$-S ist.

**45.** Verbindung nach Anspruch 44, **dadurch gekennzeichnet, daß** R H ist.

**46.** Verbindung nach Anspruch 45, **dadurch gekennzeichnet, daß** X-Y S-$CH_2$ ist.

**47.** Verbindung nach Anspruch 41, **dadurch gekennzeichnet, daß**

$R_1$ H, $NO_2$ oder Alkoxy mit von 1 bis 5 Kohlenstoffatomen ist;
$R_2$ H, Cl, Br, unsubstituiertes oder substituiertes cyclisches, verzweigtes oder geradkettiges Alkyl mit von 1 bis 5 Kohlenstoffatomen oder Alkoxy mit von 1 bis 5 Kohlenstoffatomen ist;
$R_3$ und $R_4$ H sind;
$R_5$ $N(CH_2CH_2)_2NCH_3$ ist; und
X-Y $CH_2$-S, S-$CH_2$ oder $CH_2$-$CH_2$ ist.

**48.** Verbindung nach Anspruch 47, **dadurch gekennzeichnet, daß** die Verbindung

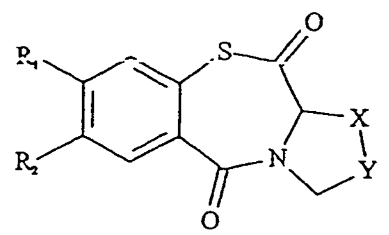

ist,
worin

$R_1$ H, $NO_2$ oder Methoxy ist;
$R_2$ H, Halogen oder Methoxy ist; und
X-Y $CH_2$-S oder S-$CH_2$ ist.

**49.** Verbindung nach einem der Ansprüche 26 bis 48, **dadurch gekennzeichnet, daß** die Behandlung die Hemmung einer HIV-Integrase umfaßt, indem die Integrase einer Integrase-hemmenden Menge einer oder mehrerer von besagten Verbindungen ausgesetzt wird.

**50.** Pharmazeutische Zusammensetzung, die eine Verbindung nach einem der Ansprüche 26 bis 49 umfaßt.

**51.** HIV-Integrase-Inhibitorverbindung mit der folgenden Formel oder pharmazeutisch annehmbares Salz davon:

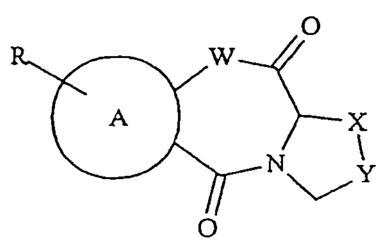

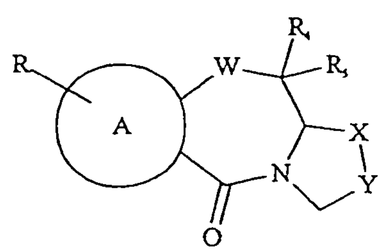

worin

A Thiazol, Benzol, Naphthalin, Pyridin, Pyrimidin, Pyrazin oder Chinolin ist;
R eines oder mehrere von Halogen oder $NO_2$ ist;
X-Y $CH_2$-S, S-$CH_2$, $CH_2$-O, $CH_2$-S(O), S(O)-$CH_2$, $CH_2$-$CH_2$, $CH_2$-$CH_2$-$CH_2$ oder $CH_2$-$CH_2$-$CH_2$-$CH_2$ ist;
$R_4$ H oder Hydroxy ist;
$R_5$ H, Phenyl oder Alkylamino ist; und
W S oder O ist;

oder wobei die Verbindung

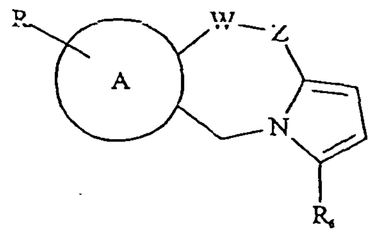

ist, worin

A Thiazol, Benzol, Naphthalin, Pyridin, Pyrimidin, Pyrazin oder Chinolin ist; und
R eines oder mehrere von Halogen oder $NO_2$ ist;
$R_6$ H, unsubstituiertes cyclisches, verzweigtes oder geradkettiges Alkyl, cyclisches, verzweigtes oder geradkettiges Alkyl, substituiert mit einem oder mehreren von Halogen, unsubstituiertem oder substituiertem cyclischen, verzweigten oder geradkettigen Alkyl, Alkoxy, Alkylthio, Trifluormethyl, Acyloxy, Hydroxy, Mercapto, Carboxy, Aryloxy, Aryloxy, Aryl, Arylalkyl, Heteroaryl, Amino, Alkylamino, Dialkylamino, Morpholino, Piperidino, Pyrrolidin-1-yl oder Piperazinyl-1-yl, oder Amino ist;
W S ist; und
Z S, O, $CH_2$, $CH_2CH_2$ oder C=O ist.

**52.** HIV-Integrase-Inhibitorverbindung mit der folgenden Formel, oder pharmazeutisch annehmbares Salz davon, **dadurch gekennzeichnet, daß** die Verbindung:

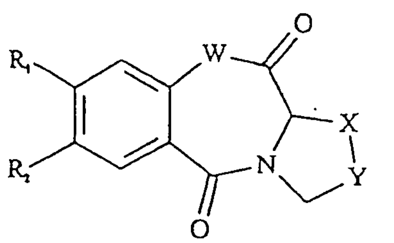

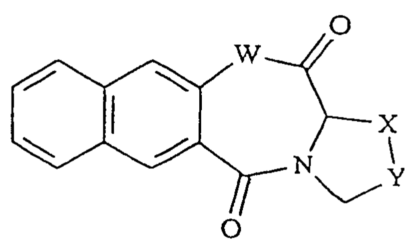

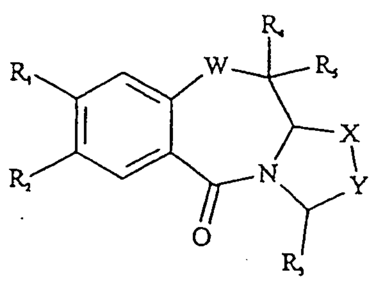

oder

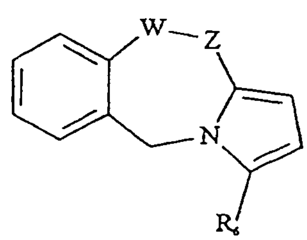

ist

worin X-Y S-$CH_2$, $CH_2$-S, S(O)-$CH_2$, $CH_2$-S(O) oder $CH_2CH_2$ ist;
W S oder O ist;
$R_1$ H oder $NO_2$ ist;
$R_2$ H, Halogen, unsubstituiertes oder substituiertes cyclisches, verzweigtes oder geradkettiges Alkyl mit von 1 bis 5 Kohlenstoffatomen oder Alkoxy mit von 1 bis 5 Kohlenstoffatomen ist;
$R_3$ H ist;
Z S, O, $CH_2$, $CH_2CH_2$ oder C=O ist;
$R_4$ Hydroxy oder H ist;

$R_5$ Phenyl oder $N(CH_2CH_2)_2NCH_3$ ist; und
$R_6$ $CH_2N(CH_2CH_2)_2NCH_3$ ist;
vorausgesetzt, daß $R_1$ und $R_2$ nicht beide H oder nicht beide Alkoxy sind.

**53.** Verbindung nach Anspruch 52, **dadurch gekennzeichnet, daß** die Verbindung

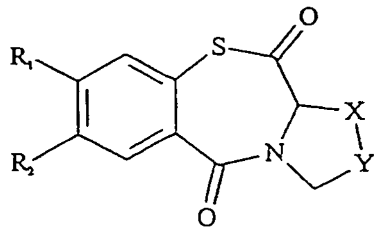

ist und $R_1$ H oder $NO_2$ ist;
$R_2$ H, Halogen, unsubstituiertes oder substituiertes cyclisches, verzweigtes oder geradkettiges Alkyl mit von 1 bis 5 Kohlenstoffatomen oder Alkoxy mit von 1 bis 5 Kohlenstoffatomen ist;
vorausgesetzt, daß $R_1$ und $R_2$ nicht beide H oder nicht beide Alkoxy sind.

**54.** Verbindung nach Anspruch 52, **dadurch gekennzeichnet, daß**

$R_1$ H ist, $R_2$ Cl ist, X-Y S-$CH_2$ ist; oder
$R_1$ H ist, $R_2$ Br ist, X-Y S-$CH_2$ ist; oder
$R_1$ H ist, $R_2$ $CH_3$ ist, X-Y S-$CH_2$ ist; oder
$R_1$ H ist, $R_2$ Cl ist, X-Y $CH_2$-S ist; oder
$R_1$ H ist, $R_2$ Br ist, X-Y $CH_2$-S ist; oder
$R_1$ H ist, $R_2$ $CH_3$ ist, X-Y $CH_2$-S ist; oder
$R_1$ $NO_2$ ist, $R_2$ H ist, X-Y $CH_2$-S ist; oder
$R_1$ H ist, $R_2$ $OCH_3$ ist, X-Y $CH_2$-S ist; oder
$R_1$ H ist, $R_2$ $CH_3$ ist, X-Y S(O)-$CH_2$-S ist; oder
$R_1$ H ist, $R_2$ Cl ist, X-Y $CH_2$-S(O) ist; oder
$R_1$ H ist, $R_2$ $OCH_3$ ist, X-Y $CH_2$-S(O) ist.

**55.** Verbindung nach Anspruch 52, **dadurch gekennzeichnet, daß** die Verbindung

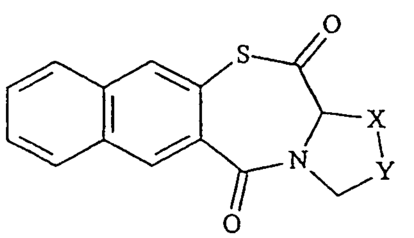

ist und X-Y S-$CH_2$ oder $CH_2$-S ist.

**56.** Verbindung nach Anspruch 55, **dadurch gekennzeichnet, daß** X-Y S-$CH_2$ ist.

**57.** Verbindung nach Anspruch 52, **dadurch gekennzeichnet, daß** die Verbindung

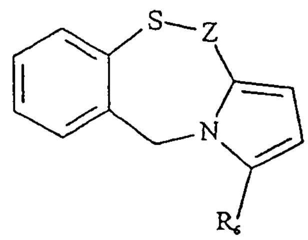

ist und $R_6$ $CH_2N(CH_2CH_2)_2NCH_3$ ist.

**58.** Verbindung mit der Formel

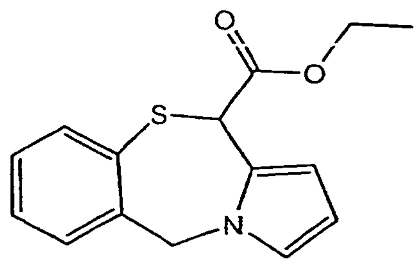

**59.** Verbindung nach Anspruch 58 zur prophylaktischen oder therapeutischen Behandlung von HIV-Infektion bei einem Patienten.

**60.** Verwendung einer Verbindung nach Anspruch 58 zur Herstellung eines Arzneimittels zur prophylaktischen oder therapeutischen Behandlung von HIV-Infektion bei einem Patienten.

**Revendications**

**1.** Utilisation d'un composé choisi dans l'ensemble constitué de :

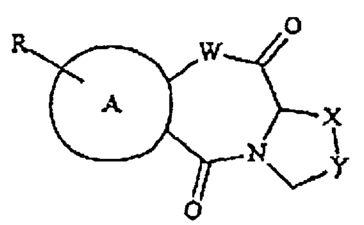

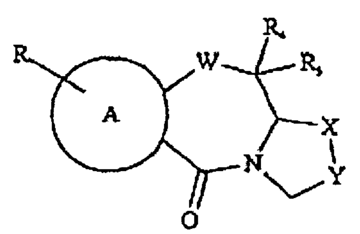

où

A est un groupe thiazole, benzène, naphtalène, pyridine, pyrimidine, pyrazine ou quinoléine;
R est l'un ou plusieurs de H, un halogène, un groupe alkyle non substitué ou substitué, à chaîne cyclique, ramifiée ou linéaire, ayant de 1 à 5 atomes de carbone, un groupe alkoxy ayant de 1 à 5 atomes de carbone, $NO_2$ ou un acide carboxylique ;
X-Y est $CH_2$-S, S-$CH_2$, $CH_2$-O, $CH_2$-S(O), S(O)-$CH_2$, $CH_2$-$CH_2$, $CH_2$-$CH_2$-$CH_2$ ou $CH_2$-$CH_2$-$CH_2$-$CH_2$ ;
$R_4$ est H ou un groupe hydroxy ;
$R_5$ est H, un groupe phényle ou alkylamino ; et
W est S ou O

ou dans laquelle le composé est

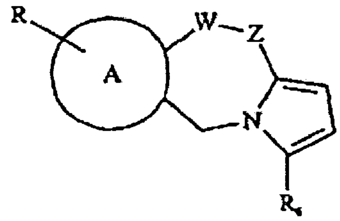

où

A est un groupe thiazole, benzène, naphtalène, pyridine, pyrimidine, pyrazine ou quinoléine ; et
R est l'un ou plusieurs de H, $NO_2$, un halogène, un groupe alkyle non substitué ou substitué, à chaîne cyclique, ramifiée ou linéaire, ayant de 1 à 5 atomes de carbone, ou un acide carboxylique ;
$R_6$ est H, un groupe alkyle non substitué à chaîne cyclique, linéaire ou ramifiée, ou un groupe alkyle à chaîne cyclique, ramifiée ou linéaire, substitué par un ou plusieurs éléments choisis parmi un halogène, un groupe alkyle, alkoxy, alkylthio, trifluorométhyle, acyloxy, hydroxy, mercapto, carboxy, aryloxy, aryloxy, aryle, arylalkyle, hétéroaryle, amino, alkylamino, dialkylamino, morpholino, pipéridino, pyrrolidin-1-yle ou pipérazin-1-yle ou amino ;
W est S ou O ; et
Z est S, O, $CH_2$, $CH_2CH_2$ ou C=O, pour la fabrication d'un médicament pour le traitement prophylactique ou thérapeutique d'une infection par le VIH chez un sujet.

**2.** Utilisation selon la revendication 1, dans laquelle le composé est choisi dans l'ensemble constitué de :

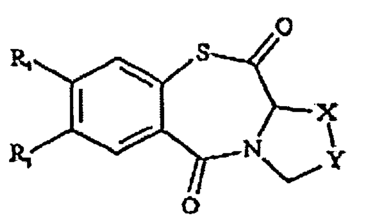

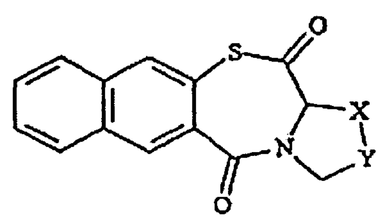

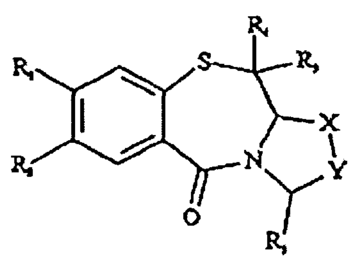

et

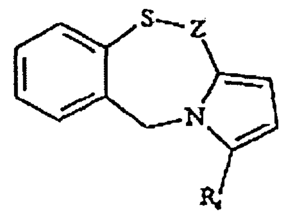

où

X-Y est S-$CH_2$, $CH_2$-S, $CH_2CH_2$ ou S(O)$CH_2$;
$R_1$ est H, $NO_2$ ou un groupe alkoxy ayant de 1 à 5 atomes de carbone ;
$R_2$ est H, Cl, Br, un groupe alkyle non substitué ou substitué, à chaîne cyclique, linéaire ou ramifiée, ayant de 1 à 5 atomes de carbone, ou un groupe alkoxy ayant de 1 à 5 atomes de carbone ;
$R_3$ et $R_4$ sont H ;
$R_5$ est $N(CH_2CH_2)_2NCH_3$ ; et
$R_6$ est H.

**3.** Utilisation selon la revendication 1, dans laquelle le composé est

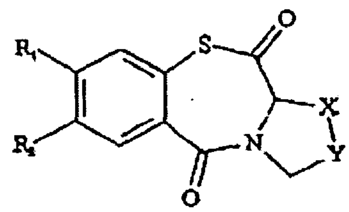

et

$R_1$ et $R_2$ sont H et X-Y est S-$CH_2$ ; ou
$R_1$ est H, $R_2$ est Cl ou Br ou un groupe méthyle et X-Y est S-$CH_2$ ; ou
$R_1$ est $NO_2$, $R_2$ est H et X-Y est $CH_2$-S ; ou
$R_1$ et $R_2$ sont un groupe méthoxy et X-Y est $CH_2$-S ; ou
$R_1$ est H, $R_2$ est un groupe méthyle et X-Y est S(O)-$CH_2$.

**4.** Utilisation selon la revendication 1, dans laquelle le composé est

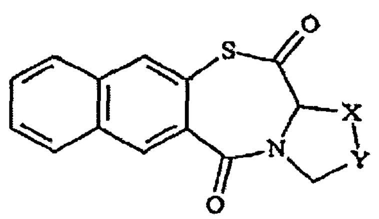

où X-Y est S-$CH_2$ ou $CH_2$-S.

**5.** Utilisation selon la revendication 1, dans laquelle le composé est

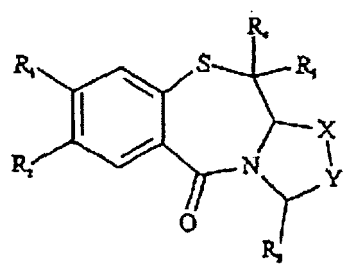

où

X-Y est $CH_2$-$CH_2$ ;
$R_1$, $R_2$, $R_3$ et $R_4$ sont H ; et
$R_5$ est $N(CH_2CH_2)_2NCH_3$.

**6.** Utilisation selon la revendication 1, dans laquelle le composé comprend

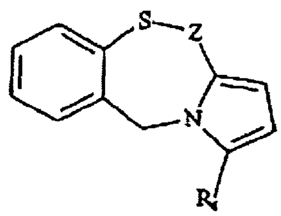

où $R_6$ est H et Z est C=O.

**7.** Utilisation selon la revendication 1, dans laquelle

A est un groupe thiazole, benzène, naphtalène, pyridine, pyrimidine, pyrazine ou quinoléine ;
R est l'un ou plusieurs d'un halogène ou $NO_2$ ;
X-Y est $CH_2$-S, S-$CH_2$, $CH_2$-O, $CH_2$-S(O), S(O)-$CH_2$, $CH_2$-$CH_2$, $CH_2$-$CH_2$-$CH_2$ ou $CH_2$-$CH_2$-$CH_2$-$CH_2$ ;
$R_4$ est H ou un groupe hydroxy ;
$R_5$ est H, un groupe phényle ou alkylamino ;
$R_6$ est H ou un groupe alkyle non substitué à chaîne cyclique, ramifiée ou linéaire, ou un groupe alkyle à chaîne cyclique, ramifiée ou linéaire, substitué par un ou plusieurs éléments choisis parmi un halogène, un groupe alkyle, alkoxy, alkylthio, trifluorométhyle, acyloxy, hydroxy, mercapto, carboxy, aryloxy, aryloxy, aryle, arylalkyle, hétéroaryle, amino, alkylamino, dialkylamino, morpholino, pipéridino, pyrrolidin-1-yle ou pipérazin-1-yle ou amino ; et
W est S ou O.

**8.** Utilisation selon la revendication 1, dans laquelle le composé comprend

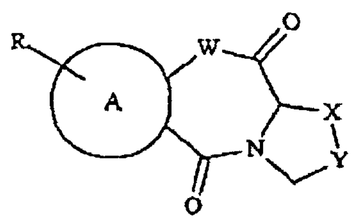

et A est un groupe benzène ou naphtalène ;
R est H, $NO_2$ ou un groupe alkoxy ayant de 1 à 5 atomes de carbone ; et
X-Y est $CH_2$-S ou S-$CH_2$.

**9.** Utilisation selon la revendication 1, dans laquelle le composé est choisi dans l'ensemble constitué de :

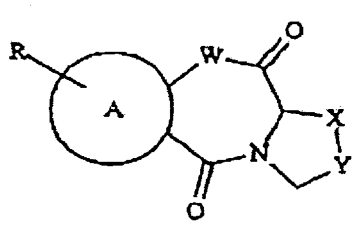

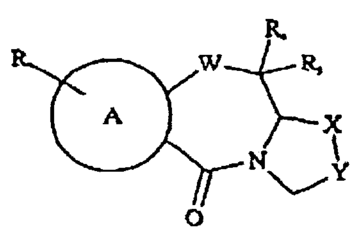

où X-Y est $CH_2$-S, S-$CH_2$, $CH_2$-O ou $CH_2$-$CH_2$, et W est S.

**10.** Utilisation selon la revendication 9, dans laquelle :

A est un groupe benzène, naphtalène, pyridine, pyrimidine, pyrazine ou quinoléine.

**11.** Utilisation selon la revendication 10, dans laquelle A est un groupe benzène ou naphtalène.

**12.** Utilisation selon la revendication 11, dans laquelle R est H, un halogène, un groupe alkoxy ayant de 1 à 5 atomes de carbone ou $NO_2$.

**13.** Utilisation selon la revendication 1, dans laquelle le composé est:

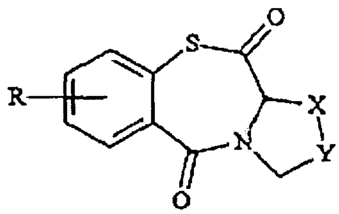

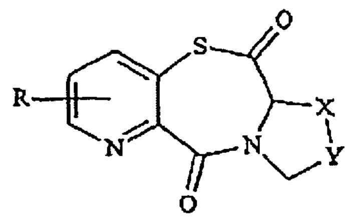

ou

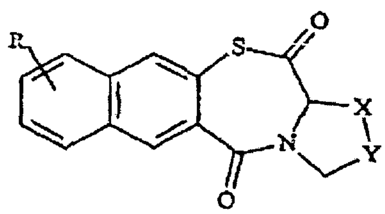

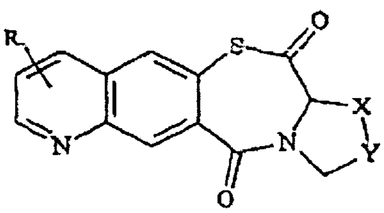

**14.** Utilisation selon la revendication 1, dans laquelle le composé est :

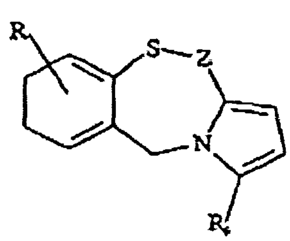

ou

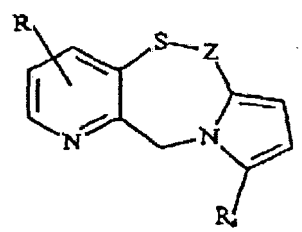

**15.** Utilisation selon la revendication 13, dans laquelle le composé est

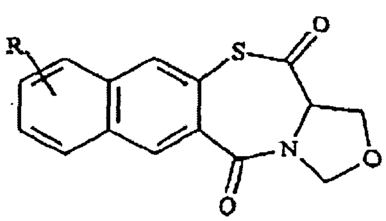

ou

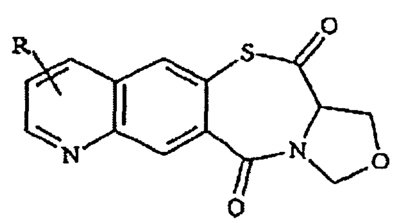

**16.** Utilisation selon la revendication 1, dans laquelle le composé est l'un des suivants :

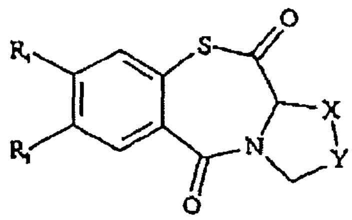

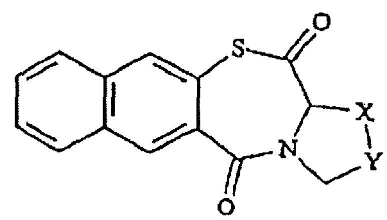

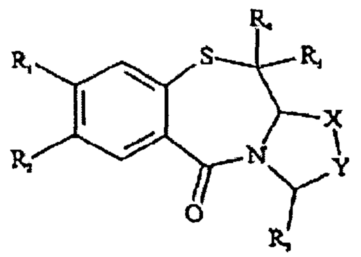

ou

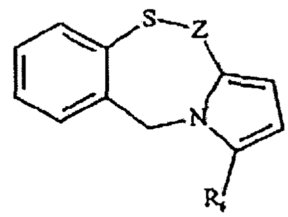

où

X-Y est $S-CH_2$, $CH_2-S$, $CH_2-O$, $CH_2-CH_2$, $S(O)-CH_2$ ou $CH_2-S(O)$ ;
$R_1$ et $R_2$ sont choisis indépendamment dans l'ensemble constitué de H, $NO_2$, un halogène, un groupe alkyle non substitué ou substitué, à chaîne cyclique, ramifiée ou linéaire, ayant de 1 à 5 atomes de carbone, ou un groupe alkoxy ayant de 1 à 5 atomes de carbone ;
$R_3$ est H ou un groupe phényle ;
$R_4$ est H ou un groupe hydroxy ;
$R_5$ est H, un groupe phényle ou alkylamino ; et
$R_6$ est H, un groupe phényle ou alkylamino.

**17.** Utilisation selon la revendication 16, dans laquelle l'alkylamine est $-N(CH_2CH_2)_2NCH_3$, $-CH_2NCH_2CH_3$ ou $-CH_2N(CH_2CH_2)_2NCH_3$.

**18.** Utilisation selon la revendication 14, dans laquelle le composé est

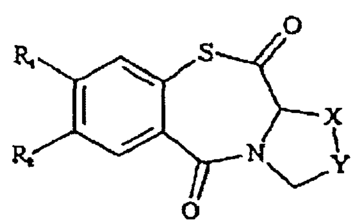

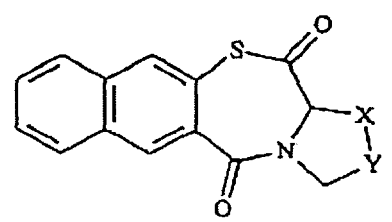

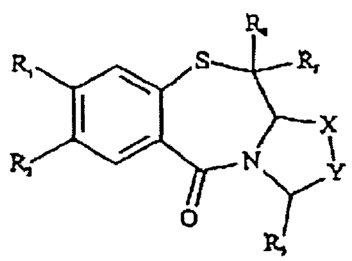

ou

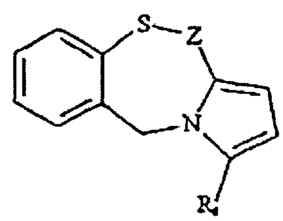

X-Y est $S-CH_2$, $CH_2-S$ ou $CH_2-S(O)$ ;

et $R_1$ et $R_2$ sont choisis indépendamment dans l'ensemble constitué de H, $NO_2$, un halogène, un groupe alkyle non substitué ou substitué, à chaîne cyclique, ramifiée ou linéaire, ayant de 1 à 5 atomes de carbone et un groupe alkoxy ayant de 1 à 5 atomes de carbone ;
$R_3$ est H ; et
$R_4$, $R_5$ et $R_6$ sont H.

**19.** Utilisation selon la revendication 1, dans laquelle le composé est

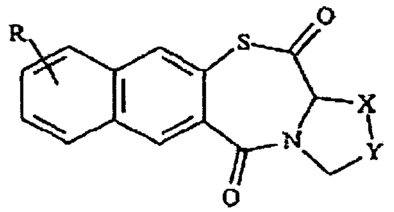

et X-Y est S-$CH_2$ ou $CH_2$-S.

**20.** Utilisation selon la revendication 19, dans laquelle R est H.

**21.** Utilisation selon la revendication 20, dans laquelle X-Y est S-$CH_2$.

**22.** Utilisation selon la revendication 16, dans laquelle

$R_1$ est H, $NO_2$ ou un groupe alkoxy ayant de 1 à 5 atomes de carbone,
$R_2$ est H, Cl, Br, un groupe alkyle non substitué ou substitué, à chaîne cyclique, linéaire ou ramifiée, ayant de 1 à 5 atomes de carbone, ou un groupe alkoxy ayant de 1 à 5 atomes de carbone ;
$R_3$ et $R_4$ sont H ;
$R_5$ est $N(CH_2CH_2)_2NCH_3$ ; et
X-Y est $CH_2$-S, S-$CH_2$ ou $CH_2$-$CH_2$.

**23.** Utilisation selon la revendication 22, dans laquelle le composé est

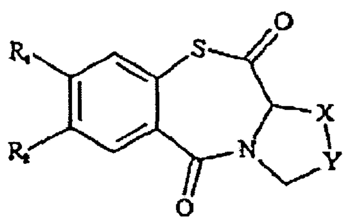

où $R_1$ est H, $NO_2$ ou un groupe méthoxy ;
$R_2$ est H, un halogène ou un groupe méthoxy ; et X-Y est $CH_2$-S ou S-$CH_2$.

**24.** Utilisation selon l'une quelconque des revendications précédentes, dans laquelle le traitement comprend l'inhibition d'une intégrase du VIH par exposition de l'intégrase à une quantité d'un ou plusieurs desdits composés qui inhibe l'intégrase.

**25.** Utilisation selon l'une quelconque des revendications précédentes, dans laquelle le médicament est destiné à une administration en une quantité thérapeutiquement efficace au sujet.

**26.** Composé choisi dans l'ensemble constitué de :

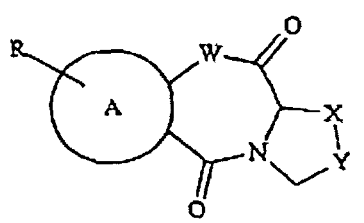 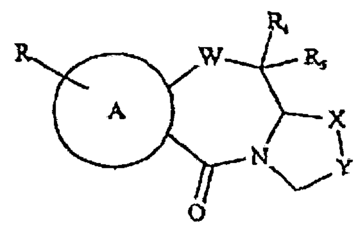

où

A est un groupe thiazole, benzène, naphtalène, pyridine, pyrimidine, pyrazine ou quinoléine ;
R est l'un ou plusieurs de H, un halogène, un groupe alkyle non substitué ou substitué, à chaîne cyclique, ramifiée ou linéaire, ayant de 1 à 5 atomes de carbone, un groupe alkoxy ayant de 1 à 5 atomes de carbone, $NO_2$ ou un acide carboxylique ;
X-Y est $CH_2$-S, S-$CH_2$, $CH_2$-O, $CH_2$-S(O), S(O)-$CH_2$, $CH_2$-$CH_2$, $CH_2$-$CH_2$-$CH_2$ ou $CH_2$-$CH_2$-$CH_2$-$CH_2$ ;
$R_4$ est H ou un groupe hydroxy ;
$R_5$ est H, un groupe phényle ou alkylamino ; et
W est S ou O ; à condition que le composé ne soit pas choisi parmi :

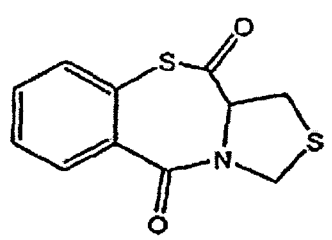 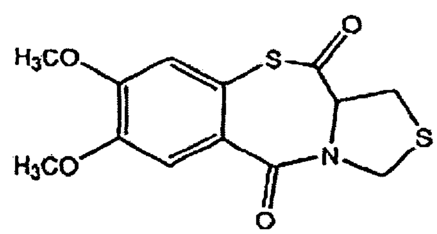

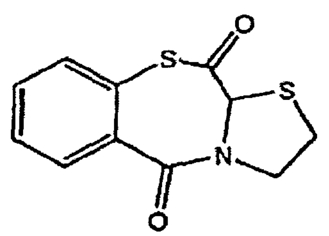 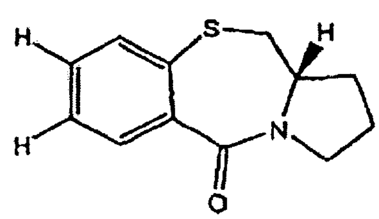

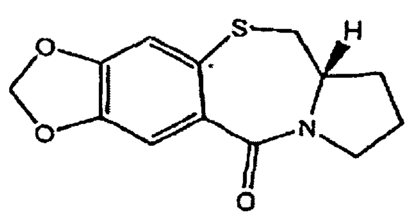 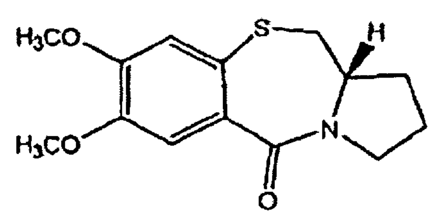

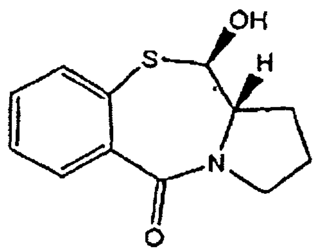

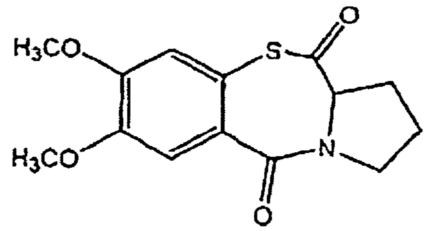

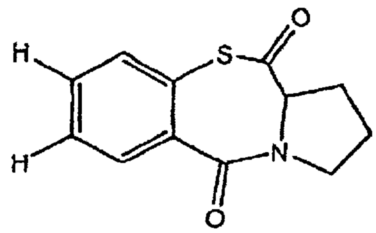

ou où le composé est

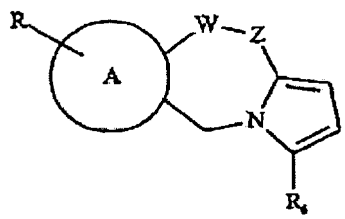

où

A est un groupe thiazole, benzène, naphtalène, pyridine, pyrimidine, pyrazine ou quinoléine ; et
R est l'un ou plusieurs de H, $NO_2$, un halogène, un groupe alkyle non substitué ou substitué, à chaîne cyclique, ramifiée ou linéaire, ayant de 1 à 5 atomes de carbone ou un acide carboxylique ;
$R_6$ est H, un groupe alkyle non substitué à chaîne cyclique, ramifiée ou linéaire, un groupe alkyle à chaîne cyclique, ramifiée ou linéaire, substitué par un ou plusieurs éléments choisis parmi un halogène, un groupe alkyle, alkoxy, alkylthio, trifluorométhyle, acyloxy, hydroxy, mercapto, carboxy, aryloxy, aryloxy, aryle, arylalkyle, hétéroaryle, amino, alkylamino, dialkylamino, morpholino, pipéridino, pyrrolidin-1-yle ou pipérazin-1-yle ou amino ;
W est S ; et
Z est S, O, $CH_2$, $CH_2CH_2$ ou C=O
à condition que le composé ne soit pas

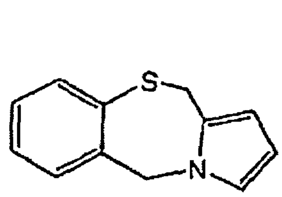

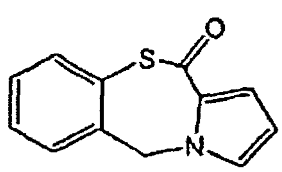

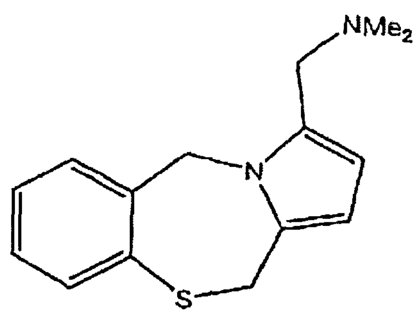

pour un traitement prophylactique ou thérapeutique d'une infection par le VIH chez un sujet.

**27.** Composé selon la revendication 26, ledit composé étant choisi dans l'ensemble constitué de :

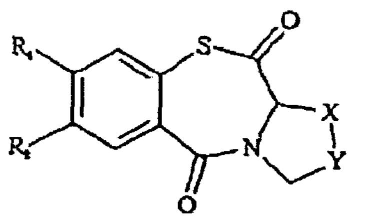

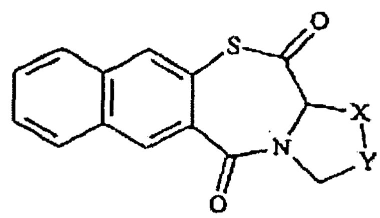

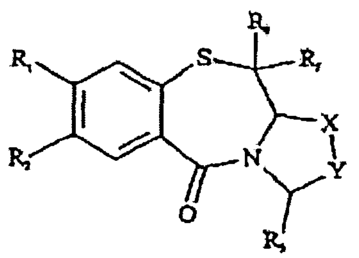

et

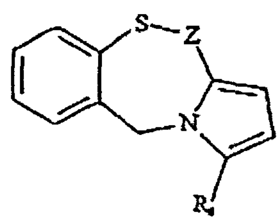

où X-Y est S-$CH_2$, $CH_2$-S, $CH_2CH_2$ ou S(O)$CH_2$ ;
$R_1$ est H, $NO_2$ ou un groupe alkoxy ayant de 1 à 5 atomes de carbone ;
$R_2$ est H, Cl, Br, un groupe alkyle non substitué ou substitué, à chaîne cyclique, ramifiée ou linéaire, ayant de 1 à 5 atomes de carbone, ou un groupe alkoxy ayant de 1 à 5 atomes de carbone ;
$R_3$ et $R_4$ sont H ;
$R_5$ est $N(CH_2CH_2)_2NCH_3$; et
$R_6$ est H.

**28.** Composé selon la revendication 26, ledit composé étant

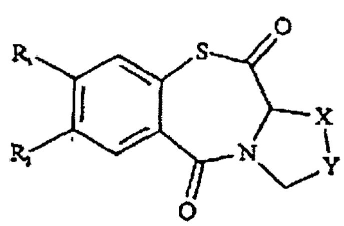

et

$R_1$ et $R_2$ sont H et X-Y est S-$CH_2$ ; ou
$R_1$ est H, $R_2$ est Cl ou Br ou un groupe méthyle et X-Y est S-$CH_2$ ; ou
$R_1$ est $NO_2$ $R_2$ est H et X-Y est .$CH_2$-S ; ou
$R_1$ et $R_2$ sont un groupe méthoxy et X-Y est $CH_2$-S ; ou
$R_1$ est H, $R_2$ est un groupe méthyle et X-Y est S(O)-$CH_2$.

**29.** Composé selon la revendication 26, ledit composé étant

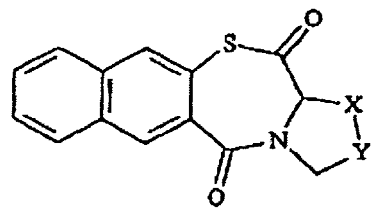

où X-Y est S-$CH_2$ ou $CH_2$-S.

**30.** Composé selon la revendication 26, ledit composé étant

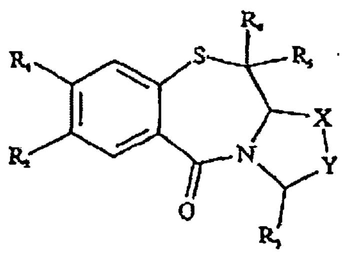

où X-Y est $CH_2$-$CH_2$;
$R_1$, $R_2$, $R_3$ et $R_4$ sont H ; et
$R_5$ est $N(CH_2CH_2)_2NCH_3$.

**31.** Composé selon la revendication 26, ledit composé comprenant

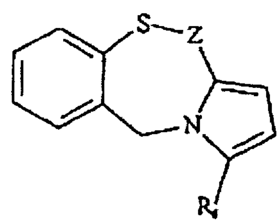

où $R_6$ est H et Z est C=O.

**32.** Composé selon la revendication 26, dans lequel

A est groupe thiazole, benzène, naphtalène, pyridine, pyrimidine, pyrazine ou quinoléine ;
R est l'un ou plusieurs d'un halogène ou $NO_2$;
X-Y est $CH_2$-S, S-$CH_2$, $CH_2$-O, $CH_2$-S(O), S(O)-$CH_2$, $CH_2$-$CH_2$, $CH_2$-$CH_2$-$CH_2$ ou $CH_2$-$CH_2$-$CH_2$-$CH_2$;
$R_4$ est H ou un groupe hydroxy ;
$R_5$ est H, un groupe phényle ou alkylamino ;
$R_6$ est H, ou un groupe alkyle non substitué, à chaîne cyclique, ramifiée ou linéaire, ou un groupe alkyle à chaîne cyclique, ramifiée ou linéaire, substitué par un ou plusieurs éléments choisis parmi un halogène, un groupe alkyle non substitué ou substitué, à chaîne cyclique, ramifiée ou linéaire, ayant de 1 à 12 atomes de carbone, alkoxy, alkylthio, trifluorométhyle, acyloxy, hydroxy, mercapto, carboxy, aryloxy, aryloxy, aryle, arylalkyle, hétéroaryle, amino, alkylamino, dialkylamino, morpholino, pipéridino, pyrrolidin-1-yle ou pipérazin-1-yle ou amino ; et
w est S ou O.

**33.** Composé selon la revendication 26, ledit composé comprenant

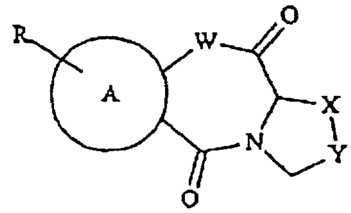

et A est un groupe benzène ou naphtalène ;
R est H, $NO_2$ ou un groupe alkoxy ayant de 1 à 5 atomes de carbone ; et
X-Y est $CH_2$-S ou S-$CH_2$.

**34.** Composé selon la revendication 26, ledit composé étant choisi dans l'ensemble constitué de :

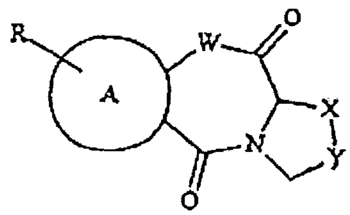

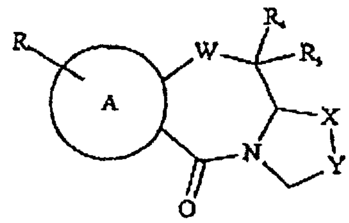

où X-Y est $CH_2$-S, S-$CH_2$, $CH_2$-O ou $CH_2$-$CH_2$ et W est S.

**35.** Composé selon la revendication 34, dans lequel :

A est un groupe benzène, naphtalène, pyridine, pyrimidine, pyrazine ou quinoléine.

**36.** Composé selon la revendication 35, dans lequel A est un groupe benzène ou naphtalène.

**37.** Composé selon la revendication 36, dans lequel R est H, un halogène, un groupe alkoxy ayant de 1 à 5 atomes de carbone ou $NO_2$.

**38.** Composé selon la revendication 26, ledit composé étant :

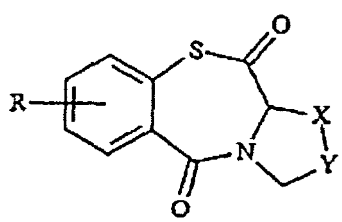

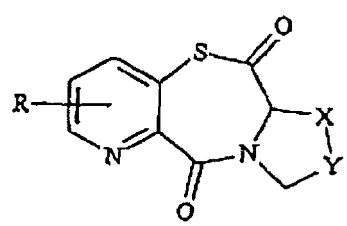

ou

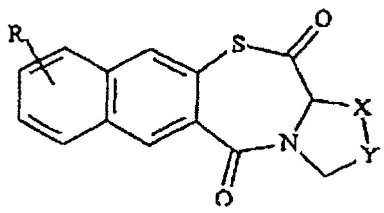

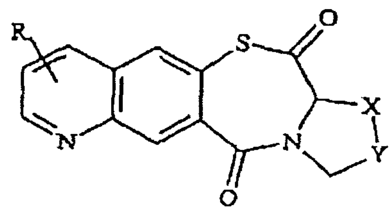

**39.** Composé selon la revendication 26, ledit composé étant :

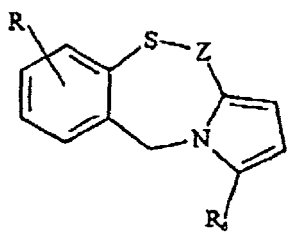

ou

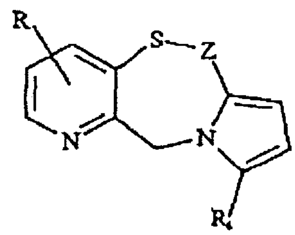

**40.** Composé selon la revendication 38, ledit composé étant

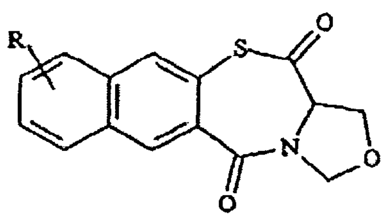

ou

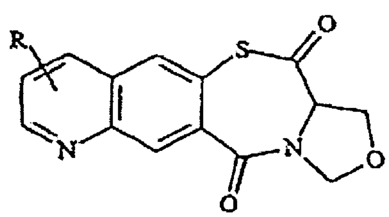

**41.** Composé selon la revendication 26, ledit composé étant l'un des suivants :

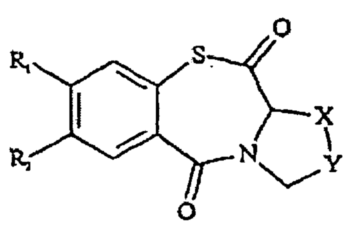

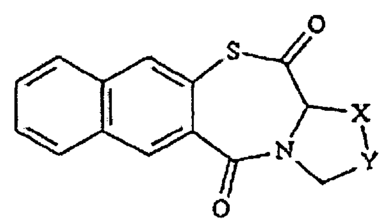

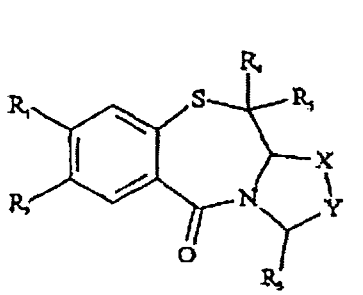

ou

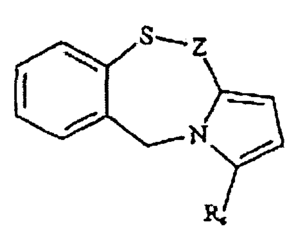

où

X-Y est S-$CH_2$, $CH_2$-S, $CH_2$-O, $CH_2$-$CH_2$, S(O)-$CH_2$ ou $CH_2$-S(O);
$R_1$ et $R_2$ sont choisis indépendamment dans l'ensemble constitué de H, $NO_2$, un halogène, un groupe alkyle non substitué ou substitué, à chaîne cyclique, ramifiée ou linéaire, ayant de 1 à 5 atomes de carbone, ou un groupe alkoxy ayant de 1 à 5 atomes de carbone ;
$R_3$ est H ou un groupe phényle ;
$R_4$ est H ou un groupe hydroxy ;
$R_5$ est H, un groupe phényle ou alkylamino ; et
$R_6$ est H, un groupe phényle ou alkylamino.

**42.** Composé selon la revendication 41, dans lequel l'alkylamine est -$N(CH_2CH_2)_2NCH_3$, -$CH_2NCH_2CH_3$ ou -$CH_2N(CH_2CH_2)_2NCH_3$.

**43.** Composé selon la revendication 39, ledit composé étant

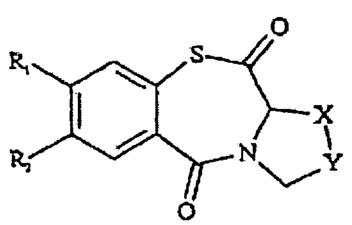

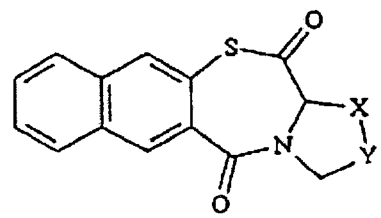

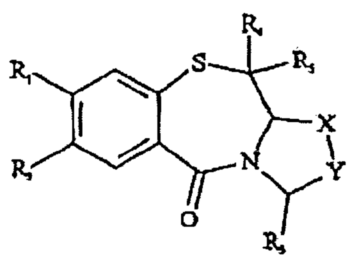

ou

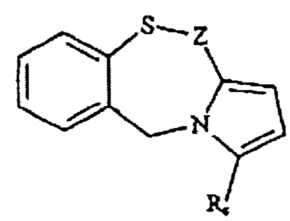

X-Y est S-$CH_2$, $CH_2$-S ou $CH_2$-S(O) ;
et $R_1$ et $R_2$ sont choisis indépendamment dans l'ensemble constitué de H, $NO_2$, un halogène, un groupe alkyle non substitué ou substitué, à chaîne cyclique, ramifiée ou linéaire, ayant de 1 à 5 atomes de carbone et un groupe alkoxy ayant de 1 à 5 atomes de carbone ;
$R_3$ est H ; et
$R_4$, $R_5$ et $R_6$ sont H.

**44.** Composé selon la revendication 26, ledit composé étant

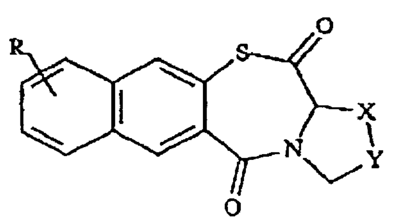

et X-Y est S-$CH_2$ ou $CH_2$-S.

**45.** Composé selon la revendication 44, dans lequel R est H.

**46.** Composé selon la revendication 45, dans lequel X-Y est S-$CH_2$.

**47.** Composé selon la revendication 41, dans lequel

$R_1$ est H, $NO_2$ ou un groupe alkoxy ayant de 1 à 5 atomes de carbone,
$R_2$ est H, Cl, Br, un groupe alkyle non substitué ou substitué, à chaîne cyclique, ramifiée ou linéaire, ayant de 1 à 5 atomes de carbone, ou un groupe alkoxy ayant de 1 à 5 atomes de carbone ;
$R_3$ et $R_4$ sont H ;
$R_5$ est $N(CH_2CH_2)_2NCH_3$ ; et
X-Y est $CH_2$-S, S-$CH_2$ ou $CH_2$-$CH_2$.

**48.** Composé selon la revendication 47, ledit composé étant

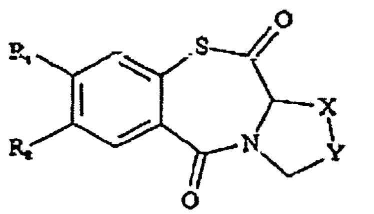

où $R_1$ est H, $NO_2$ ou un groupe méthoxy ;
$R_2$ est H, un halogène ou un groupe méthoxy ; et
X-Y est $CH_2$-S ou S-$CH_2$.

**49.** Composé selon l'une quelconque des revendications 26 à 48, ledit traitement comprenant l'inhibition d'une intégrase du VIH par exposition de l'intégrase à une quantité d'un ou plusieurs desdits composés qui inhibe l'intégrase.

**50.** Composition pharmaceutique comprenant un composé selon l'une quelconque des revendications 26 à 49.

**51.** Composé inhibiteur d'intégrase de VIH ayant la formule suivante, ou un sel pharmaceutiquement acceptable de celui-ci :

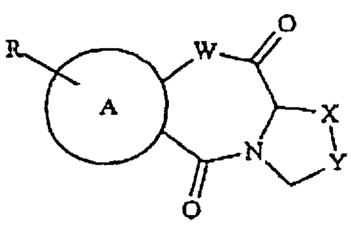

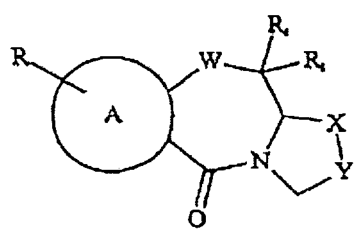

où

A est un groupe thiazole, benzène, naphtalène, pyridine, pyrimidine, pyrazine ou quinoléine;
R est l'un ou plusieurs d'un halogène ou $NO_2$;
X-Y est $CH_2$-S, S-$CH_2$, $CH_2$-O, $CH_2$-S(O), S(O)-$CH_2$, $CH_2$-$CH_2$, $CH_2$-$CH_2$-$CH_2$ ou $CH_2$-$CH_2$-$CH_2$-$CH_2$ ;
$R_4$ est H ou un groupe hydroxy;
$R_5$ est H, un groupe phényle ou alkylamino; et
W est S ou O

ou ledit composé étant

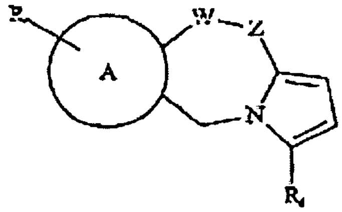

où

A est un groupe thiazole, benzène, naphtalène, pyridine, pyrimidine, pyrazine ou quinoléine ; et
R est l'un ou plusieurs d'un halogène ou $NO_2$;
$R_6$ est H, un groupe alkyle non substitué, à chaîne cyclique, ramifiée ou linéaire, un groupe alkyle à chaîne cyclique, ramifiée ou linéaire, substitué par un ou plusieurs éléments choisis parmi un halogène, un groupe alkyle non substitué ou substitué, à chaîne cyclique, ramifiée ou linéaire, alkoxy, alkylthio, trifluorométhyle, acyloxy, hydroxy, mercapto, carboxy, aryloxy, aryloxy, aryle, arylalkyle, hétéroaryle, amino, alkylamino, dialkylamino, morpholino, pipéridino, pyrrolidin-1-yle ou pipérazin-1-yle ou amino ;
W est S ; et Z est S, O, $CH_2$, $CH_2CH_2$ ou C=O.

**52.** Composé inhibiteur de l'intégrase du VIH ayant la formule suivante, ou un sel pharmaceutiquement acceptable de celui-ci, ledit composé étant :

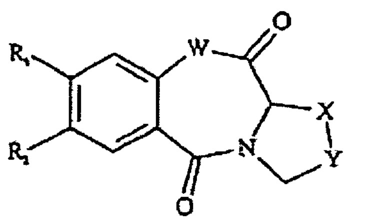

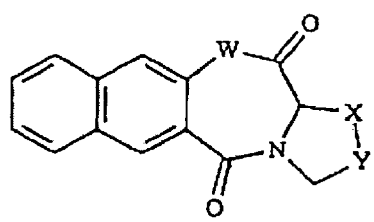

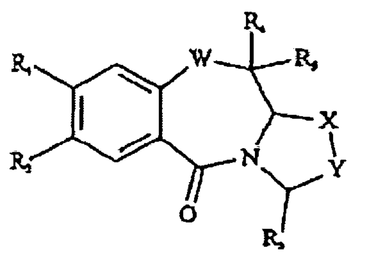

ou

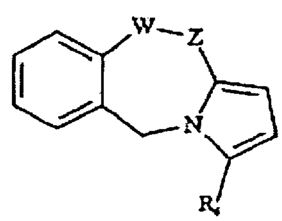

où

X-Y est S-$CH_2$, $CH_2$-S, S(O)-$CH_2$, $CH_2$-S (O) ou $CH_2CH_2$;
W est S ou O ;
$R_1$ est H ou $NO_2$ ;
$R_2$ est H, un halogène, un groupe alkyle non substitué ou substitué, à chaîne cyclique, ramifiée ou linéaire, ayant de 1 à 5 atomes de carbone, ou un groupe alkoxy ayant de 1 à 5 atomes de carbone ;
$R_3$ est H ;
Z est S, O, $CH_2$, $CH_2CH_2$ ou C=O ;
$R_4$ est un groupe hydroxy ou H ;
$R_5$ est un groupe phényle ou $N(CH_2CH_2)_2NCH_3$ ; et
$R_6$ est $CH_2N(CH_2CH_2)_2NCH_3$,

à condition que $R_1$ et $R_2$ ne soient pas tous les deux H ou qu'ils ne soient pas tous les deux un groupe alkoxy.

**53.** Composé selon la revendication 52, ledit composé étant

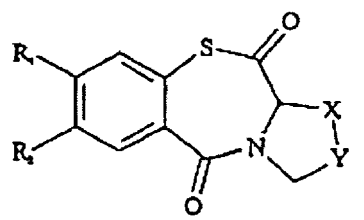

et

$R_1$ est H ou $NO_2$;
$R_2$ est H, un halogène, un groupe alkyle non substitué ou substitué, à chaîne cyclique, ramifiée ou linéaire, ayant de 1 à 5 atomes de carbone, ou un groupe alkoxy ayant de 1 à 5 atomes de carbone ;
à condition que $R_1$ et $R_2$ ne soient pas tous les deux H ou qu'ils ne soient pas tous les deux un groupe alkoxy.

**54.** Composé selon la revendication 52, dans lequel

$R_1$ est H, $R_2$ est Cl, X-Y est S-$CH_2$ ; ou
$R_1$ est H, $R_2$ est Br, X-Y est S-$CH_2$; ou
$R_1$ est H, $R_2$ est $CH_3$, X-Y est S-$CH_2$ ; ou
$R_1$ est H, $R_2$ est Cl, X-Y est $CH_2$-S ; ou
$R_1$ est H, $R_2$ est Br, X-Y est $CH_2$-S ; ou
$R_1$ est H, $R_2$ est $CH_3$, X-Y est $CH_2$-S ; ou
$R_1$ est $NO_2$, $R_2$ est H, X-Y est $CH_2$-S ; ou
$R_1$ est H, $R_2$ est $OCH_3$, X-Y est $CH_2$-S ; ou
$R_1$ est H, $R_2$ est $CH_3$, X-Y est S(O)-$CH_2$ ; ou
$R_1$ est H, $R_2$ est Cl, X-Y est $CH_2$-S (O) ; ou
$R_1$ est H, $R_2$ est $OCH_3$, X-Y est $CH_2$-S(O).

**55.** Composé selon la revendication 52, ledit composé étant

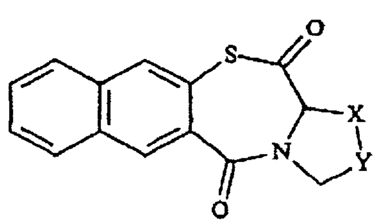

et X-Y est S-$CH_2$ ou $CH_2$-S.

**56.** Composé selon la revendication 55, dans lequel X-Y est S-$CH_2$.

**57.** Composé selon la revendication 52, ledit composé étant

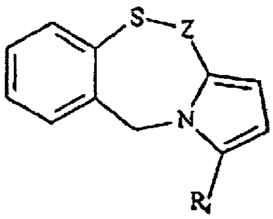

et $R_6$ est $CH_2N(CH_2CH_2)_2NCH_3$.

**58.** Composé ayant la formule

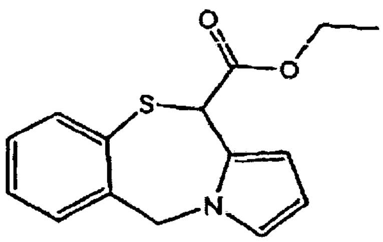

**59.** Composé selon la revendication 58, pour le traitement prophylactique ou thérapeutique d'une infection par le VIH chez un sujet.

**60.** Utilisation d'un composé selon la revendication 58, pour la fabrication d'un médicament pour le traitement prophylactique ou thérapeutique d'une infection par le VIH chez un sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4861770 A
- WO 9818473 A


### Non-patent literature cited in the description


- Remington: The Science and Practice of Pharmacology. 1995
- The McGraw-Hill Dictionary of Chemical Terms. 1985
- The Condensed Chemical Dictionary. 1981
- Remington: The Science and Practice of Pharmacy. 1995
- **BROWN, P. O.** Integration. Cold Spring Harbor Press, 1998
- **MAZUMDER, A. ; NEAMATI, N. ; SUNDER, S. ; OWEN, J. ; POMMIER, Y.** Retroviral Integrase: A Novel Target in Antiviral Development; Basic In Vitro Assays with the Purified Enzyme. The Humana Press, Inc, 1998, 1998
- **CHOW, S. A.** In vitro assays for activities of retroviral integrase. *Methods,* 1997, vol. 12, 306-17
- **CHOW, S. A. ; VINCENT, K. A. ; ELLISON, V. ; BROWN, P. O.** Reversal of integration and DNA splicing mediated by integrase of human immunodeficiency virus. *Science,* 1992, vol. 255, 723-6
- **POMMIER, Y. ; PILON, A. ; BAJAJ, K. ; MAZUMDER, A. ; NEAMATI, N.** HIV-1 integrase as a target for antiviral drugs. *Antiviral Chem Chemother,* 1997, vol. 8, 483-503
- **NEAMATI, N. ; SUNDER, S. ; POMMIER, Y.** Design and discovery of HIV-1 integrase inhibitors. *Drug Discovery Today,* 1997, vol. 2, 487-498
- **STANWELL, C. ; YE, B. ; YUSPA, S. H. ; BURKE, T. R., JR.** Cell protein crosslinking by erbstatin and related compounds. *Biochem Pharmacol,* 1996, vol. 52, 475-80
- **NEAMATI, N. ; HONG, H. ; OWEN, J. M. ; SUNDER, S. ; WINSLOW, H. E. ; CHRISTENSEN, J. L. ; ZHAO, H. ; BURKE, J. T. R. ; MILNE, G. W. A. ; POMMIER, Y.** Salicylhydrazine-containing inhibitors of HIV-1 integrase: implication for a selective chelation in the integrase active site. *J Med Chem,* 1998, vol. 41, 3202-3209
- **NEAMATI, N. ; HONG, H. ; MAZUMDER, A. ; WANG, S. ; SUNDER, S. ; NICKLAUS, M. C. ; MILNE, G. W. ; PROKSA, B. ; POMMIER, Y.** Depsides and depsidones as inhibitors of HIV-1 integrase: discovery of novel inhibitors through 3D database searching. *J Med Chem,* 1997, vol. 40, 942-51
- **NICKLAUS, M. C. ; NEAMATI, N. ; HONG, H. ; MAZUMDER, A. ; SUNDER, S. ; CHEN, J. ; MILNE, G. W. ; POMMIER, Y.** HIV-1 integrase pharmacophore: discovery of inhibitors through three- dimensional database searching. *J Med Chem,* 1997, vol. 40, 920-9
- **LUBKOWSKI, J. ; YANG, F. ; ALEXANDRATOS, J. ; WLODAWER, A. ; ZHAO, H. ; BURKE, T. R., JR. ; NEAMATI, N. ; POMMIER, Y. ; MERKEL, G. ; SKALKA, A. M.** Structure of the catalytic domain of avian sarcoma virus integrase with a bound HIV-1 integrase-targeted inhibitor. *Proc Natl Acad Sci U S A,* 1998, vol. 95, 4831-6
- **NEAMATI, N. ; MAZUMDER, A. ; ZHAO, H. ; SUNDER, S. ; BURKE, T. R., JR. ; SCHULTZ, R. J. ; POMMIER, Y.** Diarylsulfones, a novel class of human immunodeficiency virus type 1 integrase inhibitors. *Antimicrob Agents Chemother,* 1997, vol. 41, 385-93
- **NEAMATI, N. ; MAZUMDER, A. ; SUNDER, S. ; OWEN, J. M. ; SCHULTZ, R. J. ; POMMIER, Y.** 2-Mercaptobenzenesulfonamides as novel class of human immunodeficiency type 1 (HIV-1) integrase and HIV-1 replication. *Antimicrob Agents Chemother,* 1997, vol. 8, 485-495
- **GAROFALO, A. ; BALCONI, G. ; BOTTA, M. ; CORELLI, F. ; D'INCALCI, M. ; FABRIZI, G. ; FIORINI, I. ; LAMBA, D. ; NACCI, V.** Thioanalogues of anti-tumor antibiotics. II. Synthesis and preliminary in vitro cytotoxicity evaluation of tricyclic [1,4]benzothiazepine derivatives. *Eur J Med Chem,* 1993, vol. 29, 213-220

- **NACCI, V. ; GAROFALO, A. ; ANZINI, M.** Thioanalogues of antitumor antibiotics. I. Synthesis of 7,8-disubstituted 5,11-dioxo-1,2,3,11a-tetrahydro-5H,11H- and 5-oxo-2,3,11,11a-tetrahydro-1H,5H-pyrrolo[2,1-c][1,4]benzothiazepine. *Farmaco,* 1989, vol. 44, 423-433
- **NACCI, V. ; GAROFALO, A. ; FIORINI, I.** olycondensed heterocycles. II. A new preparative route to 11-oxo-5H,11H-pyrrolo[2,1-c][1,4]benzothiazepine. *J Heterocycl Chem,* 1986, vol. 23, 769-773
- **GAROFALO, A. ; NACCI, V. ; CORELLI, F. ; CAMPIANI, G.** Polycondensed heterocycles. V. Synthesis of 5H,11H-pyrrolo[2,1-c][1,4]benzothiazepine. *Heterocycles,* 1990, vol. 31, 1291-1300
- **GAROFALO, A. ; CAMPIANI, G. ; CIANI, S. M. ; FIORINI, I. ; NACCI, V.** Polycondensed heterocycles. IX. Pyrrolo[2,1-c][1,4]benzothiazepines Synthesis of 3-(dimethylamino)methyl derivatives. *Tetrahedron,* 1996, vol. 52, 7745-7754
- **GAROFALO, A. ; CAMPIANI, G. ; NACCI, V. ; FIORINI, I.** Polycondensed heterocycles. VIII. Synthesis of 11-aryl-5H,11H-pyrrolo[2,1-c][1,4] benzothiazepines by Pummerer rearrangement-cyclization reaction. *Heterocycles,* 1992, vol. 34, 51-60
- **ARCHER, S. ; MILLER, J. K. ; REJ, R. ; PERIANA, C. ; FRICKER, L.** Ring-hydroxylated analogues of lucantone as antitumor agents. *J Med Chem,* 1982, vol. 25, 220-227
- **KATZ, L. ; KARGER, L. S. ; SCHROEDER, W. ; COHEN, M. S.** Hydrazine derivatives. I. Benzalthio- and bisbenzaldithio-salicylhydrazides. *J Org Chem,* 1953, vol. 18, 1380-1402
- **PENDERGAST, W. ; DICKERSON, S. H. ; DEV, I. K. ; FERONE, R. ; DUCH, D. S. ; SMITH, G. K.** Benzo[f]quinazoline inhibitors of thymidylate synthase: methyleneamino-linked aroylglutamate derivatives. *J Med Chem,* 1994, vol. 37, 838-844
- **GUISE, G. B. ; OLLIS, W. D. ; PEACOCK, J. E. ; STEPHANATOU, J. S. ; STODDART, J. F.** Dithiosalicylides and trithiosalicylides. Their conformational behavior in solution. *Tetrahedron Lett,* 1980, vol. 21, 4203-4206
- **VITALI, T. ; MOSSINI, F. ; PLAZZI, P. V.** Sintesi ed attivita' antifungine di bis-(3-carbossiamido-2-naftil)disolfuri e di nafto[3,2-d]isotiazolin-3-oni. *Il Farmaco Ed Sc,* 1974, vol. 29, 27-36
- **LALEZARI, I. ; SCHWARTZ, E. L.** Synthesis and antineoplastic activity of 5-Aryl-2,3-dihydropyrrolo[2,1-b]thiazole-6,7-dimethanol 6,7-bis(isopropylcarbamates. *J Med Chem,* 1988, vol. 31, 1427-1429
- **FALORNI, M. ; CONTI, S. ; GIACOMELLI, G. ; COSSU, S. ; F., S.** Optically active 4-oxaproline derivatives: new useful chiral synthons derived from serine and threonine. *Tetrahedron: Asymmetry,* 1995, vol. 6, 287-294
- **GOLDGUR, Y. ; DYDA, F. ; HICKMAN, A. B. ; JENKINS, T. M. ; CRAIGIE, R. ; DAVIES, D. R.** Three new structures of the core domain of HIV-1 integrase: An active site that binds magnesium. *Proc Natl Acad Sci U S A,* 1998, vol. 95, 9150-4
- **FESEN, M. R. ; POMMIER, Y. ; LETEURTRE, F. ; HIROGUCHI, S. ; YUNG, J. ; KOHN, K. W.** Inhibition of HIV-1 integrase by flavones, caffeic acid phenethyl ester (CAPE) and related compounds. *Biochem Pharmacol,* 1994, vol. 48, 595-608
- **CLANTON, D. J. ; BUCKHEIT, R. W., JR. ; TERPENING, S. J. ; KISER, R. ; MONGELLI, N. ; BORGIA, A. L. ; SCHULTZ, R. ; NARAYANAN, V. ; BADER, J. P. ; RICE, W. G.** Novel sulfonated and phosphonated analogs of distamycin which inhibit the replication of HIV. *Antiviral Res,* 1995, vol. 27, 335-54
- **BADER, J. P. ; MCMAHON, J. B. ; SCHULTZ, R. J. ; NARAYANAN, V. L. ; PIERCE, J. B. ; HARRISON, W. A. ; WEISLOW, O. S. ; MIDELFORT, C. F. ; STINSON, S. F. ; BOYD, M. R.** Oxathiin carboxanilide, a potent inhibitor of human immunodeficiency virus reproduction. *Proc Natl Acad Sci U S A,* 1991, vol. 88, 6740-4
- **KAGEYAMA, S. ; MIMOTO, T. ; MURAKAWA, Y. ; NOMIZU, M. ; FORD, H., JR. ; SHIRASAKA, T. ; GULNIK, S. ; ERICKSON, J. ; TAKADA, K. ; HAYASHI, H. et al.** In vitro anti-human immunodeficiency virus (HIV) activities of transition state mimetic HIV protease inhibitors containing allophenylnorstatine. *Antimicrob Agents Chemother,* 1993, vol. 37, 810-7
- **BUCKHEIT, R. W., JR. ; ROBERSON, J. L. ; LACKMAN-SMITH, C. ; WYATT, J. R. ; VICKERS, T. A. ; ECKER, D. J.** Potent and specific inhibition of HIV envelope-mediated cell fusion and virus binding by G quartet-forming oligonucleotide (ISIS 5320. *AIDS Res Hum Retroviruses,* 1994, vol. 10, 1497-506
- **RICE, W. G. ; SUPKO, J. G. ; MALSPEIS, L. ; BUCKHEIT, R. W., JR. ; CLANTON, D. ; BU, M. ; GRAHAM, L. ; SCHAEFFER, C. A. ; TURPIN, J. A. ; DOMAGALA, J. et al.** Inhibitors of HIV nucleocapsid protein zinc fingers as candidates for the treatment of AIDS. *Science,* 1995, vol. 270, 1194-7
- **TURPIN, J. A. ; TERPENING, S. J. ; SCHAEFFER, C. A. ; YU, G. ; GLOVER, C. J. ; FELSTED, R. L. ; SAUSVILLE, E. A. ; RICE, W. G.** Inhibitors of human immunodeficiency virus type 1 zinc fingers prevent normal processing of gag precursors and result in the release of noninfectious virus particles. *J Virol,* 1996, vol. 70, 6180-9

- **RICE, W. G. ; BAKER, D. C. ; SCHAEFFER, C. A. ; GRAHAM, L. ; BU, M. ; TERPENING, S. ; CLANTON, D. ; SCHULTZ, R. ; BADER, J. P. ; BUCKHEIT, R. W., JR.** Inhibition of multiple phases of human immunodeficiency virus type 1 replication by a dithiane compound that attacks the conserved zinc fingers of retroviral nucleocapsid proteins. *Antimicrob Agents Chemother,* 1997, vol. 41, 419-26
- **WEISLOW, O. W. ; KISER, R. ; FINE, D. ; BADER, J. ; SHOEMAKER, R. H. ; BOYD, M. R.** New soluble-formazan assay for HIV-1 cytopathic effects: application to high-flux screening of synthetic and natural products for AIDS antiviral activity. *J Natl Cancer Inst,* 1989, vol. 81, 577-586